# EUROPEAN PATENT SPECIFICATION

(11) **EP 3 438 093 B1**
(45) Date of publication and mention of the grant of the patent: **01.07.2020**
(21) Application number: 17382539.9
(22) Date of filing: 03.08.2017
(51) Int. Cl.: C07C 323/20, C07C 323/22, C07C 323/66, C07D 493/10, C09J 4/00

(54) **CATECHOL-DERIVATIVE COMPOUNDS AND THEIR USE**
CATECHOL-DERIVATEN UND IHRE VERWENDUNG
DÉRIVÉS DE CATÉCHOL ET LEUR UTILISATION

(43) Date of publication of application: 06.02.2019
(73) Proprietor: Fundació Institut Català de Nanociència i Nanotecnologia, 08193 Bellaterra (ES); Consejo Superior de Investigaciones Científicas (CSIC), 28006 Madrid (ES)
(72) Inventor: RUIZ MOLINA, Daniel, 08206 Sabadell (ES); SEDÓ VEGARA, Josep, 08750 Molins de Rei (ES); MANCEBO ARACIL, Juan, 8000 Buenos Aires (AR)
(74) Representative: Ponti & Partners, S.L.P

(56) References cited:
- WO-A1-2008/049108

## Description

### FIELD OF THE INVENTION

The present invention falls within the technical field of organic chemistry. More specifically, the present invention relates to the development of compounds which can be used to modify surfaces or to manufacture multifunctional coatings.

### ANTECEDENTES

Various catechol derivatives are known in the art. In particular, European patent application EP2589578 relates to alkyl (or fluoroalkyl) derivatives of catechol, wherein the alkyl chains are in 3 and 5 positions of the aromatic ring bound thereto by carbon atoms. These catechol derivatives allow the modification of the properties of a substrate by providing hydrophobic and also oleophobic properties.

On the other hand, the patent application WO2008/049108 A1 describes catechol derivatives of general formula: 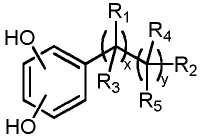 wherein each of R₁, R₂, R₃, R₄ and R₅ is independently selected from the group consisting of a thiol, a primary amine, a secondary amine, a nitrile, an aldehyde, an imidazole, an azide, a halide, a polyhexamethylene dithiocarbonate, a hydrogen, a hydroxyl, a carboxylic acid, an aldehyde, a carboxylic acid ester, a carbamide, providing that at least one from R₁, R₂, R₃, R₄ and R₅ is different from hydrogen, wherein x ranges between 0 and 10, wherein y ranges between 0 and 10, providing that x or y is at least 1. In particular, the catechol derivative disclosed in WO2008/049108A1 can be 3,4-dihydroxy-L-phenylalanine (DOPA), 3,4-dihydroxyphenylalanine methyl ester, dopamine, norepinephrine and epinephrine.

Additionally, WO2008/049108 A1 discloses various uses of the catechol derivatives of the general formula as described. These uses include: the use of catechol derivatives as constituent subunits, or their use to obtain a substrate with a modified surface resistant to *biofouling.*

However, in none of these documents a catechol derivative comprising functional chains bonded to the aromatic ring by a sulfur atom, wherein said chains are located at positions 3 and 6 of the aryl is disclosed. Also, with the information available in the state of the art, it is not possible to predict a priori, with a reasonable probability of success, the properties that would have a catechol derivative with these characteristics, since it would be expected that the properties of these compounds, and of the polymers obtained therefrom may be modified depending on whether the side chains are o-, m- or p- with respect to hydroxyl groups and depending on the atom bonded to the aromatic ring.

In the catechol derivatives disclosed in the present invention, each catechol ring can act as an "adhesive" fragment, capable of being adsorbed, or covalently attached or by a coordination bond, to a surface, while substituents R¹ and, in the case of the di-substituted derivatives, R², can act as functional fragments. The incorporation of said functional fragments into the structure of the disclosed compounds can be carried out by a sequence of reactions comprising the oxidation of a catecholic ring, followed by the reaction of the resulting o-quinone with a molecule incorporating a functional fragment substantially inert to the reaction conditions, and a thiol group responsible for the nucleophilic addition of said fragment to the o-quinone ring (thia-Michael reaction). In these fragments, the sulfur atom acts as a covalent bond bridge between the functional fragment and the catechol ring, generating a structure of aryl ether-type in the final compound. The final structure of the disclosed compounds presents important advantages over those disclosed in the state of the art.

In particular, the bonds through sulfur atoms provide a robust covalent bond between the functional fragment and the catecholic ring, in particular resistant to hydrolysis and enzymatic attack. This is a significant advantage over other already known catechol derivatives (WO2008/049108A1), wherein the bond between the functional chain and the catechol ring-containing fragment, i.e. the adhesive subunit formed by the catechol ring and the group - CH₂CH₂-, was carried out by an easily hydrolysable amide linkage type. Therefore, these types of compounds had a weak point in the structure, since by hydrolysis of the amide linkage the total or partial loss of the desired functionality could be produced by separation between the functional chain and the adhesive catecholic fragment.

On the other hand, the synthetic strategy disclosed in the present invention allows the incorporation of the functional fragment in a single reaction step in contrast to more complex multi-step synthetic routes disclosed in the state of the art, and therefore much more costly from an economic point of view. Redundant in this cost efficiency, the nucleophilic addition reaction exhibits optimum atomic efficiency, since the molecular structures of the reactants are integrally incorporated into the final structure of the derivative. In particular, this is an advantage over a previous development (EP2589578A1), in which the incorporation of the functional fragment required a four-step process with its respective purification processes, among which a Wittig reaction was performed, whose atomic efficiency is very low.

Additionally the structure of the compounds of the present invention allows the incorporation of a large variety of functional fragments with technological relevance, particularly in the fields of hydrophobicity, oleophobicity, solubility in aqueous media, compatibility in physiological media, solubility in organic solvents of medium or low polarity, bacteriostatic properties, bactericidal properties, antifouling properties, detergent capacity, surfactant capacity, fluorescence, enhancement of cellular recognition and internalization and mucoadhesivity.

The process for obtaining catechol derivatives disclosed herein may be sequentially applied in order to incorporate two functional fragments at symmetric ring positions with respect to and adjacent to the hydroxyl groups of the aromatic ring. The incorporation of functional fragments in said positions allows to obtain a great variety of compounds, suitable for the preparation of a wide range of functional coatings based on the adhesive properties of the catecholic fragments. When these derivatives contain two or more functional fragments of the same nature, the properties provided by this type of functional fragment to the molecule can be enhanced. Alternatively, the incorporation of moieties with different functionalities complementary to each other allows to obtain a great variety of bi- or multifunctional compounds. Additionally, in certain cases it is possible to create new functionalities by combining functional fragments with different properties. In particular, the incorporation of an alkyl moiety and a PEG moiety would make it possible to obtain compounds of formula (I) with non-ionic surfactant properties.

In contrast to compounds disclosed in the state of the art, wherein the functional fragments are attached to the catechol ring at the 4-position, the compounds disclosed herein have functional fragments at the 3-position, and optionally at the 6-position. Surprisingly, it has been found that the location of the functional fragment at the 3-position of the catecholic ring, i.e., adjacent to the hydroxyl groups, does not result in a higher steric hindrance capable of compromising the adhesive properties of the catecholic fragment.

As mentioned above, the synthetic strategy disclosed herein allows the preparation of a wide range of structures, among which are monopodal or multipodal compounds, as well as homo-or copolymeric compounds, which allow to improve the robustness of the coatings, and hence the attachment between the compounds and the substrate, by incorporating multiple binding sites (catechol rings) in the same molecule.

Finally, the presence of the sulfur atom which provides the covalent bond between the catechol ring and the functional fragment does not impair the stability of the catecholic ring, as compared to analogous molecules where the same chain is attached by a carbon-carbon bond. In particular, the presence of a sulfur atom directly attached to the ring does not make it significantly more prone to oxidation compared to the original catechol ring.

### BRIEF DESCRIPTION OF THE DRAWINGS

**Figure 1****:** Measurement of the contact angle measurement with a drop of water on a TiO₂ surface (Fig. 1.a); measurement of the contact angle with a drop of water on a surface of TiO₂ coated with compounds **1b** (Fig. 1.b), **2b** (Fig. 1.c), **4** (Fig. 1.d) and **5** (Fig. 1.e).
**Figure 2****:** Measurement of the contact angle with a drop of water on a surface of cotton fabric coated with the polymer **p2.**
**Figure 3****:** Measurement of the contact angle with a drop of water on a surface of cotton fabric coated with the polymer **p4** (Fig. 3.a), and measurement of the contact angle with a drop of tetradecane in a cotton fabric coated with the polymer **p4** (Fig. 3.b).
**Figure 4****:** Two vials with the magnetite nanoparticles stabilized with oleic acid (Fig. 4.a) and the nanoparticles after coating, stabilized by the compound **6** (Fig. 4.b).
**Figure 5****:** MALDI Mass spectrometry for the homopolymer **26.**

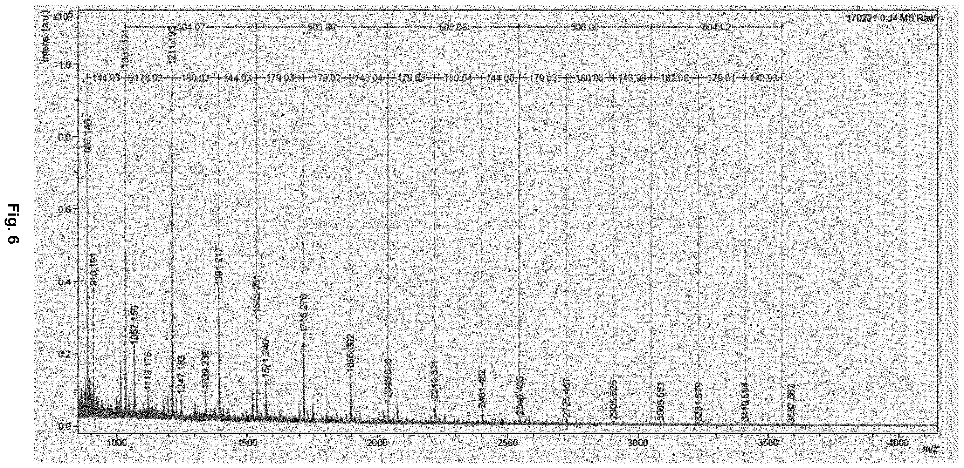

**Figure 6****:** MALDI Mass spectrometry for the co-polymer **27.**
**Figure 7****:** Images of HAADF STEM (20 kV) of amorphous SiO₂ nanoparticles coated with the compound **31.**
**Figure 8****:** Images of (a) *brightfield* and (b) HAADF STEM (20 kV) of amorphous SiO₂ nanoparticles coated with the compound **32.**
**Figure 9****:** Images of optical microscopy in fluorescence mode (Xe lamp, λₑₓ = 450 - 490 nm) of amorphous SiO₂ nanoparticles coated with the compound **32.**

### SUMMARY OF THE INVENTION

In a first aspect, the present invention relates to catechol-derivative compounds of formula (I). In particular, the present invention disclose new linear monopodal derivatives of catechol of formula (I) substituted at the 3-position of aryl (monosubstituted derivatives) with a moiety attached to said ring through a sulfur atom (-SR¹). Additionally, the present invention is also related to catechol derivatives of formula (I) substituted at the 3- and 6-position of aryl (disubstituted derivatives) with moieties attached to said ring through a sulfur atom (-SR¹ and -SR²). The present invention is also related to linear bipodal, mono- and multipodal in star, and polymeric compounds.

In a second aspect, the present invention relates to polymeric compounds obtained by the condensation of the compounds according to the first aspect of the invention.

In a third aspect, the present invention relates to the use of the compounds according to the second aspect of the invention for preparing functional coatings and as adhesive compounds. Thus, such compounds can be used as precursors, as well as main constituents of functional coatings, with strong adhesion to substrates of different nature, presenting significant advantages over the compounds known in the state of the art.

### DETAILED DESCRIPTION OF THE INVENTION

In a first aspect, the present invention relates to catechol-derivative compounds of formula (I): 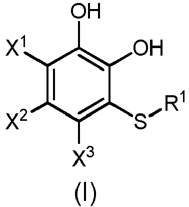 wherein,
**X¹** is selected from the group consisting of hydrogen and a substituent **-SR²**,
**X²** and **X³** are hydrogen atoms,
**R¹** and **R²** are independently selected from the group consisting of:
   - a moiety **FUNC,** which is selected from the group consisting of:
      ∘ an alkyl moiety of formula -CₙH₂ₙ₊₁, wherein n ranges between 12 and 18;
      ∘ an alkenyl moiety of formula -CₙH₂ₙ₋₁, wherein n ranges between 2 and 6;
      ∘ an alkynyl moiety of formula -CₙH₂ₙ₋₃, wherein n ranges between 2 and 6;
      ∘ a polyfluoroalkyl moiety of formula -(CH₂)ₚ₁-CₙF₂ₙ₊₁, wherein p₁ is comprised between 0 and 3, and n is comprised between 5 and 8;
      ∘ a moiety of formula -(CHRCH₂O)_{q}R', wherein R is a hydrogen atom or a methyl group, and R' is selected from a hydrogen atom, a C₁-C₁₀ alkyl group, terminal C₁-C₁₈alkenyl, terminal C₁-C₁₈ alkynyl, -COOH, -NH₂, - N₃ or *N*-maleimido, and q is a value comprised between 5 and 300;
      ∘ a fluorescent tag;
      ∘ an oligopeptide selected from glutathione and an oligopeptide comprised of 5-25 amino acids, from which from 20% to 100% are arginine; and
   - a moiety **-R³-S-Z**, wherein,
      ∘ S is a sulfur atom,
      ∘ **R³** is selected from the group consisting of:
         ▪ an alkandiyl moiety of formula -(C_{n'}-H_{2n'})-, wherein n' is comprised between 2 and 18;
         ▪ a (polyalkylenoxy)alkyl moiety of formula - (CHR"CH₂O)_{q1}(CHR"CH₂)-, wherein **R**" is selected from the group consisting of a hydrogen atom and a methyl moiety, and q₁ is comprised between 2 and 300;
      ∘ **Z** is selected from the group consisting of:
         ▪ a hydrogen atom;
         ▪ a -COCH₃ moiety;
         ▪ a -CH₂-CH₂-Y-FUNC moiety, wherein **Y** is selected from the group consisting of -O-, -COO-, -CONH-, -(CH₂)ᵣO-, being r a value comprised between 1 and 10; and FUNC is the moiety as previously defined;
         ▪ a moiety of structure (**J***): 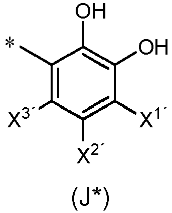 wherein **X¹**' is selected from the group consisting of hydrogen, and a substituent **-SR²**; wherein R² is as previously defined; and **X^{2'}** and **X^{3'}** are hydrogen atoms;
   - a moiety of the type **(BRANCH*):** 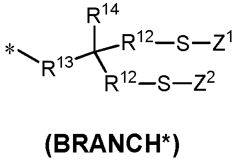 wherein,
      ∘ **R¹²** is a moiety of formula -(CH₂-O-Q-CH₂-W)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of - CHR"'-, and -CH(OH)CH₂-, wherein **R**'" is a hydrogen atom or a methyl group.
      ∘ **R¹³** is a moiety of formula -(W-CH₂-Q-O-CH₂)-, wherein **Q** and **W are** defined in the same way as for R¹²;
      ∘ **Z¹** and **Z²** are independently selected from the group consisting of:
         ▪ a hydrogen atom;
         ▪ a -COCH₃ group;
         ▪ a moiety of structure (**J***)
         ▪ a -CH₂-CH₂-Y-FUNC moiety, wherein **Y** is selected from the group consisting of -O-, -COO-, -CONH-, -(CH₂)ᵣO-, being r a value comprised between 1 and 10; and FUNC is the moiety as previously defined
         ▪ a moiety of structure **FUNC,** as previously defined.
      ∘ **R¹⁴** is selected from the group consisting of a hydrogen atom, a methyl moiety, an ethyl moiety and a moiety **-R¹²-S-Z³**, wherein **Z³** is independently selected from the same group as Z¹ and Z².

In a preferred embodiment for the compound of formula (I), **X¹** is a hydrogen atom, and **Z** is a hydrogen atom or an acetyl moiety (-COCH₃).

In another preferred embodiment of the compound of formula (I), **X¹** is a hydrogen atom; and **R¹** is a moiety **-R³-S-Z**, wherein **Z** is a moiety -CH₂-CH₂-Y-FUNC, wherein Y is selected from the group consisting of -O-, -COO-, -(CH₂)ᵣO-, being r a value comprised between 1 and 10 and FUNC is as previously defined.

In another preferred embodiment of the compound of formula (I), **X¹** is a hydrogen atom; and **R¹** is a moiety **-R³-S-Z**, wherein **Z** is a moiety of structure (**J***) wherein **X¹**' is a hydrogen atom.

In another preferred embodiment of the compound of formula (I), **X¹** is a hydrogen atom; and **R¹** is a moiety of the type (BRANCH*), as previously defined, wherein,
- **Z¹** and **Z² are** independently selected from the group consisting of:
   ∘ a hydrogen atom;
   ∘ a -COCH₃ group;
   ∘ a moiety of structure (**J***), wherein **X¹', X²'** and **X³'** are hydrogen atoms;
   ∘ a moiety of structure **FUNC,** as previously defined;
- **R¹⁴** is an ethyl moiety;
optionally, **Z¹** and **Z²** are independently selected from the group consisting of
- a hydrogen atom;
- a moiety of structure (**J***), wherein **X¹', X²'** and **X³'** are hydrogen atoms; or optionally, **Z¹** is a moiety of formula -(CH₂CH₂O)_{q}R', wherein R' is a hydrogen atom or a methyl group and q is a value comprised between 5 and 300; or optionally, **Z¹** is a fluorescent tag.

In another preferred embodiment of the compound of formula (I), **X¹** is a hydrogen atom; and **R¹** is a moiety of the type (BRANCH*), as previously defined, wherein,
- **Z¹** and **Z²** are independently selected from the group consisting of:
   ∘ a hydrogen atom;
   ∘ a -COCH₃ group;
   ∘ a moiety of structure (**J***), wherein **X¹'**, **X²'** and **X³'** are hydrogen atoms.
   ∘ a moiety of structure **FUNC,** as previously defined.
- **R¹⁴** is a moiety **-R¹²-S-Z³**, wherein **Z³** is independently selected from the same group as Z¹ and Z²;
optionally **Z¹**, **Z² and Z³** are independently selected from the group consisting of
- a hydrogen atom;
- a moiety of structure (**J***), wherein **X¹', X²'** and **X³'** are hydrogen atoms; or optionally **Z¹** and **Z²** are hydrogen atoms, and **Z³** is a moiety of formula -(CH₂CH₂O)_{q}R', wherein R' is a hydrogen atom or a methyl group and q is a value comprised between 5 and 300; or
optionally **Z¹** and **Z²** are hydrogen atoms, and **Z³** is a fluorescent tag.

The different compounds obtainable under general formula (I) will be further described below.

### I. Linear monopodal compounds

Firstly, the present invention relates to a catechol derivative of formula (I), 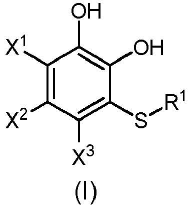 wherein,
**X²** and **X³** are independently selected from the group consisting of hydrogen and a blocking group of said positions;
**X¹** is selected from the group consisting of hydrogen, a blocking group of said position, and a substituent **-SR²**, wherein
**R¹**y **R²** are independently selected from the group consisting of:
   - a moiety **FUNC,** which is selected from the group consisting of:
      - a linear or branched alkyl moiety of formula -CₙH₂ₙ₊₁, wherein n ranges between 1 and 30;
      - a linear or branched alkenyl moiety of formula -CₙH₂ₙ₋₁, wherein n ranges between 1 and 30;
      - a linear or branched alkynyl moiety of formula -CₙH₂ₙ₋₃, wherein n ranges between 1 and 30;
      - a polyfluoroalkyl moiety of formula -(CH₂)ₚ₁-CₙF₂ₙ₊₁, wherein p₁ is equal to or higher than 0, and n ranges between 1 and 30;
      - an alkyl sulphonic acid moiety of formula -CₙH₂ₙSO₃H, wherein the alkandiyl moiety -CₙH₂ₙ is linear or branched, and n ranges between 1 and 30;
      - an alkylamine moiety of formula -CₙH₂ₙNR⁹R¹⁰ or alkylammonium salt of formula -CₙH₂ₙN⁺R⁹R¹⁰R¹¹, wherein the alkandiyl moiety -CₙH₂ₙ is linear or branched, n ranges between 1 and 30, and R⁹, R¹⁰ and R¹¹ are independently selected from the group consisting of hydrogen, a C₁-C₃₀ alkyl moiety, a C₅-C₂₀ aryl moiety, a C₃-C₄₀ alicyclic moiety, a C₃-C₄₀ aralkyl moiety and a C₃-C₄₀ alkylaryl moiety;
      - a moiety of formula -(CHRCH₂O)_{q}R', wherein R is a hydrogen or C₁-C₁₈ alkyl substituent, and R' is selected from a C₁-C₁₈ alkyl group, terminal C₁-C₁₈ alkenyl, terminal C₁-C₁₈ alkynyl, -COOH, -NH₂, -N₃ or *N*-maleimido, and q is a value comprised between 2 and 1000;
      - a C₅-C₂₀ aromatic moiety comprising none, one or more heteroatoms selected from the group consisting of O, N, S and a combination thereof;
      - an alicyclic C₃-C₄₀ moiety comprising none, one or more heteroatoms selected from the group consisting of O, N, S and a combination thereof;
      - a moiety with fluorescent tag properties;
      - an oligopeptide selected from glutathione and an oligopeptide comprised of 5-25 aa and rich in arginine;
      - a moiety derived from a mono-, oligo- or polysaccharide; and
   - a moiety **R³-S-Z**, wherein,
      ∘ **S** is a sulfur atom,
      ∘ **R³** is selected from the group consisting of:
         - a linear or branched alkandiyl moiety of formula -(C_{n'}H_{2n'})-, wherein n' is equal to or higher than 1;
         - a linear or branched alkendiyl moiety of formula -(C_{n'}H_{2n'-2})-, wherein n' is equal to or higher than 1;
         - a linear or branched alkyndiyl moiety of formula -(C_{n'}H_{2n'-4})-, wherein n' is equal to or higher than 1;
         - an arenediyl moiety which may comprise none, one or more heteroatoms capable of imparting aromaticity to the substructure selected from the group consisting of O, N, S and a combination thereof, and/or may comprise none, one or more substituents different from hydrogen;
         - a polyfluoroalkandiyl moiety of formula -(CH₂)ₚ₃(CF₂)ₚ₄(CH₂)ₚ₅-, wherein p₃ and p₅ are independently selected from values equal to or higher than 0, and p₄ is equal to or higher than 1;
         - a (polyalkylenoxy)alkyl moiety of formula -(CHRCH₂O)_{q1}(CHRCH₂)-, wherein R is selected from the group consisting of hydrogen and a C₁-C₃₀ alkyl moiety, and q₁ is a value equal to or higher than 1;
         - a diarylene ether or diarylene thioether moiety, wherein each arylene moiety may comprise none, one or more heteroatoms selected from the group consisting of O, N, S and a combination thereof, and/or may comprise none, one or more substituents different from hydrogen; and
         - a moiety of formula -[(CH₂)ᵣ₁(CHOR⁴)(CH₂)ᵣ₂(CHOR⁵)(CH₂)ᵣ₃]-, wherein r₁ and r₃ are independently selected from values equal to or higher than 1, r₂ is equal to or higher than 0, and **R⁴** and **R⁵** are independently selected from the group consisting of:
            ▪ a hydrogen atom;
            ▪ an activated ester group;
            ▪ any of the moieties previously defined for FUNC;
         - a moiety of formula -(CH₂)ₛ₁(CNR⁶R⁷)(CH₂)ₛ₂- or - (CH₂)ₛ₁(CN⁺R⁶R⁷R⁸)(CH₂)ₛ₂-, wherein s₁ and s₂ are independently selected from values equal to or higher than 1, and **R⁶**, **R⁷** and **R⁸** are independently selected from the group consisting of:
            ▪ a hydrogen atom;
            ▪ an activated ester group;
            ▪ any of the moieties previously defined for FUNC;
            ▪ a moiety comprising a combination of any of the moieties mentioned above;
      ∘ **Z** is selected from the group consisting of a hydrogen atom, a -COCH₃ group; and a -CH₂-CH₂-Y-R" group, wherein Y is selected from the group consisting of -O-, -COO-, -CONH-, -(CH₂)ᵣO-, being r a value comprised between 1 and 10, and R" is any of the moieties previously defined for R¹ and R².
   - a moiety **R³-T** wherein R³ is defined as previosuly and T is a moiety -OH or an an activated ester.

Herein, the term *"blocking group"* is to be understood as any substituent inert to the sequence of reactions formed by the oxidation of a catechol and a thia-Michael reaction, i.e., a substituent which blocks the position of the ring supporting thereof during the aforementioned sequence of reactions.

Also, the term *"3-position of the ring"* is used to refer to any of the two positions of the catechol ring immediately adjacent to the hydroxyl groups. Similarly, the term *"6-position of the ring"* is used to denote the other position adjacent to the hydroxyl groups, once the 3-position is already occupied by a substituent of the type SR¹.

Herein, the term *"monosubstituted derivative"* is used to refer to a molecule of formula (I) where a -SR¹ substituent has been incorporated at the 3-position of the ring. Similarly, the term *"disubstituted derivative"* is used to refer to a molecule of formula (I) where two distinct substituents (-SR¹ and -SR², wherein R¹ and R² are different) or the same substituent (-SR¹ and -SR², where R¹ and R² are the same) have been incorporated at the 3- and 6-position of the ring. In both cases, and optionally, the starting molecules may have substituents at the other ring positions (4, 5 and optionally, 6) prior to the sequence of reactions of the described process. In the case of the substituents at the 4- or 5-position, those which are attached to the catechol ring through a sulfur atom are excluded.

Herein, the term *"monopodal derivative"* is used to describe a molecule having a single catechol ring. By analogy, the terms *"bipodal derivative",* "tripodal derivative", and *"tetrapodal derivative"* will be used to describe molecules having, respectively, two, three or four rings of catechol, preferably incorporated into the structure by the mentioned sequence of reactions.

Herein, the term *"comprised between N₁ and N₂*" is used to describe a range comprising all possible values between N₁ and N₂, including N₁ and N₂, being the synonymous expression *"higher than or equal to N₁ and less or equal to N₂*".

Herein, the terms *"fluorescent tag", "fluorescent label*" or *"fluorescent moiety"* synonymously describe any substituent or molecular fragment whose fluorescent label properties are known and commonly used by the person skilled in the art for the purpose of labelling another molecule in an analytical context, e.g., but not exclusively, in the fields of biology, biochemistry and medicine. Fluorescence is understood as the ability of a molecule to absorb energy in the form of electromagnetic radiation and to emit subsequently part of that energy in the form of electromagnetic radiation at a different wavelength. Examples of fluorescent moieties which the person skilled in the art will recognize as useful for the specific labeling of molecules are e.g. fluorescent compounds with thiol groups, such as 9-mercaptofluorene or 7-mercapto-4-methylcoumarin, as well as fluorone/xanthene derivatives, such as fluorescein, eosin, rhodamines such as Rhodamine B, Rhodamine 6G, Rhodamine123, Rhodamine Red, tetramethylrodamine, Texas Red and Oregon Green; boron-dipyrromethene (BODIPY) fluorescent derivatives; fluorescent derivatives of azo structure (diazoarenes), fluorescent derivatives of pyridine-methoxyphenyleneoxazoles (PyMPO), fluorescent derivatives of Lucifer Yellow, fluorescent derivatives of benzoxadiazol (BD), including benzoxadiazoles (ABD) and nitrobenzoxadiazoles (NBD), Fluorescent Red, Fluorescent Orange, and other fluorescent labels available under registered trademarks, such as Atto and Alexa, etc., all of which can be conjugated through ad hoc reactive groups, such as, for example, acrylates, iodoacetates, N-substituted maleimides, azides, alkynes, N-hydroxysuccinimide esters (NHS) and thiocyanates, among others.

By *"activated ester"* is meant herein any ester commonly used by the person skilled in the art for the conversion of an alcohol into a leaving group in a nucleophilic substitution reaction. Representative activated esters are, for example, the reaction products from the esterification of alcohols with sulphonic acids such as, for example, methanesulphonic, paratoluenesulphonic or perfluoromethanesulphonic acid.

By *"thiol precursor group"* is meant herein a functional group directly convertible into a thiol group by processs known to the person skilled in the art. Examples of thiol precursor groups are disulphides and thioesters, preferably thioacetyl.

*"Functional chain"* or *"functional fragment*" means herein any moiety attached to a catechol ring capable of providing the coatings derived from the compound in question with one or more of the following functional properties of technological interest, for example hidrophobicity, oleohidrophobicity, solubility in aqueous media, solubility in organic solvents of medium or low polarity, bacteriostatic properties, bactericidal properties, *antifouling* properties, detergent capacity, surfactant capacity, fluorescence, improved recognition and cellular internalization and mucoadhesivity. These terms need to be differentiated from the term *"functional group"* which, in the sense usually given by the person skilled in the art, is to be understood as a specific grouping of atoms and bonds responsible for characteristic chemical reactions in the compound.

Herein, *"antifouling"* or resistant to *"biofouling"* is the property of a coating or surface under which it is able to prevent the accumulation on itself of proteins or living organisms, such as microorganisms and algae, especially in aqueous media, such as aquatic and physiological media.

Thus, the compounds of formula (I) which are described in the present invention comprise, at least one substituent of the type SR¹ (monosubstituted derivatives). Optionally, compounds of formula (I) may incorporate a substituent SR² at position X¹ (disubstituted derivatives).

The blocking group which may be present in one or more of the X¹, X² or X³ positions of the compound of formula (I) may be independently selected from the group consisting of:
- an halide, such as -F, -Cl, -Br or -I, preferably F;
- a linear or branched alkyl moiety of formula -CₙH₂ₙ₊₁, wherein n ranges between 1 and 30, more preferably n ranges between 1 and 18, being the moiety preferably methyl, ethyl, propyl, butyl, isobutyl, tert-butyl, pentyl, hexyl, heptyl, octyl, nonyl, dodecyl, hexadecyl, octadecyl, and even more preferably methyl or tert-butyl;
- an oxialkyl moiety of formula -OCₙH₂ₙ₊₁, wherein the alkyl moiety may be linear or branched, and n can range between 1 and 30, more preferably n ranges between 1 and 18, being the alkyl moiety preferably methyl, ethyl, propyl, tert-butyl, hexyl, nonyl, dodecyl, hexadecyl, octadecyl; and still more preferably methyl, ethyl, nonyl or octadecyl;
- an oxyalkylenaryl moiety of formula -OCₙH₂ₙAr, wherein the alkylene moiety may be linear or branched; n can range between 1 and 30, preferably n ranges between 1 and 18, and even more preferably n is equal to 1 (methylene), 2 (ethylene), 6 (hexamethylene), 11 (undecamethylene), 12 (dodecamethylene), 17 (heptadecamethylene) and 18 (octadecamethylene); and Ar may be an aryl moiety, preferably phenyl;
- a linear or branched acylalkyl moiety of formula -COCₙH₂ₙ₊₁, wherein n can range between 1 and 30, more preferably n ranges between 1 and 18, being the alkyl moiety preferably methyl, ethyl, isopropyl, tert-butyl, hexyl, dodecyl or octadecyl;
- a polyfluoroalkyl moiety of formula -(CH₂)ₚ₁(CF₂)ₚ₂F, wherein p₁ is equal to or higher than 0 and p₂ is equal to or higher than 1, being preferably p₁ equal to 2 and p₂ higher than 5;
- an alkandiyl moiety substituted with a terminal functional group, for example an alkylsulphonic acid of formula -CₙH₂ₙSO₃H, alkylamine of formula -CₙH₂ₙNR⁹R¹⁰, alkylammonium salt of formula -CₙH₂ₙN⁺R⁹R¹⁰R¹¹ or alkylacarboxylic acid of formula -CₙH₂ₙCOOH, wherein the moiety -CₙH₂ₙ- may be linear or branched, n can range between 1 and 30, more preferably n ranges between 1 and 18, and still more preferably n is selected from 1, 2, 6, 11, 12, 17 and 18; and wherein R⁹, R¹⁰ and R¹¹ can be independently selected from hydrogen, a linear or branched C₁-C₃₀ alkyl moiety, a C₅-C₂₀aryl moiety, a C₃-C₄₀alicyclic moiety, a C₃-C₄₀ aralkyl moiety and a C₃-C₄₀ alkylaryl moiety; being these preferably methyl, ethyl, isopropyl, tert-butyl, hexyl, dodecyl, octadecyl, phenyl, benzyl, tolyl, furyl, pyrryl, thiophenyl, pyridyl, cyclopropyl, cyclopentyl, cyclohexyl, piperidyl, cholesteryl, phenethyl or ethylphenyl;
- a C₅-C₂₀ aryl moiety which can comprise none, one or more heteroatoms selected from the group consisting of O, N, S and a combination thereof, and/or can comprise none, one or more substituents different from hydrogen, being the aryl moiety preferably phenyl, tolyl, xylyl, naphthyl, anthryl, phenanthryl, pyryl, thiophenyl, pyrryl, furyl or pyridyl;
- a moiety with C₃-C₄₀ alicyclic structure, preferably cyclopropyl, cyclobutyl, cyclopentyl, cyclohexyl, piperidyl, tetrahydronaphthyl or cholesteryl;
- a polyalkylene glycol chain of formula -(CHRCH₂O)_{q}R', wherein R and R' are independently selected from hydrogen and C₁-C₁₈ alkyl, and q is a value comprised between 2 and 1000; preferably R is hydrogen or methyl, R' is preferably selected from methyl, ethyl, isopropyl, tert-butyl, hexyl, dodecyl and octadecyl, and q is a value comprised between 6 and 300;
- a functional group directly attached to the catechol ring, selected from the group consisting of nitro (-NO₂), cyano (-CN), carboxylic acid (-COOH), sulphonic acid (-SO₃H) and ester of carboxylic acid of formula -COOR, wherein R is a linear or branched C₁-C₃₀ alkyl moiety, a C₅-C₂₀ aryl moiety, a C₃-C₄₀ alicyclic moiety, a C₃-C₄₀ aralkyl moiety and a C₃-C₄₀ alkylaryl moiety; and
- a blocking group formed by a combination of those mentioned above, such as, for example, aralkyl or alkylaryl moieties, preferably phenethyl, phenylhexyl, ethylphenyl and propylphenyl.

Alternatively, X² and X³ may form part of the same blocking group, so that the 4 and 5-positions of the catechol ring are joined forming a cycle.

Additionally, in the compound of formula (I) disclosed in the present invention, any two adjacent positions of the catechol ring may be covalently connected by chains of two or three atoms, forming 6-membered or 7-membered rings orthofused to the catechol ring .

In the catechol derivative of formula (I) disclosed in the present invention it is preferred that X² and X³ are hydrogen atoms, in which case the starting molecule in the preparation process disclosed herein may be pyrocatechol, having a simple structure, wide availability and economic price. As it is known in the art for numerous o-quinones obtained by oxidation of their respective catechol derivatives, including the pyrocatechol itself, the *thia*-Michael reaction runs regioselectively towards one of the positions adjacent to the hydroxyl groups of the aromatic ring. In addition, and unexpectedly, in obtaining the compounds of formula (I) by the synthesis process disclosed herein, it has been found that once the 3-position of the pyrocatechol molecule is substituted, when the 6-position is free, i.e. the other position adjacent to the hydroxyl groups, the second substitution takes place regioselectively at said position, leaving intact the 4 and 5-positions of the ring, i.e., those bearing the substituents X² and X³. Therefore, when pyrocatechol is used as the starting catecholic molecule, such ring positions, occupied by different hydrogen atoms, may be considered as non-reactive under the reaction conditions described herein, and therefore their blocking or protection is unnecessary.

Optionally, in those cases where the starting molecule incorporates substituents X² and X³ different from hydrogen, it is preferred that these are functional fragments capable of enhancing or complementing the properties of the functional fragments R¹ and/or R².

On the other hand, when X¹ is a blocking group, it is preferred that it is a fluorine atom because, in addition to its blocking capacity of this aromatic position, which is reactive in the synthetic process disclosed herein, can provide the catechol derivative with additional storage stability against oxidizing means, such as air.

The compounds of formula (I) may comprise a linear or branched alkyl moiety of formula - CₙH_{2n + 1} in one or more of the substituents R¹, R², R⁴, R⁵, R⁶, R⁷, R⁸, R⁹, R¹⁰, R¹¹, X¹, X² or X³. In the case of comprising more than one alkyl moiety, these may be the same or different from each other. The incorporation of alkyl moieties in these catechol derivatives, in particular in R¹ and optionally in one or more of R², R⁴, R⁵, R⁶**,** R⁷, R⁸, R⁹, R¹⁰, R¹¹, X¹, X² or X³, allows to obtain suitable compounds for use in obtaining coatings with hydrophobic properties. In particular, by incorporating various alkyl moieties, preferably using sequentially the synthetic strategy disclosed in the present invention when these residues are located at the 3,6-positions of the ring, derivatives with a reinforced hydrophobic character can be obtained.

The compounds of formula (I) may comprise a linear or branched alkenyl moiety of formula - CₙH₂ₙ₋₁, or a linear or branched alkynyl moiety of formula -CₙH₂ₙ₋₃ at one or more of the substituents R¹, R², R⁴, R⁵, R⁶, R⁷, R⁸, R⁹, R¹⁰, R¹¹, X¹, X² or X³. In the case of comprising more than one alkenyl or alkynyl moiety, these may be the same or different from one another. The incorporation of both alkenyl and alkynyl moieties in these catechol derivatives, in particular in R¹ and optionally in one or more of R², R⁴, R⁵, R⁶, R⁷, R⁸, R⁹, R¹⁰, R¹¹, X¹, X² or X³, allows to obtain suitable compounds for use in obtaining coatings with hydrophobic properties, as well as proportional to the molecule of reactive points for radical polymerization reactions, as well as radical addition, e.g., but not exclusively, by means of reactions of the thiol-ene or thiol-yne type. In particular, by incorporating various alkyl residues, preferably using sequentially the synthetic strategy disclosed in the present invention when these residues are located at the 3,6-positions of the ring, derivatives with a reinforced hydrophobic character can be obtained, as well as a higher reactivity, which confers the molecule higher possibilities to incorporate substituents or a higher cross-linking in the case of polymerization reactions through the unsaturations provided by the alkenyl and/or alkynyl moieties.

Preferably, the alkyl moieties which may be comprised in one or more of the substituents R¹, R², R⁴, R⁵, R⁶, R⁷, R⁸, R⁹, R¹⁰, R¹¹, X¹, X² or X³, are independently selected from linear or branched alkyl moieties of formula -CₙH₂ₙ₊₁, wherein n is a value comprised between 6 and 30, being still more preferably n having a value between 10 and 22 and, specially preferably, between 12 and 18. The compounds of formula (I) wherein one or more of the substituents R¹, R², R⁴, R⁵, R⁶, R⁷, R⁸, R⁹, R¹⁰, R¹¹, X¹, X² or X³ is a linear alkyl of formula CₙH₂ₙ₊₁, wherein n is equal to 18 are specially preferred, since said chain length provides optimum values in the hydrophobic properties of the coatings derived from said compounds.

Preferably, the alkenyl or alkynyl moieties which may be comprised in one or more of the substituents R¹, R², R⁴, R⁵, R⁶, R⁷, R⁸, R⁹, R¹⁰, R¹¹, X¹, X² or X³, are independently selected, and respectively, from alkenyl or alkynyl moieties of formula -CₙH₂ₙ₋₁ or -CₙH₂ₙ₋₃, wherein n is a value comprised between 2 and 10, being still more preferably n having a value between 2 and 8 and, specially preferably, between 2 and 6, since said chain length provides a suitable balance between chain flexibility and reactivity of the unsaturated moiety.

Additionally, the compounds of formula (I) which are disclosed in the present invention may comprise aromatic-, alicyclic-, arylalkyl- or alkylaryl-type moieties in one or more of the substituents R¹, R², R⁴, R⁵, R⁶, R⁷, R⁸, R⁹, R¹⁰, R¹¹, X¹, X² or X³. When comprising more than one of the aforementioned moieties, these may be the same or different from each other. The incorporation of aromatic, alicyclic, arylalkyl or alkylaryl moieties into these catechol derivatives, in particular in R1 and optionally in one or more of R², R⁴, R⁵, R⁶, R⁷, R⁸, R⁹, R¹⁰, R¹¹, X¹, X² or X³, allows to obtain suitable compounds for use in obtaining coatings with an improved solubility in organic solvents of medium or low polarity, which favors their application in the form of coatings. Thus, by incorporating various aromatic moieties, preferably using sequentially the synthetic strategy described in the present invention when these moieties are at the 3,6-positions of the ring, derivatives with higher compatibility to solvents of medium or low polarity can be obtained.

The aromatic-type moieties may be hydrocarbon-based aryls containing 5 to 20 carbon atoms, preferably 6 to 16 carbon atoms, for example phenyl, benzyl, tolyl, xylyl, naphthyl, anthryl, phenanthryl and pyrenyl. Additionally, these moieties may also be aromatic heterocycles incorporating O, N and S atoms in their structure, such as thiophen-2-yl, pyrro-2-yl, furan-2-yl, 2-pyridyl, benzopyrrolyl, etc. On the other hand, the alicyclic moieties may comprise from 3 to 40 carbon atoms in their structure, such as, for example, cyclopropyl, cyclobutyl, cyclopentyl, cyclohexyl, piperidyl, tetrahydronaphthyl or cholesteryl, as well as alicyclic heterocyclic moieties, such as 2-piperidyl, 2-pyryl and 2-thiopyryl. The residues of the arylalkyl and alkylaryl type may be, for example, phenethyl, phenylhexyl, ethylphenyl, hexylphenyl, diphenylmethyl, triphenylmethyl, among others.

Additionally, the compounds of formula (I) which are disclosed in the present invention may comprise one or more polyfluoroalkyl moieties of formula -(CH₂)ₚ₁CₙF₂ₙ₊₁ in one or more of the substituents R¹, R², R⁴, R⁵, R⁶, R⁷, R⁸, X¹, X² or X³. When comprising more than one of these moieties, these may be the same or different from each other. The incorporation of polyfluoroalkyl moieties in these catechol derivatives, in particular in R¹ and, optionally in one or more of R², R⁴, R⁵, R⁶**,** R⁷, R⁸, X¹, X² or X³, allows to obtain compounds with oleohydrophobic and antifouling properties. Thus, by incorporating various polyfluoroalkyl moieties, preferably using sequentially the synthetic strategy described in the present invention when these moieties are at the 3,6-positions of the ring, derivatives with reinforced oleohydrophobic and antifouling characteristics can be obtained.

Preferably, the polyfluoroalkyl moieties in the compound of formula (I) have the formula - (CH₂)ₚ₁CₙF₂ₙ₊₁, wherein p₁ is comprised between 0 and 3, and n is comprised between 5 and 8. These functional chain structures allow to obtain coatings with optimum oleohydrophobic and antifouling properties, especially when p is equal to 0 and n is equal to 8. Alternatively, when p₁ equal to 2 and n is equal to 6, that is, when a hydrocarbon telomer is incorporated and limits the length of the perfluorinated fragment to 6 carbon atoms, the catechol derivatives of formula (I) may exhibit sufficient oleohyphobicity, and in turn may have higher biological and environmental compatibility, by decreasing the biopersistence of the fluorinated fragment.

The compounds of formula (I) which are disclosed in the present invention may comprise an alkylsulphonic acid moiety of formula -CₙH₂ₙSO₃H in one or more of the substituents R¹, R², R⁴, R⁵, R⁶, R⁷, R⁸, X¹, X² or X³, being these alkylsulphonic acid moieties the same or different from each other. By incorporating alkylsulphonic acid moieties in these catechol derivatives, in particular in R¹ and, optionally in one or more of R², R⁴, R⁵, R⁶, R⁷, R⁸, X¹, X² or X³, compounds with detergent and surfactant properties can be obtained, as well as high solubility in aqueous media can be imparted thereto. Additionally, incorporating various alkylsulphonic acid moieties, preferably using sequentially the synthetic strategy described in the present invention when the moieties are at the 3,6-positions of the ring, catechol derivatives of formula (I) can be obtained with reinforced detergent and aqueous solubility properties.

Preferably the alkyl chain present on the alkylsulphonic acid moieties is linear and n is a value comprised between 1 and 30, and still more preferably n is a value between 2 and 12. Especially preferably n is 12, to provide the compound of formula (I) with satisfactory detergent properties. Alternatively, it is also especially preferred that n is comprised between 2 and 3, since a compound of formula (I) with improved water solubility can thus be obtained.

Additionally, the compounds of formula (I) of the present invention may comprise alkylamine moieties of formula -CₙH₂ₙNR⁹R¹⁰ or alkylammonium salt of formula -CₙH₂ₙN⁺R⁹R¹⁰R¹¹ in one or more of the substituents R¹, R², R⁴, R⁵, R⁶, R⁷ and R⁸, X¹, X² or X³. When comprising more than one alkylamine moiety or alkylammonium salt, these may be the same or different from each other. The incorporation of these moieties in these catechol derivatives disclosed in the present invention, in particular in R¹ and, optionally in one or more of R², R⁴, R⁵, R⁶, R⁷, R⁸, X¹, X² or X³, allows to obtain compounds suitable for use in the preparation of coatings having bactericidal, bacteriostatic and/or detergent properties, as well as increasing their solubility in aqueous media. In particular, by incorporating various alkylamine moieties or alkylammonium salts, preferably using sequentially the synthetic strategy described in the present invention when the residues are at the 3,6-positions of the catechol ring, catechol derivatives of formula (I) with reinforced bactericidal and detergent characteristics can be obtained.

Preferably, the chain -CₙH₂ₙ- is lineal and n is comprised between 6 and 30, still more preferably n may be comprised between 10 and 22, and still more preferably between 12 and 18. In these particular embodiments of the compound of formula (I), the substituents R⁹, R¹⁰ and R¹¹ may be defined as set forth herein, both in their more general scope and in the particular embodiments defined hereinbefore. Particularly preferably, R⁹, R¹⁰ and R¹¹ are independently selected from hydrogen, C₁-C₆ alkyl and C₆-C₁₆ aryl. In particular, R⁹, R¹⁰ and R¹¹ may be independently selected from methyl, ethyl and benzyl moieties.

The compounds of formula (I) which are disclosed in the present invention may comprise, in one or more of the substituents R¹, R², R⁴, R⁵, R⁶, R⁷, R⁸, X¹, X² and X³, polyalkylene glycol moieties of formula -(CHRCH₂O)_{q}R', wherein R is independently selected from hydrogen atoms and C₁-C₁₈ alkyl moieties which may be linear or branched, and R' is independently selected from the group consisting of a hydrogen atom, an C₁-C₁₈alkyl moiety, terminal C₁-C₁₈ alkenyl, terminal C₁-C₁₈ alkynyl, -COOH, -NH₂, -N₃ or *N*-maleimido. When the catechol derivative comprises more than one polyalkylene glycol moiety, these may be the same or different from each other. The incorporation of these moieties in these catechol derivatives, in particular in R¹ and, optionally in one or more of R², R⁴, R⁵, R⁶, R⁷, R⁸, X¹, X² or X³, allows to obtain compounds suitable for use in obtaining coatings with antifouling properties and capable of biocompatibilizing substrates to which they are adhered in physiological media, optionally with reactive points suitable for *click chemistry* reactions. On the other hand, and particularly, by incorporating various moieties of the polyalkylene glycol type, preferably using sequentially the synthetic strategy described in the present invention when the moieties are at the 3,6-positions of the catechol ring, derivatives with reinforced antifouling and biocompatibility character, and optionally reactivity, can be obtained.

Preferably, R can be selected from the group consisting of an hydrogen atom and a linear alkyl chain -CₙH₂ₙ₊₁, wherein n is a value comprised between 1 and 6. Still more preferably, R is a hydrogen atom or a methyl group (i.e. n = 1), and especially preferably R is a hydrogen atom, so that the resulting chain is that of polyethylene glycol.

In a preferred embodiment, R' is selected from the group consisting of a C1-C18 alkyl moiety, linear or branched, preferably with n comprised between 1 and 10. In this case, said moiety confers, in combination with the polyalkylene glycol chain to which is linked, chemical inertia and amphiphilic properties to the molecule, i.e. surfactant character. In another preferred embodiment, R' is independently selected from the group consisting of a hydrogen atom, and especially preferably, a terminal C₁-C₁₈ alkenyl moiety, a terminal C₁-C₁₈ alkynyl moiety, - COOH, -NH₂, -N₃ or N-maleimido. In any of such cases, said moiety confers to the polyalkylene glycol chain a reactive point, and with the exception of the hydrogen atom, said point is especially suitable for the conjugation of additional moieties by *click chemistry* reactions, known in the state of the art.

In the above-mentioned preferred embodiments referred to polyalkylene glycol-type moieties, the average chain length (q) may be comprised between 2 and 1000. However, it is preferred that q has a value between 2 and 500, and still more preferably between 5 and 300.

The compounds of formula (I) of the present invention may comprise moieties incorporating a fragment capable of conferring fluorescent properties to the molecule, in one or more of the substituents R¹, R², R⁴, R⁵, R⁶, R⁷ or R⁸. The incorporation of these moieties in these catechol derivatives, in particular in R¹ and, optionally in one or more of R², R⁴, R⁵, R⁶**,** R⁷ or R⁸, allows to obtain compounds suitable for use in obtaining coatings bearing fluorescent tags or labels. In particular, by incorporating various fluorescent moieties, preferably using sequentially the synthetic strategy described in the present invention when these moieties are at the 3,6-positions of the catechol ring, derivatives with reinforced fluorescent labeling (when the fluorescent moiety is the same) or allowing dual fluorescent labeling (when generally two fluorescent moieties are of different nature) can be obtained.

The compounds of formula (I) which are disclosed in the present invention may comprise moieties derived from mono, oligo- or polysaccharide, in one or more of the substituents R¹, R², R⁴, R⁵, R⁶, R⁷ or R⁸. When comprising more than one moiety derived from mono-, oligo- or polysaccharide, said moieties may be the same or different from each other. The incorporation of these moieties in these catechol derivatives, in particular in R¹ and, optionally in one or more of R², R⁴, R⁵, R⁶, R⁷ or R⁸, allows to obtain suitable compounds for use in obtaining coatings with mucoadhesive properties, which confer on the substrate compatibility in physiological media and favorable susceptibility to cell recognition and internalization. In particular, by incorporating various moieties derived from a mono-, oligo- or polysaccharide, preferably using sequentially the synthetic strategy disclosed herein when the moieties are at the 3,6-positions of the ring, derivatives with reinforced properties can be obtained. Preferably, this moiety is 1-β-D-glucosyl.

The compounds of formula (I) which are disclosed in the present invention may comprise, independently, glutathione or oligopeptides, in one or more of the substituents R¹, R², R⁴, R⁵, R⁶, R⁷ or R⁸. When comprising more than one moiety, said moieties may be the same or different from each other. The incorporation of these moieties in these catechol derivatives, in particular in R¹ and, optionally in one or more of R², R⁴, R⁵, R⁶, R⁷ or R⁸, allows to obtain suitable compounds for use in obtaining coatings that specifically confer a favorable susceptibility to cell recognition and internalization. In particular, by incorporating various oligopeptide moieties, preferably using sequentially the synthetic strategy disclosed herein when the moieties are at the 3,6-positions of the ring, derivatives with reinforced properties can be obtained. Preferably, said moieties are glutathione, or oligopeptides consisting of a number of amino acids comprised between 5 and 25, being at least 20% of them arginine and up to a maximum of 100% of this amino acid.

In several preferred embodiments, the present invention relates to a compound of formula (I), wherein **R¹** and **R²** are independently selected from the group consisting of the following subgroups:
- (a) a linear or branched alkyl moiety of formula -CₙH₂ₙ₊₁, wherein n ranges between 1 and 30;
- (b) a linear or branched alkenyl moiety of formula -CₙH₂ₙ₋₁, wherein n ranges between 1 and 30;
- (c) a linear or branched alkynyl moiety of formula -CₙH₂ₙ₋₃, wherein n ranges between 1 and 30;
- (d) a polyfluoroalkyl moiety of formula -(CH₂)ₚ₁CₙF₂ₙ₊₁, wherein p₁ is equal to or higher than 0, and n ranges between 1 and 30;
- (e) a alkylsulphonic acid moiety of formula -CₙH₂ₙSO₃H, wherein the alkandiyl moiety is linear or branched, and n ranges between 1 and 30;
- (f) an alkylamine moiety of formula -CₙH₂ₙNR⁹R¹⁰ or alkylammonium salt of formula - CₙH₂ₙN⁺R⁹R¹⁰R¹¹, wherein the alkandiyl moiety is linear or branched, n ranges between 1 and 30, and R⁹, R¹⁰ and R¹¹ are independently selected from the group consisting of hydrogen, C₁-C₃₀ alkyl, C₅-C₂₀ aryl, a C₃-C₄₀ alicyclic moiety, a C₃-C₄₀ aralkyl moiety and a C₃-C₄₀ alkylaryl moiety;
- (g) a polyalkylene glycol chain of formula -(CHRCH₂O)_{q}R', wherein R is a substituent which is hydrogen or C₁-C₁₈ alkyl, and R' is selected from a C₁-C₁₈ alkyl, terminal C₁-C₁₈ alkenyl, terminal C₁-C₁₈ alkynyl, -COOH, -NH₂, -N₃ or *N*-maleimido, and q is a value comprised between 2 and 1000;
- (h) a C₅-C₂₀ aromatic moiety which can comprise none, one or more heteroatoms selected from the group consisting of O, N, S and a combination thereof;
- (i) a C₃-C₄₀ alicyclic moiety which can comprise none, one or more heteroatoms selected from the group consisting of O, N, S and a combination thereof;
- (j) a moiety incorporating a fragment capable of conferring fluorescent properties to the molecule;
- (k) an oligopeptide selected from glutathione and an oligopeptide consisting of 5-25 amino acids and rich in arginine;
- (I) a moiety derived from a mono-, oligo- or polysaccharide;
- (m) a R³-S-Z moiety as defined in the present invention; and
- (n) a R³-T moiety as defined in the present invention.

In certain preferred embodiments, the present invention relates to compounds of formula (I) wherein R¹ and R² are two fragments, chemically the same or different from each other, independently selected from the same subgroup as described above (and hence from same functional nature), in such a way that the functional properties that incorporate said fragments are reinforced in the same compound. Preferably R¹ and R² are identical.

In other preferred embodiments, the compounds of formula (I) have in R¹ and R² two fragments to be each selected from subgroups other than those described above (and therefore of different functional nature), such that the functional properties incorporated by said fragments are independent and complementary to each other. Preferably, the compound of formula (I) is characterized in that a substituent selected from R¹ and R² is a polyalkylene glycol moiety of formula -(CHRCH₂O)_{q}R' and the other is a fluorescent moiety, as described above. In this particular case, the incorporation of said moieties into the same compound allows the preparation of compounds suitable for the preparation of coatings compatible with physiological media and in turn for support for fluorescent labels.

In other preferred embodiments, the compounds of formula (I) have in R¹ and R² two fragments of different functional nature to be each selected from subgroups other than those described above (and therefore of different functional nature). Unlike the previous case, the simultaneous presence of these different functionalities in the same compound gives rise to a new functional property, fruit of the synergy of the two incorporated functionalities. Preferably, the compound of formula (I) is characterized in that a substituent selected from R¹ and R² is a polyalkylene glycol moiety of formula -(CHRCH₂O)_{q}R' and the other is an alkyl moiety of formula -CₙH₂ₙ₊₁, as previously described. In this particular case, the incorporation of said residues in the same compound allows the preparation of compounds with properties of nonionic surfactants.

In other preferred embodiments of the present invention, the compounds of formula (I) comprise in R¹, and optionally in R², a R³-S-Z moiety, wherein the R³ and Z moieties are bonded by a sulfur atom. Depending on the nature of R³ and Z groups, said compounds may be used as functional catecholic compounds, or as monomers or precursors in the preparation of bipodal or generally multipodal derivatives, as well as dimers, oligomers or polymers, which are also an object of the present invention.

In certain preferred embodiments, Z may be a hydrogen atom, an acetyl moiety (-COCH₃), or a -CH₂-CH₂-Y-FUNC group, wherein FUNC is selected from the group consisting of any of the moieties defined above for R¹ and R², and Y is selected from the group consisting of -O-, - COO-, -CONH-, -(CH₂)ᵣO-, being r a value comprised between 1 and 10.

In a particularly preferred embodiment, Z is a hydrogen atom or an acetyl moiety (-COCH₃). The person skilled in the art will recognize that when Z is a hydrogen atom, the resulting molecule has a terminal thiol group, which is likely to participate in nucleophilic addition, nucleophilic substitution or radical addition reactions, e.g. a reaction of the thiol-ene or thiol-ine type. Said reactions are of particular utility for the functionalization of catechol-derived structures with free thiol groups, since they allow the production of functional coatings in different applications, depending on the nature of said functional fragment. In another group of preferred embodiments, the FUNC functional fragment may be selected from the group consisting of the subgroups (a), (b), (c), (d), (e), (f), (g), (h), (i), (j), (k), (I), (m), (n) previously defined for R¹ and R². Also, when Z is an acetyl moiety, the resulting moiety is a thioacetyl functional group, which is especially useful as a protecting group and precursor of the thiol function.

The R³ moiety is selected in such a way that it can act as a link between two or more catechol units in multipodal derivatives and, optionally, as a single or additional carrier of functional chains or fragments.

The compound of formula (I) of the present invention may comprise a R³Z moiety, wherein R³ is a linear or branched alkandiyl moiety of formula -C_{n'}H_{2n'}, where n' is equal to or higher than 1. Preferably, n' is equal to or higher than 2, and even more preferably the alkandiyl moiety is linear and is n' is comprised between 2 and 18.

Additionally, the compound of formula (I) may comprise a R³Z moiety, wherein R³ is an arendiyl (-Ar-) moiety, wherein the arylene group may be any aromatic biradical, optionally with the presence of heteroatoms such as O, N, S in its structure and/or one or more substituents other than hydrogen. For example, the arendiyl moiety may be 1,3-phenylene, 1,4-phenylene, 2,3,5,6-tetrachloro-1,4-phenylene, 3,4-thiophenylene, and the like. Preferably, this moiety is 1,1'-bisphenylene-4,4'-thia (-C₆H₄-S-C₆H₄-).

The compound of formula (I) may also comprise a R³Z moiety, wherein R³ is a polyfluoroalkandiyl moiety of formula -(CH₂)ₚ₃(CF₂)ₚ₄(CH₂)ₚ₅-, wherein p₃ and p₅ are independently selected from values higher than or equal to zero, and p₅ is higher than or equal to 1. Preferably, p₄ is equal to or higher than 3, and still more preferably, p₄ is comprised between 3 and 10.

The compound of formula (I) may comprise a R³Z moiety, wherein R³ is a (polyalkylenoxy)alkyl moiety -(CHRCH₂O)_{q1}(CHRCH₂)-, wherein preferably q₁ is a value comprised between 2 and 1000, and R is selected from the group consisting of a hydrogen atom and a linear or branched alkyl chain of formula -CₙH₂ₙ₊₁, wherein n is a value comprised between 1 and 6. Preferably, q₁ is comprised between 2 and 500 and R is selected from the group consisting of hydrogen, methyl and ethyl. Still more preferably, q₁ is comprised between 2 and 300 and R is a hydrogen atom or a methyl group.

The compound of formula (I) may comprise a R³Z moiety, wherein R³ is a diarylene ether moiety of formula -Ar-O-Ar- or diarylenethioether of formula -Ar-S-Ar-, wherein the arylene (-Ar-) group may be any aromatic biradical, optionally with the presence of heteroatoms such as O, N, and/or S in its structure, and/or one or more substituents other than hydrogen. For example, 1,3-phenylene, 1,4-phenylene, 2,3,5,6-tetrachloro-1,4-phenylene, 3,4-thiophenylene, and the like. Preferably, this moiety is 1,1'-bisphenylene-4,4'-thia (-C₆H₄-S-C₆H₄-).

Additionally, the compound of formula (I) may comprise a R³Z moiety, wherein R³ is a moiety of formula -(CH₂)ᵣ₁(CHOR⁴)(CH₂)ᵣ₂(CHOR⁵(CH₂)ᵣ₃- wherein r1 and r3 are independently selected from values higher than or equal to 1, r2 is higher than or equal to 0. Preferably, r1 and r3 are equal to 1 and r2 is equal to 0. In these particular embodiments of the compound of formula (I), the substituents R⁴ and R⁵ may be defined as set forth in the present invention, both in its broader scope and in the particular embodiments defined herein.

Any of R⁴ and R⁵ may be an acyl residue obtained by the esterification reaction of the α-carboxylic acid group of an amino acid, preferably lysine, histidine, arginine, aspartic acid or glutamic acid. The incorporation of these moieties at the R⁴ and/or R⁵ positions of these catechol derivatives allows to obtain compounds suitable for use in obtaining coatings which confer on the substrate compatibility in physiological media and susceptibility favorable to cell recognition and internalization.

On the other hand, any of R⁴ and R⁵ may be a moiety which forms an activated ester group, preferably a sulphonyl derivative such as, for example, a mesyl, perfluoromesyl or tosyl group. The presence of these moieties at the R⁴ and/or R⁵ positions of the catechol derivative allows the subsequent incorporation of other functional moieties with nucleophilic reactive groups by nucleophilic substitution reactions.

Also the compound of formula (I) may comprise a moiety R³Z, wherein R³ is a moiety of formula -(CH₂)ₛ₁(CHN⁺R⁶R⁷R⁸)(CH₂)ₛ₂- or -(CH₂)ₛ₁(CHNR⁶R⁷)(CH₂)ₛ₂, wherein s1 and s2 are independently selected from values equal to or higher than 1. Preferably, s1 is equal to 1 and s2 is equal to 2. In particular embodiments of the compound of formula (I), the substituents R⁶, R⁷ and R⁸ can be defined as set forth in the present invention, both in its broader scope and in the particular embodiments defined herein.

Generally, the compound of formula (I) may comprise a R³-T moiety, wherein T is an alcohol group, or a derivative thereof in the form of an activated ester as a leaving group, such as for example a mesylate or a tosylate. The person skilled in the art will recognize that said leaving group is particularly suitable for the subsequent derivatization of the compound, for example for the inclusion of functional chains as mentioned above, by nucleophilic substitution reactions.

In particularly preferred embodiments, the monopodal compounds of formula (I) are selected from the group consisting of the compound 1a, 1b, 2a, 2b, 3, 4, 5, 6, 7, 8, 9, 10, 11, 12, 13 and 22.

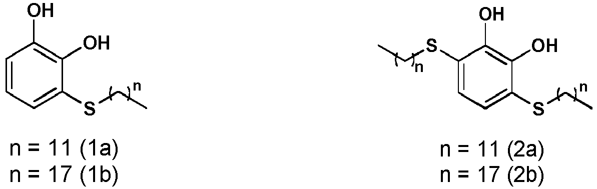

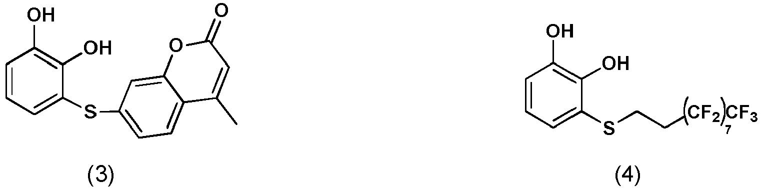

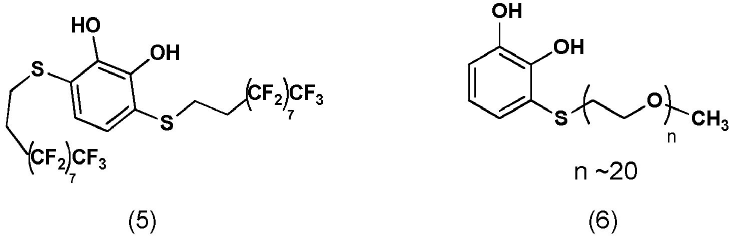

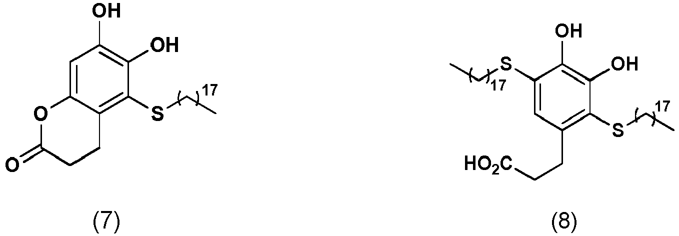

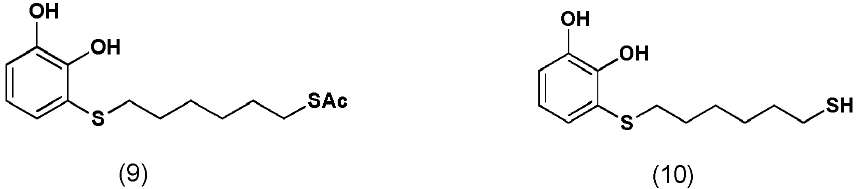

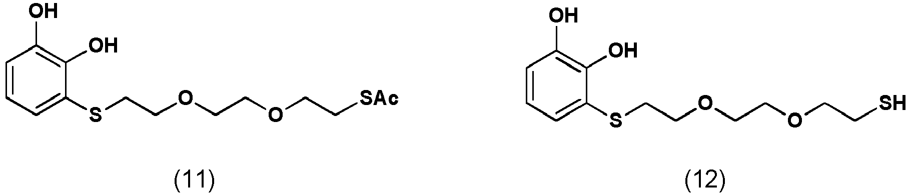

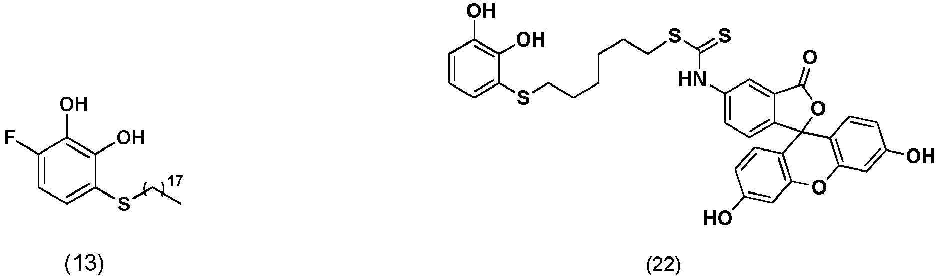

The linear monopodal compounds can be prepared following a general processology based on a sequence of reactions consisting firstly of the oxidation to the corresponding o-benzoquinone of a starting compound containing a catechol ring, followed by a reaction of *thia-*Michael type, whereby a molecule with a thiol functional group is nucleophilically, and regioselectively, added to one of the positions immediately adjacent to the carbonyl groups of o-quinone. The reaction of the thiolated derivative with the o-quinone does not require prior purification of the latter, and the addition can be made immediately after the oxidation of the catechol. Furthermore, once the covalent bond is formed, the catechol functional group, and therefore the aromaticity of the ring, is spontaneously restored by a tautomerism of the keto-enol type. Altogether, this processology allows the access to new catechol derivatives in a fast and direct way.

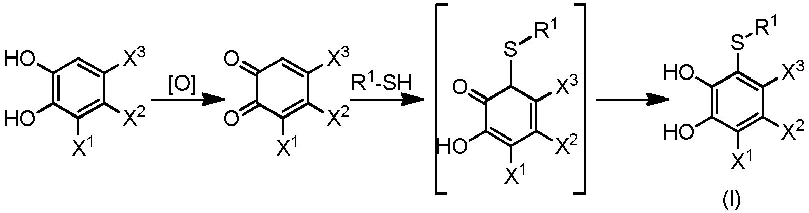

A technologically relevant aspect of the present invention is that the catechol subunit of a monopodal compound may act as a fragment with surface-adhesive properties, as well known in the art. On the other hand, a judicious choice of the functional chain R¹ allows to design the monopodal compound in such a way that it is capable of conferring the desired functional properties to the coatings prepared from said compound. In the present document the desired functional fragment is selectively incorporated into the monopodal compound by a terminal thiol group bearing molecule, which is reactive to the oxidized form of the catechol ring (o-benzoquinone), and in turn capable of providing a robust direct and efficient covalent bond, from a point of view of the atomic efficiency.

Another important advantage of this synthetic strategy is that the catechol ring is spontaneously recovered by tautomerism once the functional chain is added. For this reason, if desired, it is possible to use the monosubstituted compound as the starting material for the addition of a second functional fragment, in order to enhance the functional character of the first fragment (in the case of using fragments of similar functional nature), or to complement it (in the case of using fragments of different functional nature).

In principle, it would be expected that the application two or more times of this general processology consecutively would allow all positions of the catechol ring with an available valence to be functionalized, since all the unsubstituted positions of the ring are theoretically reactive against the *thia*-Michael addition. However, and surprisingly, the inventors have found that only positions 3 and 6 (the latter being homologous to the above with reference to the plane of symmetry perpendicular to the catecholic ring) are significantly reactive under the employed conditions. This degree of regioselectivity is high enough so that no 4- (or 5-)-substituted addition products are detected in the reaction crude as well as after the purification thereof. Therefore, the process of the present invention allows to obtain a large variety of catechol derivatives substituted at the 3-, and optionally, 6-position of the ring, in a very affordable and regioselective manner.

No other simple synthetic strategy as that disclosed herein is known for the general preparation of catechol derivatives with two functional substituents, being the same or different from each other. The processes known in the state of the art are much more complex, generally requiring various stages of protection and deprotection, as well as multistage routes to achieve double substitution. In many instances the first functional chain may be incorporated using the advantage provided by a reactive functional group, generally an aldehyde, carboxylic acid or amine, already present in the starting catechol derivative, but generally no further reactive groups are available for the incorporation of the second functional chain. In contrast to these processs, in the process disclosed herein the incorporation of the functional chains takes place taking advantage of only the reactivity of the catechol ring itself, as well as the spontaneous recovery of the catechol fragment following the *thia*-Michael reaction. Therefore, it is not necessary for the original starting catecholic compound to comprise additional functional groups, and pyrocatechol (A) may be selected for this purpose. The possibility of directly using pyrocatechol as the starting compound in the process of the present invention represents another important additional advantage, since this compound is the simplest possible catecholic molecule, is very economical and is widely available, being for this reason a particularly preferred starting catechol.

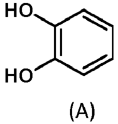

Although the monopodal compounds obtained by the process disclosed herein can be purified by chromatographic techniques for the purpose of identification, the reaction crudes, once treated simply by aqueous washing, drying and filtration, can be directly used for the preparation of effective functional coatings, which is an important advantage of this process.

In summary, the process of present invention allows to obtain directly and easily a wide variety of monopodal compounds, suitable for the preparation of coatings with a wide range of functionalities, and if desired, from a single simple, inexpensive and commercially available compound.

In a preferred synthesis strategy, the reaction of an α-thiol compound of formula HS-R¹ with one equivalent of the o-quinone of the catechol derivative used as the starting product directly generates a compound of formula (la), wherein a ring of catechol has a substituent bearing a functional fragment (-R¹) attached by a sulfur atom.

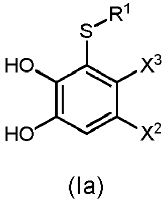

In another preferred synthesis strategy, a catechol of structure (Ia) compound is subjected to a second oxidation and nucleophilic addition process resulting in a catechol compound of structure (Ib) with two substituents bearing the functional fragments (-R¹ and -R²) attached to the catechol ring by sulfur atoms.

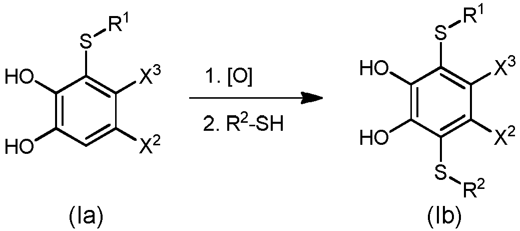

In another preferred synthesis strategy, the reaction of a compound of the α, ω-bisthiol (HS-R³-SH) type in a 1:1 molar ratio allows to obtain a catecholic derivative of formula (Ic) wherein R¹ is -R³Z and Z is a free thiol terminal group (-SH).

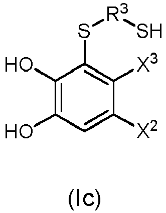

Such a catecholic compound of structure (Ic) is capable of being used in a subsequent reaction in which the free thiol group selectively reacts with another reactive molecule, which may for example be activated electrophilically, i.e. with a good leaving group for the substitution or nucleophilic addition of the thiol group. Also, said reactive molecule may have functional groups capable of selectively reacting with the thiol group by radical mechanism. In this case, the thiol group may be added in a reaction of thiol-ene or thiol-ine type to an unsaturation present in the reactive molecule. The person skilled in the art will recognize that these and other reactions are feasible for the derivatization of the monopodal compounds with all kinds of functional fragments, and in particular those described herein. In addition, the thiol-ene reaction is particularly useful, since it can be carried out selectively and in good yield without disruption of the catechol ring. Thus, the resulting compound will have a structure in which a catechol ring and a second molecular fragment are attached by a (-S-R³-S-) fragment.

In the reaction between the bis-thiol and the o-quinone as previously mentioned, it may be preferable that one of the two thiol functional groups is protected, for example, with a thioacetyl group (HS-R³-SAc). In particular, said reaction would lead to the selective formation of a catecholic compound (Id).

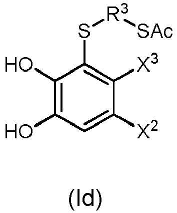

Subsequently, the hydrolysis of the compound (Id), for example under acid catalysis conditions, would lead to the deprotection of the terminal thiol group, with the consequent formation of the above-mentioned compound (Ic). The person skilled in the art will recognize that such a strategy would aim to avoid that in the course of the nucleophilic addition of the bis-thiol to the o-quinone both thiol groups react and thus to disadvantage the formation of byproducts with two catechol rings identical in structure.

Compound (Ic) may also be used in the preparation of self-condensation compounds: by virtue of the potentially complementary nature of the catechol ring and the thiol group present simultaneously in (Ic), said molecule can be used as a monomeric precursor for the preparation of catecholic homopolymers of structure (VI) by oxidation of the catechol ring and *in situ* deprotection of the thiol group. Such an operation may lead to the self-condensation of the oxidized monomer, which would lead to the formation of a polymer formed by subunits bearing a biradical of catechol and a biradical R³ of functional nature, covalently bonded by two sulfur atoms.

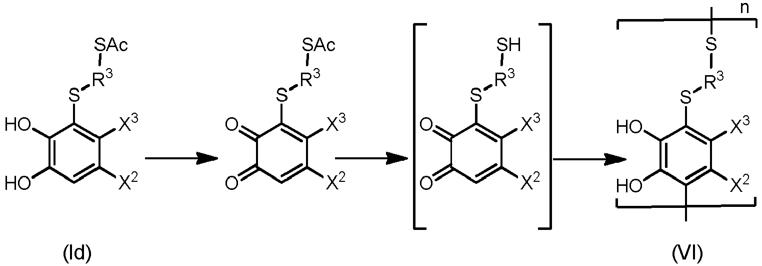

Also, the compound of formula (Id) may be used in a subsequent reaction with a third molecule, carrying a nucleophilic group capable of displacing the thioacetyl group. In this manner, the resulting compound will have a structure formed by a catechol ring and a second molecular fragment, attached by a (-S-R³-) fragment. The skilled person will recognize that both strategies are suitable for the optional incorporation of functional fragments into the catechol ring using a third molecule as carrier of said functional fragment and an intermediate or extensor fragment of the type (-S-R³-S-) or (-S-R³-), as a covalent bonding bridge between them.

In another synthetic strategy, the structure of the starting catechol compound has a blocking group, preferably a fluorine atom, in one of two positions immediately adjacent to the hydroxyl groups:

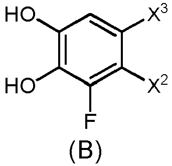

The presence of the fluorine atom in said position, along with the aforementioned regioselectivity observed for the nucleophilic addition reaction, may allow the selective production of compounds of structure (le), ensuring that said addition occurs exclusively in the other immediately adjacent position to the hydroxyl groups and therefore, no double addition by-products are generated in the reaction, even if the X ² and X ³ substituents are hydrogen atoms. Moreover, by virtue of its high electronegativity, the fluorine atom directly attached to the ring increases the oxidation potential of said ring, thus being desirable when it is desired to prioritize the resistance of the compound of formula (I) to oxidative degradation.

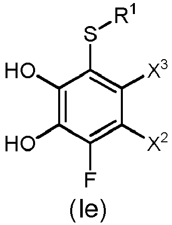

By extension, the person skilled in the art will recognize that the aforementioned synthetic strategies can be applied generally to blocked compounds of the type (B), allowing to obtain "blocked" analogs to the monomeric compounds of the type (Ib) and (Ic).

As a further example of their versatility in obtaining functional coatings, catechol derivatives of formula (I) can also be used for the preparation of polymers by other known techniques, for example, by the ammonia polymerization process disclosed in the patent application EP2589578. Although the structure of these polymers will be expected to be different from that obtained by the oxidation + *thia*-Michael sequence disclosed herein, they may also be used for the preparation of functional coatings.

### II. Linear bipodal compounds

The present invention also relates to a group of catechol derivatives of formula (I), wherein, **X¹**, **X²** and **X³** are selected, respectively, from the group of moieties as previously defined, and generally, for each of these substituents;
**R¹** is a **-R³-S-Z** moiety;
**R³** is selected from the group of moieties as previously defined, and generally, for this substituent; and,
**Z** is selected as a moiety of structure (**J***): 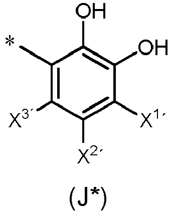 wherein,
**X^{1'}** is independently selected from the group consisting of hydrogen, a blocking group and a - SR² substituent, wherein R² has the same meaning as in the compound of formula (I), both in the broader definition and in particular or preferred embodiments disclosed herein; and,
**X^{2'}** and **X^{3'}** are independently selected from the group consisting of hydrogen and a blocking group, as previously defined and generally for compounds of the type (I).

This set of derivatives of type (I) has in its structure two catechol rings, attached by two sulfur atoms to the ends of an extender chain R³. To emphasize their linear bipodal structure, they will be represented hereinafter, and in particular, as structures of type (II):

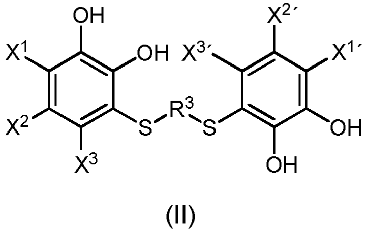

In preferred embodiments, the present invention relates to compounds of type (II), wherein **X¹**, **X^{1'}**, **X²**, **X³**, **X^{2'}** and **X^{3'}** are hydrogen atoms; and
**R³** has the same meaning as in the general compound of formula (I), both in its broader definition and in the particular or preferred embodiments disclosed in the present invention.

In particularly preferred embodiments, the bipodal compounds of formula (II) are selected from the group consisting of compound 14, 15, 16, 17, 18 and 19.

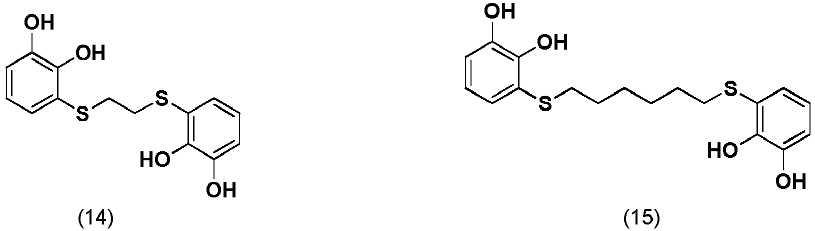

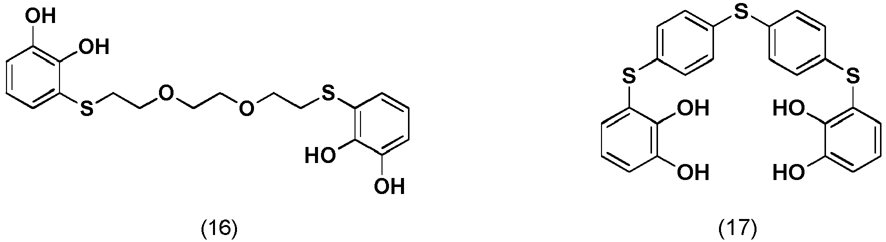

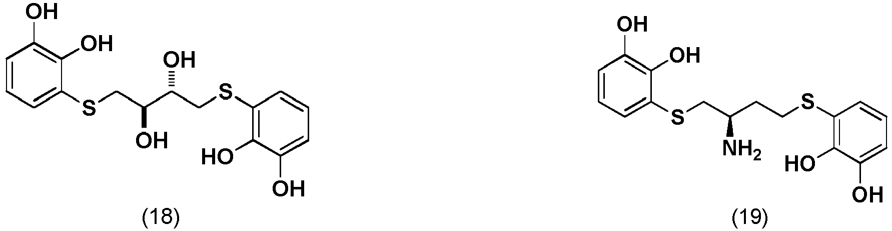

The linear bipodal compounds can be prepared following methodologies analogous to those described for monopodal compounds, that is to say, by a process comprising oxidation of starting catechol derivatives and their subsequent *thia*-Michael reaction with molecules with one or more thiol groups in their structure, without the need to isolate the o-benzoquinone intermediate obtained after the oxidation.

In a preferred synthesis strategy, the reaction with a 1:2 molar ratio between a compound having the structure of the α, ω-bisthiol type (HS-R³-SH) and an o-quinone, previously obtained by oxidation of a catechol derivative with positions adjacent to free hydroxyl groups, can directly generate a compound of formula (IIa). In this reaction, the thiol functional groups are added nucleophilically to the corresponding o-quinone molecules. This strategy is particularly useful for the preparation of linear bipodal compounds with two identical catechol fragments and R³ moieties inert to the reaction conditions.

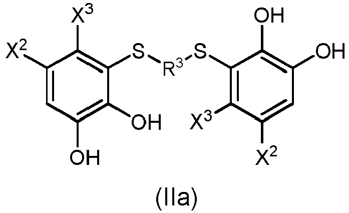

As mentioned above, the reaction in a 1:1 molar ratio between a compound having the structure of the α,ω-bisthiol type (HS-R³-SH) and an o-quinone previously obtained by oxidation of a catechol derivative with the corresponding positions adjacent to the free hydroxyl groups, allows to prepare structures of the type (Ic). The person skilled in the art will recognize that in a subsequent reaction, said compound (Ic) may react with 1 molar equivalent of the same o-quinone, or an o-quinone of different structure, to respectively generate a compound of formula (II) with two identical or different catecholic moieties.

When one of the two positions adjacent to hydroxyl are occupied by a -SR¹ moiety, as in the compounds of the type (la), such a reaction may allow the production of derivatives of the type (lib). The person skilled in the art will recognize that, alternatively, the SR¹ moieties may also be incorporated at the end of the synthetic route, i.e. to the structure (IIa), once obtained by the above-mentioned process.

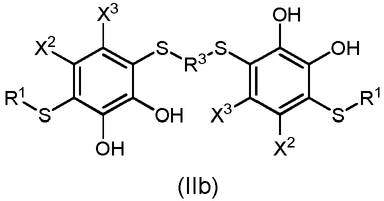

In another preferred synthetic strategy, the preparation of the linear bipodal compound (IIb) is carried out in two steps. In a first step, by reaction in a 1:1 molar ratio between α, ω-bisthiol (HS-R³-SH) and an o-quinone previously obtained by oxidation of the catechol derivative (la), a derivative catecholic of formula (If) wherein R¹ is R³-Z and Z is a free thiol terminal group can be obtained. In a second step, the reaction of this derivative with an equivalent thereof, or another o-quinone, would lead to the preparation of the corresponding linear bipodal compound of general formula (II). Although this two-stage strategy is of general applicability, it may be particularly useful for the preparation of linear bipodal derivatives with R³ groups inert to the reaction conditions, in which the functional chains (R¹ and R^{1'}) of the two catechols are required specifically to be different.

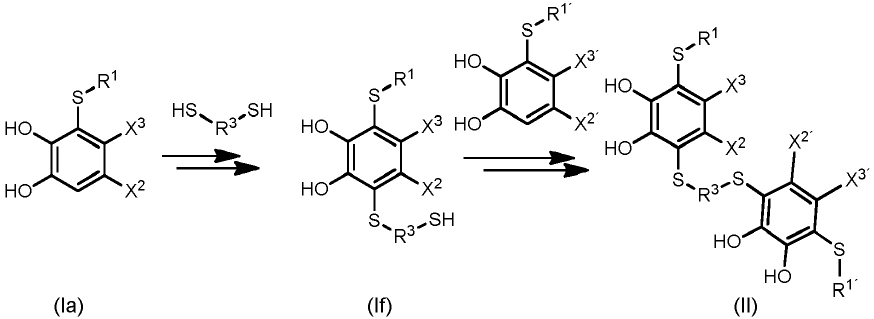

Optionally, one of the two thiol groups may be pre-protected in the starting bis-thiol, for example in the form of thioacetyl (HS-R³-SAc), to thereby disadvantage the formation of symmetrical linear bipodal byproducts during the first synthesis stage. The resulting catechol derivative (Ig) should then be deprotected prior to carrying out the second synthesis step, which would lead to the above-mentioned catechol derivative (If).

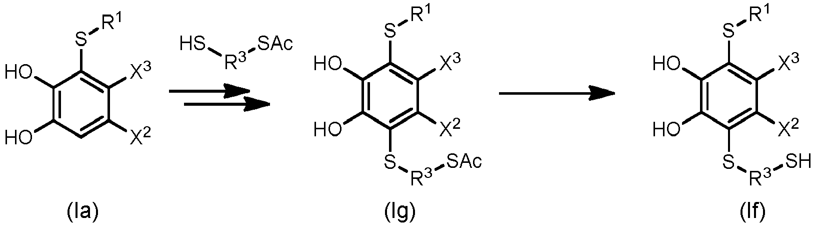

By virtue of the bis-catecholic structure of the compounds of type (IIa), such molecules can be used as co-monomers of a catecholic polymer of structure (VIIa) with an alternating co-polymer structure obtained by condensation of a second α, ω-bisthiol (HS-R^{3'}-SH) with bis-o-quinone prepared by oxidation of the corresponding bis-catechol of formula (IIa). This second α, ω-bisthiol can be selected so that its structure is equal to or different from that of α, ω-bisthiol used to construct the bis-catechol of formula (IIa), as required in order to adjust the physical-chemical and functional properties of the resulting polymer. The use of a second α, ω-bisthiol identical to the first one (i.e. R^{3'} = R³) would allow the production of a catecholic polymer of structure (VIIa).

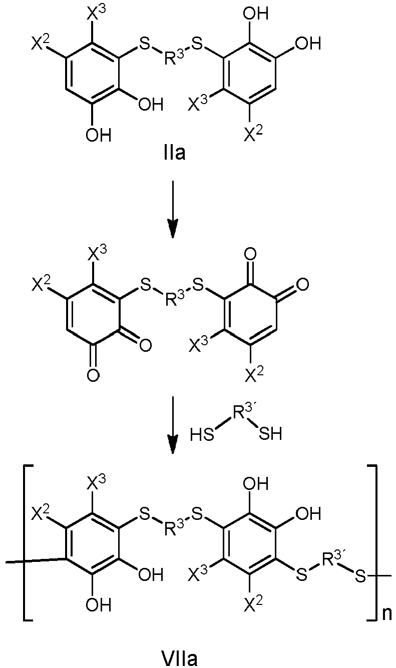

Another preferred synthesis strategy relates in particular to the use of α, ω-bisthiols, preferably those which are commercially available, carrying reactive functional groups, such as 2,3-dihydroxy-1,4-dithiol (C) and 2-aminobutan-1,4-dithiol (D):

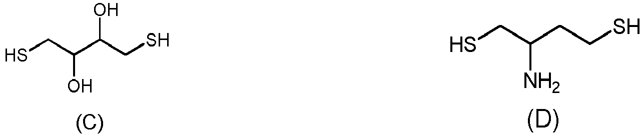

This strategy could comprise a first step, in which the protection or derivatization, at the convenience of the technologist, of the hydroxyl or amine groups would be carried out, thus obtaining the corresponding O- and N-substituted derivatives, (E) and (F), respectively.

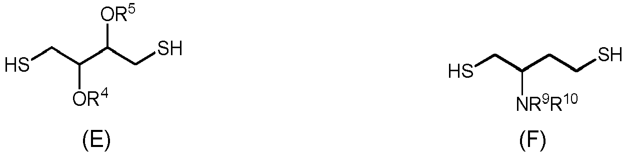

A convenient way of selectively functionalizing the hydroxyl and amino functional groups in the bis-thiols may comprise bringing the protected thiol groups as disulfide during said functionalization. In particular, the oxidized and cyclic form of 2,3-dihydroxy-1,4-dithiol, or 4,5-dihydroxy-1,2-dithiane (G), is commercially available and may be used directly in the pre-functionalization step. Subsequently, by reducing the disulfide bridge with a suitable reductant, such as a phosphine, the desired starting bis-thiol can be generated:

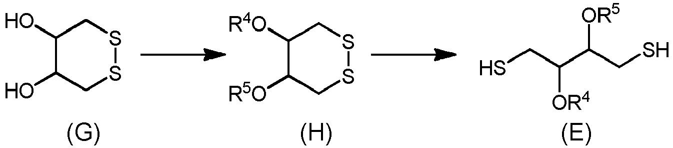

Once suitably protected or derivatized, these bis-thiols are suitable for incorporation into linear bipodal derivatives by any of the above-described strategies, to obtain compounds of formula (II) such as those listed below.

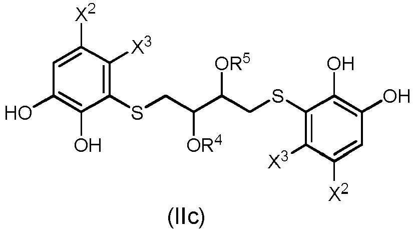

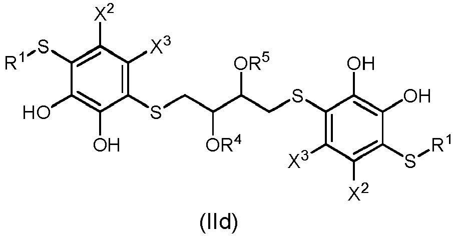

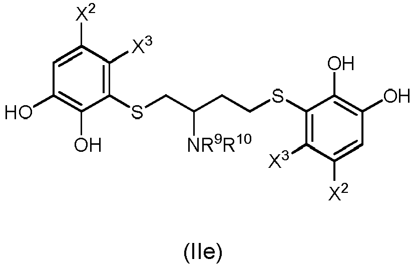

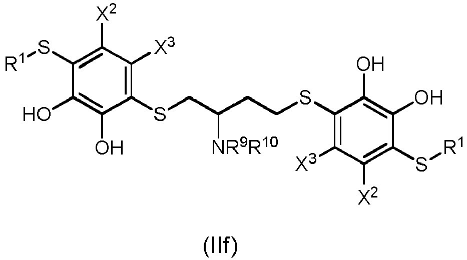

Optionally, R⁴, R⁵, R⁹, R¹⁰ and R¹¹ moieties may be selected such that they not only selectively protect the hydroxyl and amino groups under the reaction conditions of preparation of the linear bipodal compound, but also for the purpose of providing additional functionality thereto.

In the synthesis of compounds of formula (II) in which the positions X¹ and X^{1'} are free, the functionalization of said positions, if necessary or desirable to adjust the functional properties of the final compound, can be carried out at the end of the preparation of the linear bipodal skeleton, or preferably at the beginning, in order to block from the beginning said reactive positions.

### III. Compounds in 3-branch stars

The present invention also relates to a group of catechol derivatives of formula (I), wherein, **X¹**, **X²** and **X³** are selected, respectively, from the group of moieties as previously defined, and generally, for each of these substituents; and,
**R¹** is a moiety of the type **BRANCH*;** 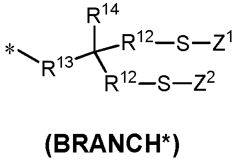 wherein,
**R¹²** is selected from the group consisting of:
   - a linear or branched alkandiyl moiety of formula -C_{n'}H_{2n'}, wherein n' is equal to or higher than 1; preferably n' is equal to or higher than 2, and still more preferably the alkandiyl moiety is linear and n' is comprised between 2 and 18;
   - a polyfluoroalkandiyl moiety of formula -(CH₂)ₚ₃(CF₂)ₚ₄(CH₂)ₚ₅-, wherein p₃ and p₅ are independently selected from values equal to or higher than a 0, and p₄ is equal to or higher than 1; preferably p₄ is equal to or higher than 3, and still more preferably, p₄ is comprised between 3 and 10;
   - a moiety of formula -(CH₂-O-**Q**-CH₂-**W**)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of -CHR'"-, and -CH(OH)CH₂-, wherein **R'"** is a hydrogen atom or a methyl group.
**R¹³** is selected from the group consisting of:
   - a linear or branched alkandiyl moiety of formula -C_{n'}H_{2n'}, wherein n' is equal to or higher than 1; preferably n' is equal to or higher than 2, and still more preferably the alkandiyl moiety is linear and n' is comprised between 2 and 18;
   - a polyfluoroalkandiyl moiety of formula -(CH₂)ₚ₅(CF₂)ₚ₄(CH₂)ₚ₃-, wherein p₃ and p₅ are independently selected from values equal to or higher than a 0, and p₄ is equal to or higher than 1; preferably p₄ is equal to or higher than 3, and still more preferably, p₄ is comprised between 3 and 10;
   - a moiety of formula -(**W**-CH₂-**Q**-O-CH₂)-, wherein **Q** is selected from the group consisting of -CO-, and -(OCₖH₂ₖ)_{q1}-, wherein k is a value comprised between 2 and 4, and q₁ is a value comprised between 2 and 300; and wherein **W** is selected from the group consisting of -CHR'"-, and -CH₂CH(OH)-, wherein **R'"** is a hydrogen atom or a methyl group.
**R¹⁴** is a hydrogen atom or a linear or branched alkyl moiety of formula -CₙH₂ₙ₊₁, wherein n is a value between 1 and 30; preferably n ranges between 1 and 6, and still more preferably R¹³ is hydrogen, methyl or ethyl; and
**Z¹ and Z²** are independently selected from the group consisting of:
   - a hydrogen atom;
   - a -COCH₃ group;
   - a moiety of structure (**J***), as previously defined for structures of type (II);
   - a moiety -CH₂-CH₂-Y-FUNC, wherein **Y** is selected from the group consisting of - O-, -COO-, -CONH-, -(CH₂)ᵣO-, being r a value comprised between 1 and 10; and FUNC is a moiety, as previously defined in the general case;
   - a moiety of structure **FUNC,** as previously defined.

This set of derivatives of type (I) has in its structure one, two or three rings of catechol, attached to a central structure in the form of a star of three branches. To emphasize the particular shape of said structure, they will hereinafter be represented, in particular, as structures of the type (III):

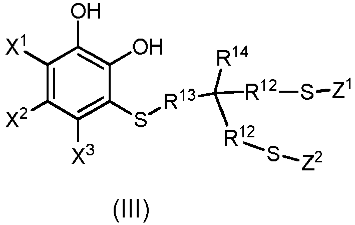

In preferred embodiments, the present invention relates to compounds of structure (III), wherein:
**X¹**, **X² and X³** are hydrogen atoms;
**R¹²** is a moiety of formula -(CH₂-O-**Q**-CH₂-**W**)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of -CHR'"-, and -CH(OH)CH₂-, wherein **R'"** is a hydrogen atom or a methyl group;
**R¹³** is a moiety of formula -(**W**-CH₂-**Q**-O-CH₂)-, wherein **Q** is selected from the group consisting of -CO-, and -(OCₖH₂ₖ)_{q1}-, wherein k is a value comprised between 2 and 4, and q₁ is a value comprised between 2 and 300; and wherein **W** is selected from the group consisting of -CHR'"-, and -CH₂CH(OH)-, wherein **R'"** is a hydrogen atom or a methyl group;
**R¹⁴** is a hydrogen atom, a methyl moiety or an ethyl moiety; and
**Z¹ and Z²** are independently selected from the group consisting of:
   - a hydrogen atom;
   - a -COCH₃ group;
   - a moiety of structure (**J***), as previously defined for structures of type (II);
   - a moiety -CH₂-CH₂-Y-FUNC, wherein **Y** is selected from the group consisting of - O-, -COO-, -CONH-, -(CH₂)ᵣO-, being r a value comprised between 1 and 10; and FUNC is a moiety, as previously defined in the general case;
   - a moiety of structure **FUNC,** as previously defined.

Depending on the nature of the substituents Z¹ and Z², the resulting star compounds of formula (III) may be mono- (Z¹ and Z² other than **J***), bi- (Z¹ or Z² of the type **J***) or tripodal (Z¹ and Z² of the type **J***).

In particularly preferred embodiments, the compounds of formula (III) are the compounds 20 and 21:

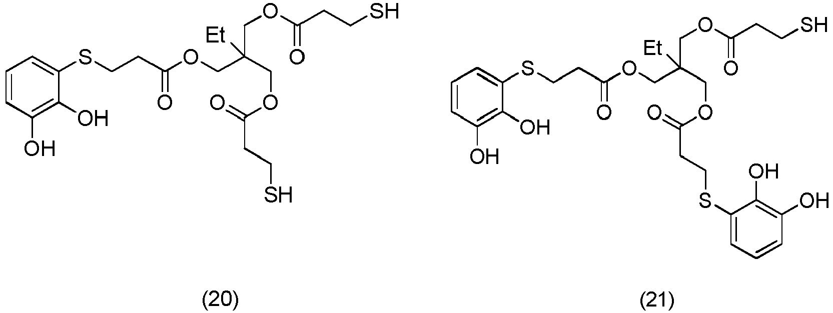

### IV. Compounds in 4-branch star

The present invention also relates to a group of catechol derivatives of formula (I), wherein, **X¹**, **X²** and **X³** are selected, respectively, from the group of moieties as previously defined, and generally, for each of these substituents; and
**R¹** is a moiety of the type **BRANCH*;** 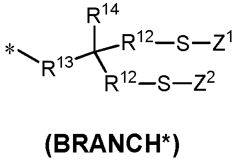 wherein,
**R¹²** is selected from the group consisting of:
   - a linear or branched alkandiyl moiety of formula -C_{n'}H_{2n'}, wherein n' is equal to or higher than 1; preferably n' is equal to or higher than 2, and still more preferably the alkandiyl moiety is linear and n' is comprised between 2 and 18;
   - a polyfluoroalkandiyl moiety of formula -(CH₂)ₚ₃(CF₂)ₚ₄(CH₂)ₚ₅-, wherein p₃ and p₅ are independently selected from values equal to or higher than 0, and p₄ is equal to or higher than 1; preferably p₄ is equal to or higher than 3, and still more preferably, p₄ is comprised between 3 and 10;
   - a moiety of formula -(CH₂-O-**Q**-CH₂-**W**)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of -CHR'"-, and -CH(OH)CH₂-, wherein **R'"** is a hydrogen atom or a methyl group.
**R¹³** is selected from the group consisting of:
   - a linear or branched alkandiyl moiety of formula -C_{n'}H_{2n'}, wherein n' is equal to or higher than 1; preferably n' is equal to or higher than 2, and still more preferably the alkandiyl moiety is linear and n' is comprised between 2 and 18;
   - a polyfluoroalkandiyl moiety of formula -(CH₂)ₚ₅(CF₂)ₚ₄(CH₂)ₚ₃-, wherein p₃ and p₅ are independently selected from values equal to or higher than 0, and p₄ is equal to or higher than 1; preferably p₄ is equal to or higher than 3, and still more preferably, p₄ is comprised between 3 and 10;
   - a moiety of formula -(**W**-CH₂-**Q**-O-CH₂)-, wherein **Q** is selected from the group consisting of -CO-, and -(OCₖH₂ₖ)_{q1}-, wherein k is a value comprised between 2 and 4, and q₁ is a value comprised between 2 and 300; and wherein **W** is selected from the group consisting of -CHR'"-, and -CH₂CH(OH)-, wherein **R'"** is a hydrogen atom or a methyl group.
**R¹⁴** is a **-R¹²-S-Z³** moiety, wherein **Z³** is independently selected from the same group as Z¹ and Z²; and
**Z¹ and Z²** are independently selected from the group consisting of:
   - a hydrogen atom;
   - a -COCH₃ group;
   - a moiety of structure (**J***), as previously defined for structures of type (II);
   - a moiety -CH₂-CH₂-Y-FUNC, wherein **Y** is selected from the group consisting of - O-, -COO-, -CONH-, -(CH₂)ᵣO-, being r a value comprised between 1 and 10; and FUNC is a moiety, as previously defined in the general case;
   - a moiety of structure **FUNC**, as previously defined.

This set of derivatives of the type (I) has in its structure one, two, three or four rings of catechol, attached to a central structure in the form of a star with four branches. To emphasize the particular shape of said structure, they will hereinafter be represented, and in particular, as structures of type (IV):

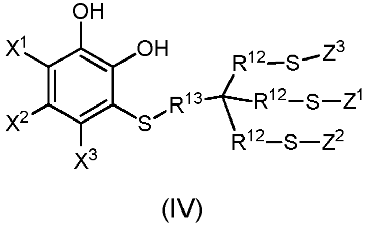

In preferred embodiments, the present invention relates to compounds of structure (IV), wherein:
**X¹**, **X² and X³** are hydrogen atoms;
**R¹²** is a moiety of formula -(CH₂-O-**Q**-CH₂-**W**)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of -CHR'"-, and -CH(OH)CH₂-, wherein **R'"** is a hydrogen atom or a methyl group;
**R¹³** is a moiety of formula -(**W**-CH₂-**Q**-O-CH₂)-, wherein **Q** is selected from the group consisting of -CO-, and -(OCₖH₂ₖ)_{q1}-, wherein k is a value comprised between 2 and 4, and q₁ is a value comprised between 2 and 300; and wherein **W** is selected from the group consisting of -CHR'"-, and -CH₂CH(OH)-, wherein **R'"** is a hydrogen atom or a methyl group;
**R¹⁴** is a **-R¹²-S-Z³** moiety, wherein **Z³** is independently selected from the same group as Z¹ and Z²; and
**Z¹ and Z²** are independently selected from the group consisting of:
   - a hydrogen atom;
   - a -COCH₃ group;
   - a moiety of structure (**J***), as previously defined for structures of the type (II);
   - a moiety -CH₂-CH₂-Y-FUNC, wherein **Y** is selected from the group consisting of - O-, -COO-, -CONH-, -(CH₂)ᵣO-, being r a value comprised between 1 and 10; and FUNC is a moiety, as previously defined in the general case;
   - a moiety of structure **FUNC,** as previously defined.
   -

Depending on the nature of the substituents Z¹, Z² and Z³, the resulting star compounds of formula (IV) may be mono- (Z¹, Z² and Z³ other than **J***), bi- (Z¹, Z² and Z³ of the type **J***), tripodal (any two of the three moieties Z¹, Z² and Z³ of the type **J***), or tetrapodal (the three moieties Z¹, Z² and Z³ of the type **J***).

In particularly preferred embodiments, the compounds of formula (IV) are the compounds 24 and 25:

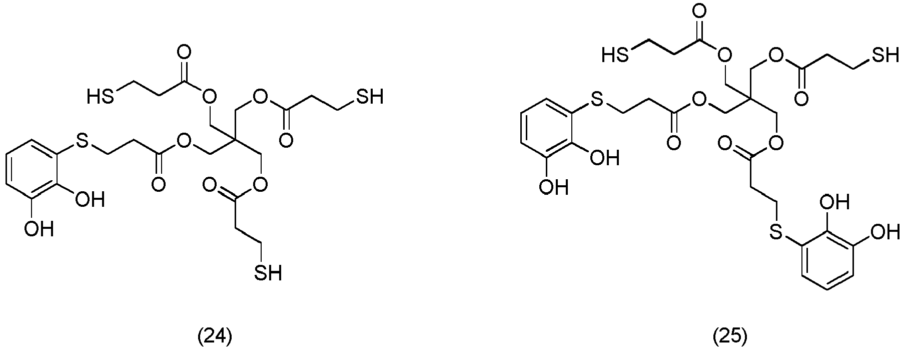

As with the rest of the general compounds of formula (I), the incorporation of the catechol rings into the structure of the branched compounds of the mono- or multipodal type of formula (III) and (IV) can be carried out by reaction of one or more compounds of the o-benzoquinone type, in this case with a branched molecule in star shape with three or four thiol functional groups at the ends (L and M, respectively). As described above, the o-benzoquinones can be obtained by oxidation of the corresponding catechol derivatives.

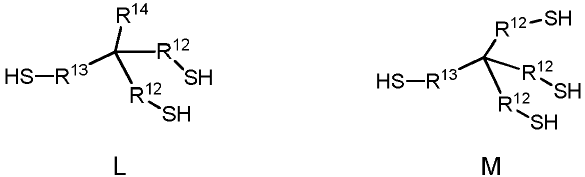

There exist on the market different branched molecules in star shape with terminal thiol groups which are particularly useful for the preparation of compounds of the type (III) and (IV), among which pentaerythritol tetrakis-(3-mercaptopropionate) (CAS No 7575-23-7), trimethylolpropane tris-(3-mercaptopropionate) (CAS No. 33007-83-9), and polythiols such as Capcure® 3-800 and Karenz® MT-PE1. All these compounds are commonly used as curatives agents for epoxy resins. The person skilled in the art will recognize that, in general, any branched molecule having a terminal thiol group in three or more of its branches is suitable for the preparation of compounds of the type (III) and (IV) in particular and, in general, of compounds with simultaneous presence of reactive thiol groups and one or more catechol rings attached to the branched structure by an arylether bridge at the 3-position of the aromatic ring, as well as derivatives in which all terminal thiol groups have been replaced with the corresponding catecholic moieties. In all of them, the choice of stoichiometry of the reaction between o-benzoquinone and tris- or tetrakis-thiol enables the number of catechol units successively incorporated into the branched structure to be controlled up to a maximum of three or four, respectively.

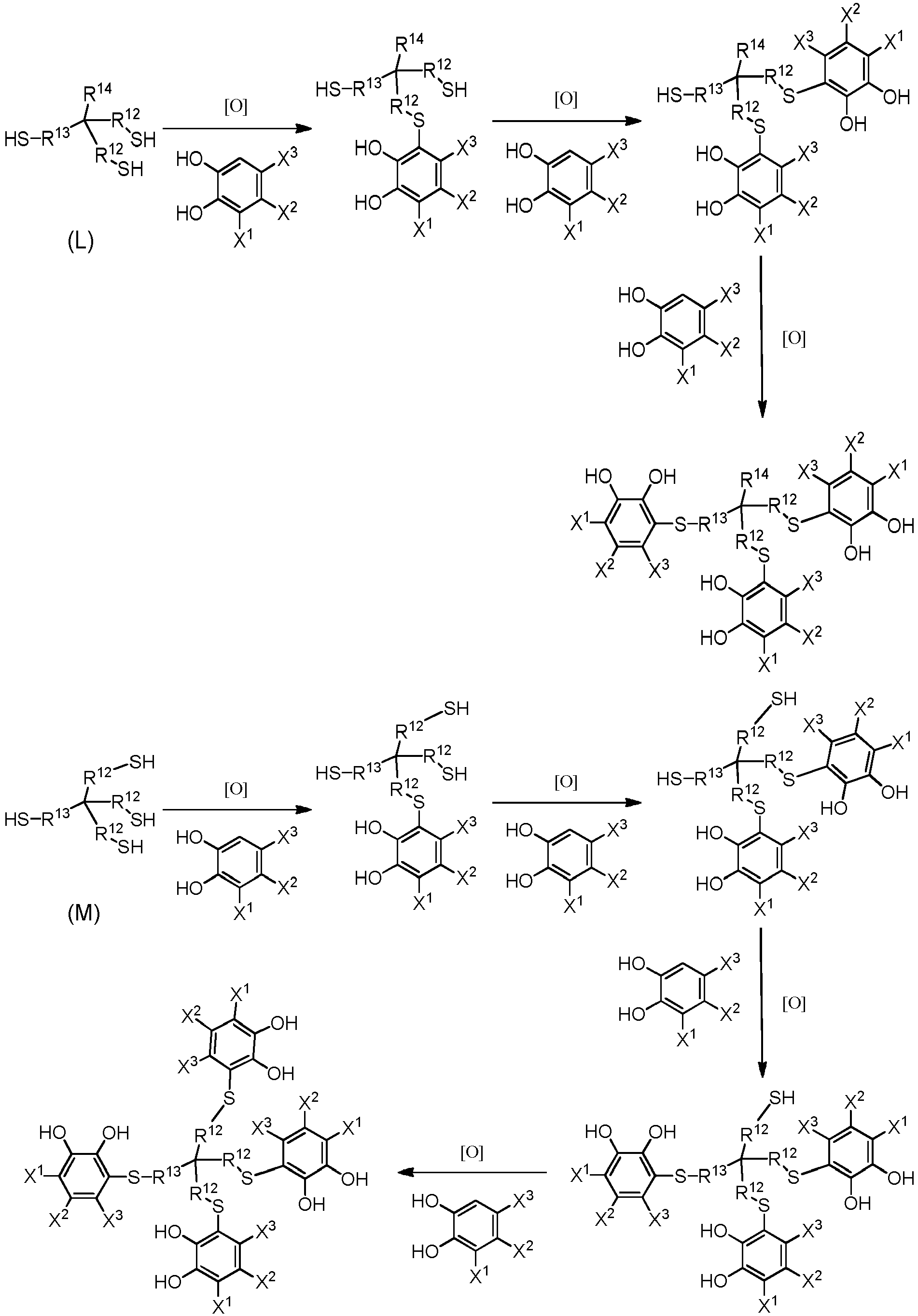

The preparation of functional catecholic compounds of types (III) and (IV) can be carried out by reaction, respectively, between a compound of type (III) or of type (IV) with m free thiol groups present, being 1 <= m <= 2 for those of the type (III) and 1 <= m <= 3 for those of the type (IV), and a molecule which has a functional chain with a group selectively reactive to thiols, such as for example, and not exclusively, a double or triple bond (by radical reaction of thiol-ene type), a good leaving group, such as for example a halogen, mesyl or tosyl (by nucleophilic substitution reaction) or an electrophilic group capable of nucleophilic addition, such as, for example, an isothiocyanate or an N-substituted maleimide.

Alternatively, the compounds of formula N or P, respectively, can be obtained firstly by means of a direct reaction in the desired stoichiometry between a compound of formula L or M and a reactive molecule which allows the coupling of a FUNC functional moiety due to the presence of an electrophile group, a leaving group or thiol-reactive group.

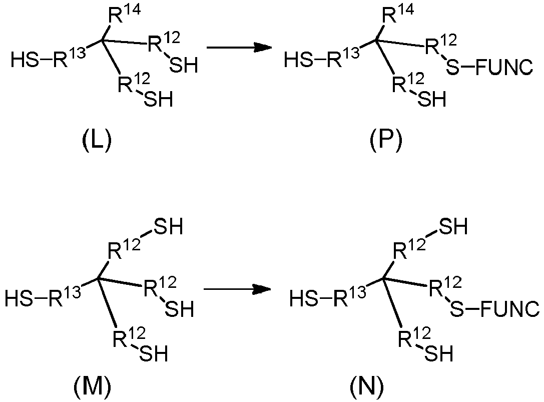

Following this synthetic route, in a second reaction step one or more catecholic rings would be incorporated into the structure by the addition reaction to o-benzoquinones as described above, being the final product a catecholic compound of type (III) or (IV), with one or more FUNC functional chains supported on any of the branches which previously had free thiol groups.

By way of example, and given its particular relevance in the preparation of functional polymers derived from compounds of the type (III) and (IV) which are disclosed below herein, the production of catecholic compounds of the type (IVb), substituted with a FUNC functional chain, can be carried out, generally, via the two alternative routes described above:

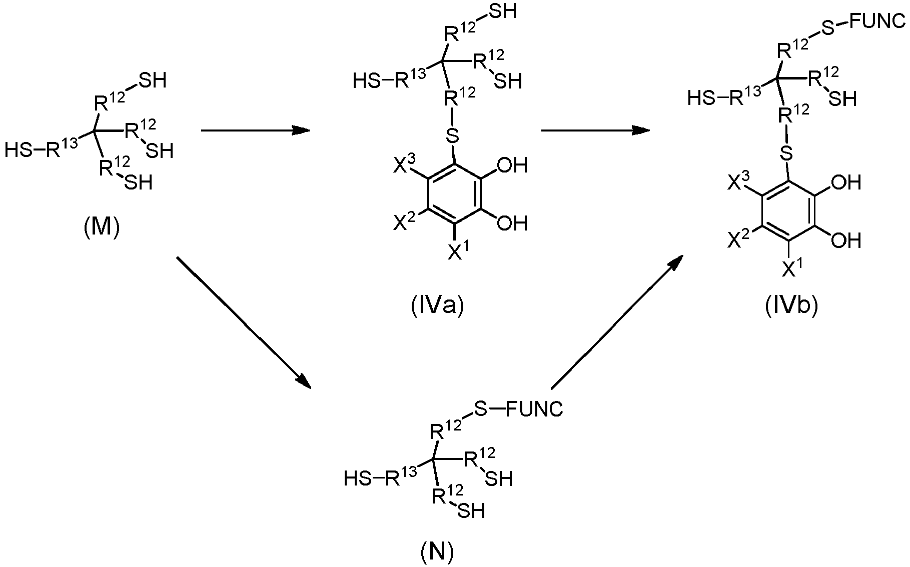

In particularly preferred embodiments, the compounds of formula (IVb) are the compound 31 (*k∼* 40 - 50) and the compound 32:

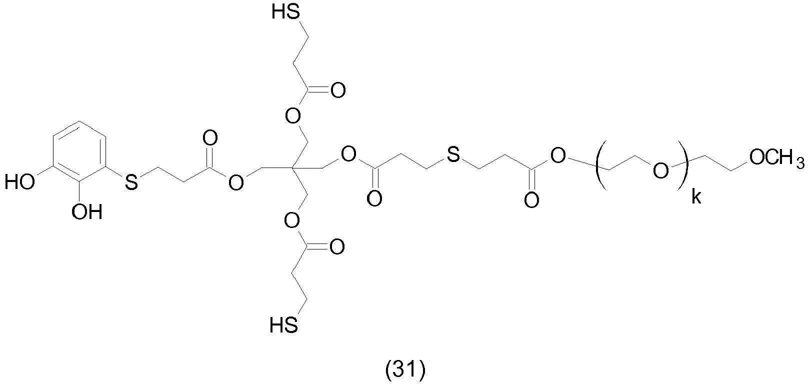

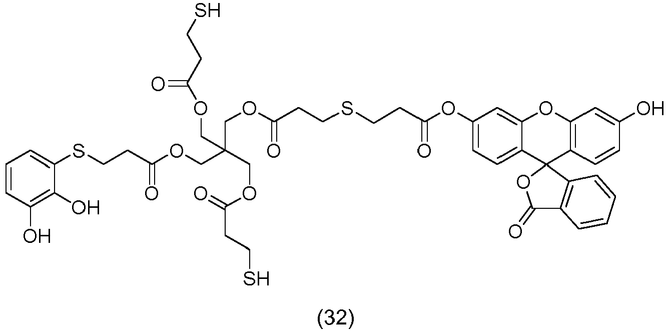

### V. Polymers and uses.

A further aspect of the present invention relates to a polymeric catecholic compound obtained by the condensation of at least one compound of the type (III) or of the type (IV), preferably a compound of the type (III) or a compound of the type (IV) 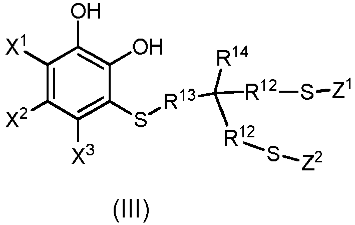 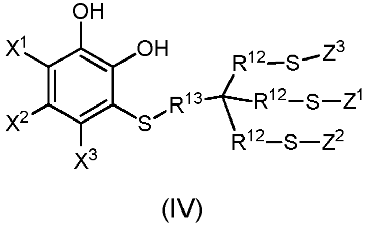
wherein Z¹ and Z² are hydrogen atoms;
X¹, X², X³, R¹², R¹³, R¹⁴ and Z³ are as defined previously according to the first aspect of the invention;
the degree of polymerization is comprised between 2 and 10,000; and the molar fraction of said catecholic compound of the type (III) or of the type (IV) is between 0.01 and 1.

When reference is made to said "polymeric catecholic compound" according to the present invention it is understood that it is formed from a monomer of formula (III), a monomer of formula (IV) or a combination thereof, provided that each of these monomers has at least two thiol groups.

In this way, it would be possible to obtain the following polymer structures.
- Homopolymers based on monomers of the type (III);
- Homopolymers based on monomers of the type (IV);
- Heteropolymers of two or more different monomers of the type (III);
- Heteropolymers of two or more different monomers of the type (IV);
- Combinations of one or more monomers of the type (III) with one of the monomers of the type (IV)
- Combinations of one or more monomers of the type (III) and/or (IV) with other co-monomers (including non-catecholic) with which they can be condensed through the thiol function. Possible non-catecholic co-monomers can condense through thiols or through any other thiol-reactive functional group.

As it will be recognized by one skilled in the art, compounds of formula (III) or (IV) are monomers or precursors, but exactly as such they are not in the polymer. The precursor of the polymers described herein will be at least one compound of the type (III) or (IV) having at least two thiol groups, whereas the corresponding constituent unit in the polymer would instead have the corresponding functional groups due to the oxidative coupling of a thiol group of the monomer (III) or (IV) with a thiol group of another monomer or a co-monomer (disulfide bridge), or due to the nucleophilic substitution or addition to an electrophilic co-monomer reactive to thiol (thioether bridge) or due to a radical addition to co-monomer with double or triple bonds (thioether bridge) depending on the chemical nature of the remaining reactive monomers.

A further aspect of the present invention is the use of catecholic compounds of general structure (I), as well as of a polymeric catecholic compound as previously defined, for the preparation of a functional coating.

A further aspect of the present invention is the use of a previously defined catecholic polymeric compound for the preparation of an adhesive substance.

In order to obtain catecholic polymers by condensation reactions using at least one compound of the type (III) or (IV), the presence of at least two thiol groups in its structure is required. Aside from homopolymers obtained by self-condensation of the corresponding monomer of the type (III) or (IV), the skilled person will recognize that it is also possible to obtain heteropolymers by cross-condensation of a compound of the type (III) or (IV) containing two free thiol groups, with one or more additional co-monomers, provided that each of these has at least in its structure either two terminal thiol groups or two thiol-reactive groups. Optionally, one or more of these co-monomers may have one or more catechol rings. Optionally, any of the constituent monomers of the final polymer structure may have functional chains in their structure, if desired. Together, the incorporation of co-monomers allows, in the case of the polymers object of this invention, the regulation of the degree of crosslinking of the polymer, the ratio of catechol rings and/or, optionally, of the functional chains, as well as the separation of moieties with adhesive properties (catechol rings) and functional chains in different co-monomers.

In a preferred synthesis strategy, condensation by oxidative polymerization of a catechol compound of the type (III) or (IV) with two or more free thiol groups with itself, or with one or more additional co-monomers with two or more free thiol groups makes it possible, respectively, to obtain condensation homopolymers and heteropolymers. By virtue of this oxidation reaction, the thiol groups are oxidatively coupled in pairs forming bridges of the disulfide type. An particularly suitable oxidizing agent for the preparation of such catecholic polymers is molecular iodine, which has the important advantage of being a thiol group selective oxidant in the presence of catechol groups, said oxidative coupling being carried out in a clean and efficient manner. This selectivity is particularly relevant because it makes the steps of protection and subsequent deprotection of the catechol ring unnecessary.

In order to emphasize the versatility of the design of catecholic polymers based on disulfide bridges, and without being in any way limiting examples, those skilled in the art will recognize that as additional co-monomers non-catecholic polythiolated compounds of the structure of type (L) or (N) can be used, as well as non-catecholic polyols previously functionalized, such as those of the type (N) or (P), in case it is desirable to incorporate functional chains into the final polymer. On the other hand, the use of a compound of the type (IV) with three free thiols as the additional co-monomer allows to provide some degree of cross-linking to the final polymer structure. When it is desirable to confer multifunctional character on the polymer, it suffices to effect the condensation of two or more monomers with different functional chains. Also, the functional content of the final polymer may be adjusted by combining functionalized and non-functionalized monomers in suitable stoichiometry. Finally, it is possible to use α,ω-bis thiols with extender chains (HS-R³-SH) as co-monomers, in order to adjust the mechanical and/or functional properties of the polymer. The person skilled in the art will recognize that it is possible to use these and other combinations of co-monomers for the preparation of catecholic polymers by direct condensation of free thiol groups, without departing from the scope of the present invention, provided that at least one of the monomers is a compound of the type (III) or of the type (IV), which has at least two free thiol groups in its structure.

In another preferred synthetic strategy, it is possible to obtain alternating polymers by condensation between monomers of the structure of type (III) or (IV) with two or more free thiol groups, and co-monomers having two or more thiol-reactive functional groups, such as, for example, isocyanate groups, N-substituted maleimides, leaving groups such as mesyl or tosyl, and terminal double or triple bonds, generally susceptible to nucleophilic addition, nucleophilic substitution or radical addition (thiol-ene, thiol-ine) by reaction with the thiol group. By way of example, the person skilled in the art will recognize the use of compounds having three terminal thiol-reactive functional groups, such as tris[2-(3-mercaptopropionyloxy)ethyl] isocyanurate, 1,3,5-triallyl-1,3,5-triazin-2,4,6(1H,3H,5H)-trione or trimethylolpropane methacrylate as polythiols crosslinking agents in condensation reactions of thiol-ene type.

To the extent that both the catecholic monomers of the type (III) and (IV) and the thiol-reactive co-monomers may optionally incorporate functional chains, and in turn generate structures with a varying degree of cross-linking and flexibility, this synthesis strategy allows a versatility similar to the condensation by disulfide bridges, with the relevant difference that the resulting final products are necessarily alternating co-polymers. The person skilled in the art will recognize that it is possible to use these and other combinations of co-monomers for the preparation of catecholic polymers by condensation of thiol groups with thiol-reactive groups without departing from the scope of the present invention, provided that at least one of the monomers is a compound of the type (III) or of the type (IV), which has at least two free thiol groups in its structure.

The optional presence of functional chains in the compounds of general structure (I), as well as in the polymers containing them, makes it possible to use such materials for the preparation of functional coatings of substrates. Depending on the functional chain(s) selected for the constituent monomer(s) of the catecholic polymer, it is possible to obtain a coating with one or more functionalities selected from the group consisting of hydrophobicity, oleohydrophobicity, solubility in aqueous media, compatibility in physiological media, solubility in organic solvents of medium or low polarity, bacteriostatic properties, bactericidal properties, *antifouling* properties, detergent capacity, surfactant capacity, fluorescence, improved cell recognition and nternalization and mucoadhesivity.

The presence of catechol rings in the polymers object of the present invention allows to obtain materials with adhesive properties for joining substrates.

Hereinafter, a number of examples are provided which are intended only to illustrate the invention without the invention being limited thereto.

### EXAMPLES

### A. Synthesis of monopodal catechol compounds

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone by the addition of dichloromethane (DCM, 4x8 mL) was carried out. Thereafter, it was dried over anhydrous sodium sulfate and filtered. On the other hand, a solution of the corresponding thiol (1 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the thiol, further protecting the mixture from light and leaving it under magnetic stirring under an inert atmosphere for six hours. After this time, both the solvent and the TFA were removed in vacuo to give the reaction crude which was purified using a chromatography column in order to separate mainly the mono- and di-substituted compounds from the remainder of impurities.

### Example 1.1: Synthesis of the compounds 1b and 2b:

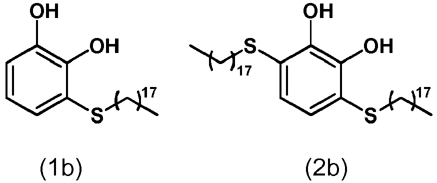

In a 250 mL volume bottom flask, a solution of 234 mg of NalO₄ (1 mmol) in 38 mL of H₂O was prepared and cooled in an ice bath. 110 mg of pyrocatechol (1 mmol) dissolved in 1 mL of Et₂O was then added and left stirring vigorously for 15 min. After this time, extraction of the corresponding formed quinone by the addition of dichloromethane (DCM, 4x8 mL) was carried out. Thereafter, it was dried over anhydrous sodium sulfate and filtered. On the other hand, a solution of 268 mg of 1-octadecanethiol (1 mmol) in 2 mL of dichloromethane was prepared in an inert atmosphere in a Schlenk flask. To this solution 229.5 µl of trifluoroacetic acid (TFA, 3 mmol) was further added. Finally, the solution of the o-quinone in DCM was added to the solution containing the thiol, further protecting the mixture from light and leaving it under magnetic stirring under an inert atmosphere for six hours. After this time, both the solvent and the TFA were removed under vacuum to obtain a reaction crude with a mixture 5:1 of the mono-substituted (1b) and di-substituted (2b) products, respectively. The product mixture was purified using a chromatography column with a 9/1 hexane/ethyl acetate eluent mixture in a total yield of 77% (64.2% of 1b and 12.8% of product 2b). Characterization of **1b**: ¹H-NMR (360 MHz, CDCl₃) δ 7.00 (dd, *J*₁ = 1.60 Hz, *J*₂ = 7.89, 1H), 6.91 (dd, *J*₁ = 1.60 Hz, *J*₂ = 7.79, 1H), 6.78 (t, *J* = 7.89 Hz, 1H), 2.70 (t, *J* = 7.57, 2H), 1.55 (q, *J* = 7.28 Hz, 2H), 1.49-1.22 (m, 30H), 0.88 (t, *J* = 6.38 Hz, 3H); ¹³C-NMR (360 MHz, CDCl₃) δ 144.15, 143.77, 126.62, 120.72, 119.26, 116.21, 36.82, 31.96, 29.72, 29.68, 29.64, 29.57, 29.49, 29.38, 29.12, 28.60, 22.71, 14.13; MS/ESI- [M-H]⁻ 393.284 Da. Characterization of **2b**: ¹H-NMR(250 MHz, CDCl₃)δ 6.92 (s, 2H), 2.76 (t, *J* = 7.52, 4H), 1.58 (q, *J* = 7.37 Hz,4H), 1.45-1.13 (m, 60H), 0.88 (t, *J* = 6.84 Hz, 6H); ¹³C-NMR(250 MHz, CDCl₃)δ 143.37, 124.69, 120.60, 35.43, 31.58, 29.69, 29.68, 29.64, 29.58, 29.55, 29.50, 29.37, 29.14, 28.67, 22.69, 14.08; MS/ESI- [M-H]⁻ 677.536 Da.

### Example 1.2: Synthesis of compound 6:

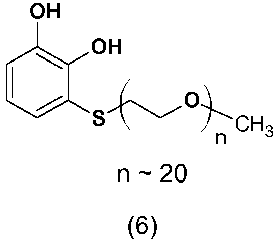

In a 50 mL volume bottom flask, a solution of 117 mg of NalO₄ (0.5 mmol) in 20 mL of H₂O was prepared and cooled in an ice bath. 55 mg of pyrocatechol (0.5 mmol) dissolved in 0.5 mL of Et₂O was then added and left under vigorous stirring for 15 min. After this time, extraction of the corresponding formed quinone by the addition of dichloromethane (DCM, 4 x 8 mL) was carried out. Thereafter, it was dried over anhydrous sodium sulfate and filtered. On the other hand, a solution of 400 mg of poly(ethylene glycol)methyl ether thiol (0.5 mmol) in 1 mL of dichloromethane was prepared in an inert atmosphere in a Schlenk flask. To this solution 115 µL of trifluoroacetic acid (TFA, 1.5 mmol) was further added. Finally, the solution of the o-quinone in DCM was added to the solution containing the thiol, further protecting the mixture from light and leaving it under magnetic stirring under an inert atmosphere for six hours. After this time, both the solvent and the TFA were removed in vacuo to obtain the reaction crude. The product mixture was purified using a chromatography column with a 8/2 ethyl acetate/methanol eluent mixture in a total yield of 40%. Characterization of **6**: ¹H-NMR (360 MHz, CDCl₃) δ 6.90 (dd, *J₁* = 1.4 Hz, *J*₂= 7.8 Hz, 1H), 6.85 (dd, *J₁* = 1.5 Hz, *J*₂= 7.9 Hz, 1H), 6.66 (t, *J*= 7.9 Hz, 1H), ∼4.70 (broad), 3.60 (s, >70H), 3.50 (t, *J* = 5.7 Hz, 2H), 2.90 (t, *J* = 5.9 Hz, 2H); ¹³C-NMR (400 MHz, CDCl₃) δ 145.77, 145.18, 126.49, 120.53, 119.12, 116.72, 70.54, 69.04, 59.19, 35.98; MS/ESI+ [M+NH₄]⁺ 1054.581 Da; [M+Na]⁺ 1059.539 Da, corresponding to n = 20, together with several satellite peaks corresponding to n around 20.

### Example 2: Synthesis of compounds of formula (I) wherein R¹ is R³Z, and Z is thioacetate

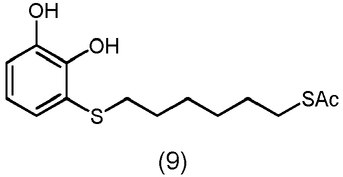

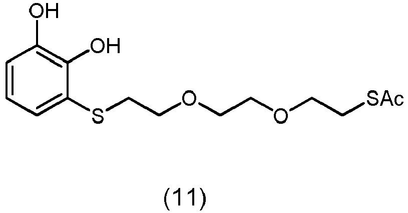

### Example 2.1: Synthesis of compound 11

Synthesis of the monoacetylated dithiol was firstly carried out. To this end, 3 mmol of the corresponding dithiol (547 mg of 2,2'-(ethylenedioxy)diethanethiol for the synthesis of molecule 11) was dissolved in 17 mL of DCM in a round bottom flask. Thereafter, 17 mL of pyridine and 282 µl of acetic anhydride (1.5 mmol) were added stirring at room temperature for at least 15 hours. After this time, the solvent was removed in vacuo and extraction was effected with several washes adding water in order to remove the pyridine. There was thus obtained a product mixture comprising 71% the desired monoacetylated product, 27% the diacetylated product (without free thiol), and in ≤2% the dithiol of the starting material. This mixture was used without further purification to carry out the Michael reaction, considering the percentage of purity of the monoacetylated product, and taking into account that of the formed by-products, the diacetylated compound is inert to the nucleophilic addition, and the dithiolated compound is found in residual proportion.

For Michael addition, in a 250 mL volume bottom flask, a solution of 234 mg of NalO₄ (1 mmol) in 38 mL of H₂O was prepared and cooled in an ice bath. 110 mg of pyrocatechol (1 mmol) dissolved in 1 mL of Et₂O was then added and left stirring vigorously for 15 min. After this time, extraction of the formed o-quinone with the addition of dichloromethane (DCM, 4x8mL) was carried out. Thereafter, it was dried over anhydrous sodium sulfate and filtered. On the other hand, a solution of 315 mg of monoacetylated dithiol previously prepared (approx. 1 mmol, considering a purity of ≈71%) in 2 mL of dichloromethane was prepared in an inert atmosphere in a Schlenk flask. To this solution 229.5 µl of trifluoroacetic acid (TFA, 3 mmol) was further added. Finally, the solution of the o-quinone in DCM was added to the solution containing the thiol, further protecting the mixture from light and leaving it under magnetic stirring under an inert atmosphere for six hours. After this time, both the solvent and the TFA were removed under vacuum to obtain the reaction crude. The product mixture was purified using a chromatography column with a 7/3 hexane/ethyl acetate eluent mixture in a yield of 38% of product 11. Characterization of **11**: ¹H-NMR (360 MHz, CDCl₃) δ 7.60 (broad, 1H), 6.99 (dd, *J₁* = 1.5 Hz, *J*₂= 7.8 Hz, 1H), 6.91 (dd, *J₁* = 1.5 Hz, *J*₂= 8.2 Hz, 1H), 6.73 (t, *J*= 7.9 Hz, 1H), 5.89 (broad, 1H), 3.65 (broad, 4H), 3.61 (t, *J* = 6.6 Hz, 2H), 3.55 (t, *J* = 5.7 Hz, 2H), 3.11 (t, *J* = 6.7 Hz, 2H), 2.91 (t, *J* = 5.7 Hz, 2H), 2.33 (s, 3H); ¹³C-NMR (360 MHz, CDCl₃) δ 196.14, 145.79, 144.90, 127.57, 120.75, 118.77, 116.75, 70.36, 70.24, 70.06, 68.99, 36.88, 30.86, 28.99; MS/ESI- [M-H]⁻ 332.075 Da.

### Example 2.2: Synthesis of compound (9)

The catechol derivative 9 was obtained in a manner analogous to that described above, but using 1,6-hexanedithiol instead of 2,2'-(ethylenedioxy)diethanethiol. After removal of the pyridine, a reaction crude was obtained with a mixture of the desired monoacetylated product, the diacetylated and the starting dithiol in a ratio of 65%, 29% and 5% respectively. This mixture was used without further purifications to carry out the Michael reaction, considering the percentage of purity of the monoacetylated product, and taking into account that of the formed by-products, the diacetylated compound is inert to the nucleophilic addition, and the dithiolated compound is found in a residual proportion as was elaborated in the previous example. After Michael addition, carried out in a manner analogous to that described for the previous example, the product 9 was obtained in a yield of 46% with respect to the addition itself, corresponding to 30% when taking the starting crude mixture. Characterization of **9**: ¹H-NMR (360 MHz, CDCl₃) δ 6.98 (dd, *J₁* = 1.3 Hz, *J*₂= 7.8 Hz, 1H), 6.90 (dd, *J₁* = 1.3 Hz, *J*₂= 8.2 Hz, 1H), 6.77 (t, *J*= 7.9 Hz, 1H), 2.84 (t, *J*= 7.3 Hz, 2H), 2.70 (t, *J*=7.4 Hz, 2H), 2.32 (s, 3H), 1.50 (m, 4H), 1.30 (m, 4H); ¹³C-NMR (360 MHz, CDCl₃) δ 196.50, 144.48, 144.11, 126.76, 121.01, 119.49, 116.53, 39.17, 36.73, 35.55, 30.94, 29.74, 29.23, 28.45; MS/ESI- [M-H]⁻ 299.078 Da.

### Example 3: Synthesis of monopodal compounds wherein R¹ is R³Z, and Z is thiol

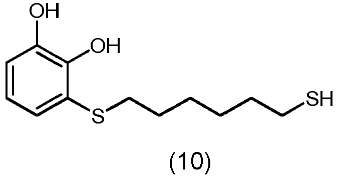

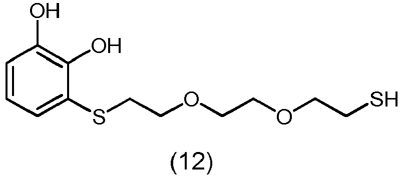

The products 10 and 12 can be synthesized by two procedures (A and B), as detailed below.

### Example 3.1: Synthesis by means of process A (deprotection of the respective thioacetylated derivatives, 9 and 11):

A solution of the monoacetylated derivative of the corresponding catechol-thiol (products 9, or 11, 1 mmol) in 15 mL of MeOH was prepared, and 400 µl of concentrated HCl (37% in water) was added. The mixture was stirred, refluxing for at least 15 hours. After this time, the solvent was evaporated in vacuo to give the corresponding thiol (10, or 12) in a yield of ≥98%. Characterization of **10**: ¹H-NMR (400 MHz, CDCl₃) δ 6.99 (dd, *J₁* = 1.5 Hz, *J*₂= 7.8 Hz, 1H), 6.92 (dd, *J₁* = 1.5 Hz, *J*₂= 8.0 Hz, 1H), 6.79 (t, *J*= 7.8 Hz, 1H), 2.70 (t, *J*=7.4, 2H), 2.50 (q, *J*= 7.3, 2H), 1.57 (m, 4H), 1.33 (t, *J*= 7.6, 1H), 1.31 (m, 4H); ¹³C-NMR (400 MHz, CDCl₃) δ 144.44, 144.06, 126.86, 121.04, 119.44, 116.59, 36.79, 34.01, 29.79, 28.25, 28.05, 24.78; MS/ESI- [M-H]⁻ 257.068 Da. Characterization of **12**: ¹H-NMR (400 MHz, CDCl₃) δ 7.59 (broad, 1H), 6.99 (dd, *J₁* = 1.5 Hz, *J*₂= 7.8 Hz, 1H), 6.92 (dd, *J₁* = 1.5 Hz, *J*₂= 8.0 Hz, 1H), 6.73 (t, *J*= 8.0 Hz, 1H), 5.79 (broad, 1H), 3.67 (s, 4H), 3.64 (t, *J* = 6.2 Hz, 2H), 3.56 (t, *J* = 5.8 Hz, 2H), 2.92 (t, *J* = 5.8 Hz, 2H), 2.72 (q, *J* = 6.5 Hz, 2H). ¹³C-NMR (400 MHz, CDCl₃) δ 145.70, 144.85, 127.61, 120.86, 118.74, 116.76, 73.21, 70.39, 70.21, 69.03, 36.85, 24.57; MS/ESI- [M-H]⁻ 290.065 Da.

### Example 3.1: Synthesis by means of process B (direct reaction with the corresponding α,ω-dithiol):

From the reaction between the catechol and the corresponding dithiol (see synthesis of compounds of formula II and synthesis of compound 16) the corresponding catechol-thiol was obtained as a by-product of the reaction in a single step, with yields of 21% (in the case of catechol-thiol 10) and 39% (catechol-thiol 12).

### Example 4: Synthesis of compounds of formula (I) wherein R¹ is R³Z, and Z is a functional fragment

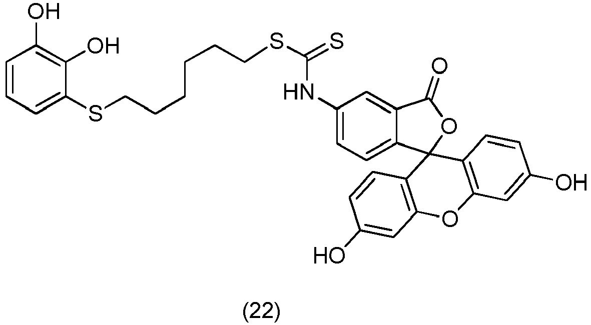

In a round bottom flask 55 mg of compound 10 (0.21 mmol) was weighed and dissolved in 4 mL of acetone. Once dissolved, 83 mg of fluorescein isothiocyanate (0.21 mmol) was added and the mixture was refluxed under magnetic stirring for 24 hours. After this time, the solvent was removed under reduced pressure to give the reaction crude, which was purified using a chromatography column with a 6/4 hexane/ethyl acetate eluent mixture in a total yield of 60% of compound 22. Characterization of **22**: ¹H-NMR (360 MHz, CDCl₃) δ 10.96 (broad, 1H), 8.90 (broad, 1H), 8.58 (s, 1H), 8.11 (dd, *J₁* = 1.8 Hz, *J*₂= 8.4 Hz, 1H), 7.30 (dd, *J₁* = 0.6 Hz, *J₂*= 8.4 Hz, 1H), 6.86 (dd, *J₁* = 1.6 Hz, *J*₂= 7.8 Hz, 1H), 6.78 (dd, *J₁* = 1.2 Hz, *J₂*= 8.0 Hz, 1H), 6.75 (d, *J*= 2.4 Hz, 2H), 6.71 (d, *J*= 8.4 Hz, 2H), 6.68 (t, *J*= 8.0 Hz, 1H), 6.64 (dd, *J₁* = 2.4 Hz, *J₂*= 8.4 Hz, 2H), 3.32 (t, *J* = 7.6 Hz, 2H), 3.22 (broad, 3H), 2.84 (t, *J* = 7.6 Hz, 2H), 1.72 (q, *J* = 7.0 Hz, 2H), 1.60 (q, *J* = 7.0 Hz, 2H), 1.47 (m, 4H); ¹³C-NMR (400 MHz, CDCl₃) δ 199.14, 160.33, 153.30, 150.70, 145.73, 145.39, 142.20, 131.15, 130.11, 128.31, 125.20, 124.14, 122.19, 120.63, 119.13, 115.43, 113.33, 111.37, 103.34, 35.72, 34.29, 28.81-30.60; MS [M+H]⁻ 648.1162 Da.

### B. Synthesis of bipodal catechol compounds

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone with the addition of dichloromethane (DCM, 4x8 mL) was carried out. Thereafter, it was dried over anhydrous sodium sulfate and filtered. On the other hand, a solution of the corresponding dithiol (0.5 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the dithiol, further protecting the mixture from light and leaving it under magnetic stirring under an inert atmosphere for six hours. After this time, both the solvent and the TFA were removed under vacuum to give the reaction crude containing a mixture of bipodal and monopodal derivatives. This mixture was purified using a chromatography column.

### Example 5: Synthesis of compound 16:

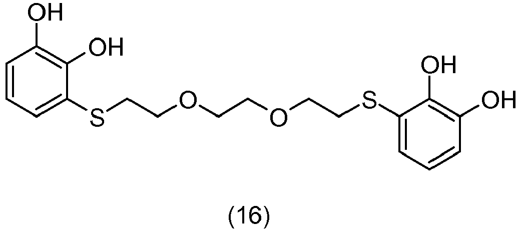

In a 250 mL volume bottom flask, a solution of 234 mg of NalO₄ (1 mmol) in 38 mL of H₂O was prepared and cooled in an ice bath. 110 mg of pyrocatechol (1 mmol) dissolved in 1 mL of Et₂O was then added and left stirring vigorously for 15 min. After this time, extraction of the formed o-quinone with the addition of dichloromethane (DCM, 4x8mL) was carried out. Thereafter, it was dried over anhydrous sodium sulfate and filtered. On the other hand, a solution of 73 mg of 2,2'-(ethylenedioxy) diethanethiol (0.5 mmol) in 2 mL of dichloromethane was prepared in an inert atmosphere in a Schlenk flask. To this solution 229.5 µl of trifluoroacetic acid (TFA, 3 mmol) was further added. Finally, the solution of the o-quinone in DCM was added to the solution containing the dithiol, further protecting the mixture from light and leaving it under magnetic stirring under an inert atmosphere for six hours. After this time, both the solvent and the TFA were removed in vacuo to obtain crude with products 16 and 12. The product mixture was purified using a chromatography column with a 7/3 hexane/ethyl acetate elution mixture with a total yield of 51% (37.3% of the bipodal derivative 16 and 20.7% of the monopodal product 12). Characterization of **16**: ¹H-NMR (400 MHz, CDCl₃) δ 7.65 (broad, 1H), 7.01 (dd, *J₁* = 1.5 Hz, *J₂*= 7.8 Hz, 2H), 6.93 (dd, *J₁* = 1.5 Hz, *J*₂= 8.0 Hz, 2H), 6.76 (t, *J*= 7.8 Hz, 2H), 5.85 (broad, 1H), 3.70 (s, 4H), 3.57 (t, *J* = 5.8 Hz, 2H), 2.94 (t, *J* = 5.8 Hz, 2H). ¹³C-NMR (400 MHz, CDCl₃) δ 145.77, 144.84, 127.75, 121.04, 118.65, 117.01, 70.23, 68.98, 36.96; MS/ESI- [M-H]⁻ 398.086 Da.

### C. Synthesis of catechol compounds in star form (3 branches)

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone by the addition of dichloromethane (DCM, 4x8 mL) was carried out. Thereafter, it was dried over anhydrous sodium sulfate and filtered. On the other hand, a solution of the corresponding trithiol (between 0.33 mmol and 1.2 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the trithiol, further protecting the mixture from light and leaving it under magnetic stirring under an inert atmosphere for six hours. After this time, both the solvent and the TFA were removed in vacuo to give the reaction crude which was purified using a chromatography column.

### Example 6: Synthesis of compound 20:

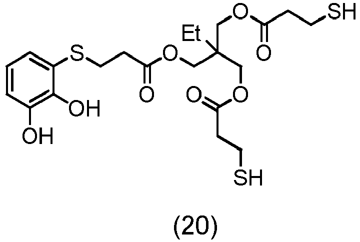

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone with the addition of dichloromethane (DCM, 4x8 mL) was carried out. Thereafter, it was dried over anhydrous sodium sulfate and filtered. On the other hand, a solution of tri-methylolpropane tri(3-mercapto-propionate) (1.2 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the trithiol, further protecting the mixture from light and leaving it under magnetic stirring under an inert atmosphere for six hours. After this time, both the solvent and the TFA were removed under vacuum to obtain the reaction crude. The product mixture was purified using a chromatography column with a 4/6 hexane/diethyl ether eluent mixture with a total yield of 45% of product 20. Characterization of **20**: ¹H-NMR (360 MHz, CDCl₃) δ 6.99 (dd, *J₁* = 1.8 Hz, *J*₂= 7.8 Hz, 1H), 6.94 (dd, *J₁* = 1.8 Hz, *J*₂= 7.8 Hz, 1H), 6.79 (t, *J*= 7.8 Hz, 1H), 4.09 (s, 6H), 2.97 (t, *J*= 6.5 Hz, 2H), 2.77 (q, *J*= 6.7 Hz, 4H), 2.67 (t, *J*= 6.2 Hz, 4H), 2.55 (t, *J*= 6.7 Hz, 2H), 1.63 (t, *J*= 8.1 Hz, 2H), 1.50 (q, *J*= 7.7 Hz, 2H), 0.91 (t, *J*= 7.6 Hz, 3H); ¹³C-NMR (400 MHz, CDCl₃) δ 172.10, 171.82, 145.16, 144.67, 127.13, 121.32, 118.00, 117.15, 64.44, 64.12, 41.03, 38.68, 34.02, 31.23, 23.19, 19.99, 7.67; MS/ESI- [M-H]⁻ 505.103 Da.

### Example 7: Synthesis of compound 21:

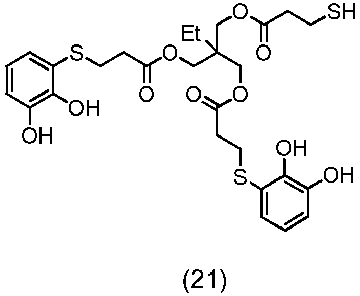

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone with the addition of dichloromethane (DCM, 4x8 mL) was carried out. Thereafter, it was dried over anhydrous sodium sulfate and filtered. On the other hand, a solution of tri-methylolpropane tri(3-mercapto-propionate) (0.33 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the trithiol, further protecting the mixture from light and leaving it under magnetic stirring under an inert atmosphere for six hours. After this time, both the solvent and the TFA were removed under vacuum to obtain the reaction crude. The product mixture was purified using a chromatography column with a 7/3 hexane/ethyl acetate eluent mixture with a total yield of 14% of product 21. Characterization of **21**: ¹H-NMR (360 MHz, CDCl₃) δ 7.00 (dd, *J₁* = 1.6 Hz, *J*₂= 7.7 Hz, 1H), 6.94 (dd, *J₁* = 1.6 Hz, *J*₂= 7.9 Hz, 1H), 6.78 (t, *J*= 7.9 Hz, 1H), 4.09 (s, 6H), 2.97 (t, *J*= 6.6 Hz, 4H), 2.77 (q, *J*= 6.5 Hz, 2H), 2.67 (t, *J*= 6.6 Hz, 2H), 2.57 (t, *J*= 6.7 Hz, 4H), 1.63 (t, *J*= 8.2 Hz, 1H), 1.50 (q, *J*= 7.6 Hz, 2H), 0.91 (t, *J*= 7.4 Hz, 3H); ¹³C-NMR (400 MHz, CDCl₃) δ 171.03, 170.79, 143.92, 143.47, 125.93, 120.15, 116.88, 115.99, 63.26, 62.96, 39.87, 37.51, 32.92, 30.45, 22.04, 18.78, 6.49; FT-IR (ATR) ν(cm⁻¹) 3404, 2968, 2944, 1727, 1602, 1587, 1459, 1354, 1245, 1222, 1189, 1138, 1056, 1002, 935, 899, 823, 777, 728, 674, 639, 567; MS/ESI-[M-H]⁻ 613.125 Da.

### D. Synthesis of catechol derivatives in star form (4 branches)

In a 250 mL volume bottom flask, a solution of 234 mg of NalO₄ (1 mmol) in 38 mL of H₂O was prepared and cooled in an ice bath. The corresponding catechol (1 mmol) dissolved in 1 mL of Et₂O was then added and left stirring vigorously for 15 min. After this time, extraction of the corresponding formed quinone by the addition of dichloromethane (DCM, 4x8 mL) was carried out. Thereafter, it was dried over anhydrous sodium sulfate and filtered. On the other hand, a solution of the corresponding tetrathiol (between 0.25 mmol and 1.1 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the tetrathiol, further protecting the mixture from light and leaving it under magnetic stirring under an inert atmosphere for six hours. After this time, both the solvent and the TFA were removed in vacuo to give the reaction crude which was purified using a chromatography column.

### Example 8: Synthesis of compound 24:

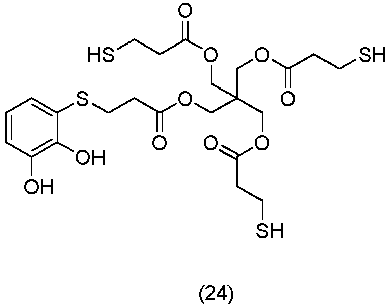

In a 250 mL volume bottom flask, a solution of 234 mg of NalO₄ (1 mmol) in 38 mL of H₂O was prepared and cooled in an ice bath. The pyrocatechol (1 mmol) dissolved in 1 mL of Et₂O was then added and left stirring vigorously for 15 min. After this time, extraction of the corresponding formed quinone with the addition of dichloromethane (DCM, 4x8 mL) was carried out. Thereafter, it was dried over anhydrous sodium sulfate and filtered. On the other hand, a solution of pentaerythritol tetrakis (3-mercaptopropionate) (1.1 mmol) in 2 mL of dichloromethane was prepared in an inert atmosphere in a Schlenk flask. To this solution 229.5 µl of trifluoroacetic acid (TFA, 3 mmol) was further added. Finally, the solution of the corresponding quinone in DCM was added to the solution containing the tetrathiol, further protecting the mixture from light and leaving it under magnetic stirring under an inert atmosphere for six hours. After this time, both the solvent and the TFA were removed under vacuum to obtain the reaction crude. The product mixture was purified using a chromatography column with a 6/4 hexane/ethyl acetate eluent mixture in a total yield of 50% of product 24, obtaining compound 25 in 20% yield as a by-product. Characterization of **24**: ¹H-NMR (360 MHz, CDCl₃) δ 6.99 (dd, *J₁* = 1.7 Hz, *J*₂= 7.8 Hz, 1H), 6.93 (dd, *J₁*= 1.7 Hz, *J*₂= 8.0 Hz, 1H), 6.79 (t, *J*= 7.8 Hz, 1H), 4.19 (s, 8H), 6.99 (dd, *J₁* = 1.8 Hz, *J*₂= 7.8 Hz, 1H), 2.97 (t, *J*= 6.4 Hz, 2H), 2.77 (m, 6H), 2.68 (t, *J*= 6.3 Hz, 6H), 2.55 (t, *J*= 6.4 Hz, 2H), 1.64 (t, *J*= 8.1 Hz, 1H); ¹³C-NMR(400 MHz, CDCl₃) δ 171.92, 171.57, 145.12, 144.63, 127.05, 121.40, 117.91, 117.22, 62.45, 62.66, 42.32, 38.54, 33.91, 31.55, 19.91; MS/ESI- [M-H]⁻ 595.081 Da.

### Example 9: Synthesis of compounds of structure (IV) with a catechol ring and a functional moiety:

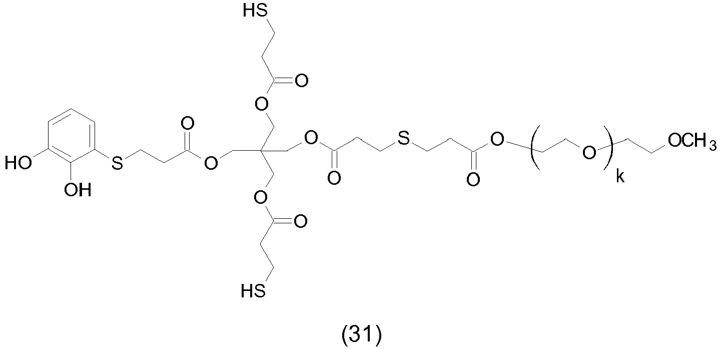

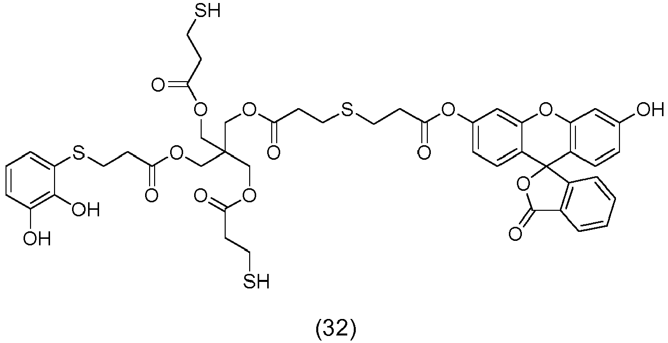

### Example 9.1: Synthesis of compound 31:

A solution of 330 mg of PEG-acrylate (MW: 2000, k ∼ 45) (0.17 mmol) in 2 mL of anhydrous toluene was prepared under an inert atmosphere in a 10 mL volume long neck Schlenk flask and heated to form a clear solution. Subsequently, 16 mg of AIBN (0.06 mmol) and a solution of 200 mg of the catechol derivative 24 (0.34 mmol) in 2.6 mL of anhydrous toluene were added in three portions over a 24 hour period. The reaction was carried out at reflux temperature and was followed by nuclear magnetic resonance until the complete disappearance of the proton characteristic signals of the acrylate double bond. After the reaction was complete, it was allowed to cool to room temperature and the reaction mixture was concentrated in vacuo. An oil was obtained which was re-dissolved in a minimum amount of dichloromethane. Finally, diethyl ether was added until a whitish turbidity was observed. The mixture was left in the freezer overnight and the white precipitate which was formed was filtered, washed several times with cold diethyl ether and dried under vacuum to provide compound 31 in 67% yield. Characterization of **31**: ¹H-NMR (400 MHz, CDCl₃) δ 7.20-6.63 (broad, 3H), 4.22 (broad, 2H), 4.16 broad, 8H), 4.05-3.47 (broad, >150H), 3.36 (s, 3H), 3.22-2.44 (broad, 20H), 1.64 (t, *J*= 8.22 Hz, 2H); ¹³C-NMR (400 MHz, CDCl₃) δ 171.92, 171.57, 145.12, 144.63, 127.05, 122.64, 121.40, 72.20, 70.80, 69.41, 64.02, 62.66, 62.45, 59.03, 43.78, 42.32, 42.03, 38.54, 37.63, 36.62, 35.51, 34.53, 33.81, 31.55, 28.17, 27.88, 26.86, 26.47, 25.79, 25.54, 23.70, 23.15, 19.91; MALDI-MS (matrix: dithranol), m/z (M+Na⁺) 1411, 1455, 1499, 1543, 1587, 1631, 1675, 1719, 1763, 1807, 1851, 1895, 1939, 1983, 2027, 2071, 2115, 2159, 2203, 2247, 2291, 2335, 2379, 2423, 2467, 2512, 2556, 2600, 2644, 2688, 2732, 2777, 2821, 2865, corresponding to k=15-48.

### Example 9.2: Synthesis of compound 32:

In a 10 mL volume long neck Schlenk flask, a solution of 150 mg of fluorescein o-acrylate (0.39 mmol) in 12 mL of anhydrous acetonitrile was prepared under an inert atmosphere and heated to form a clear solution. 36 mg of AIBN (0.22 mmol) and a solution of 450 mg of the catechol derivative 24 (0.75 mmol) in 5 mL of anhydrous acetonitrile were then added in three portions over a period of 24 hours. The reaction was carried out at reflux temperature and was followed by nuclear magnetic resonance until the complete disappearance of the proton characteristic signals of the acrylate double bond. After the reaction was complete, it was allowed to cool to room temperature and the reaction mixture was concentrated in vacuo. An oil was obtained which was purified by column chromatography using the gradient hexane:ethyl acetate 3:7 to 100% ethyl acetate and 100% acetone. The fraction with the product of interest turned out to be an oil which was crystallized from diethyl ether. The formed orange colored solid was filtered and washed repeatedly with diethyl ether. Finally, the solid was treated with hexane, filtered, washed and dried, yielding the product 32 in 31% yield. Characterization of **32**: ¹H-NMR (400 MHz, (CD₃)₂CO) δ 8.01 (m, 1H), 7.82 (td, *J*₁ = 1.32 Hz, *J*₂ = 7.41 Hz, 1H), 7.75 (td, *J*₁ = 1.01 Hz, *J*₂ = 7.41 Hz, 1H), 7.39-7.16 (broad, 2H), 6.95-6.87 (broad, 5H), 6.81 (s, 1H), 6.77 (d, *J* = 2.30 Hz, 1H), 6.72-6.65 (broad, 1H), 4.26 (s, 8H), 3.17-2.61 (broad, 20 H), 1.61 (t, *J* = 7.99 Hz, 2H); ¹³C-NMR (400 MHz, (CD₃)₂CO) δ 170.84, 168.8, 159.6, 153.1, 152.9, 152.5, 152.3, 155.2, 151.7, 135.4, 135.2, 130.1, 129.3, 129.1, 127.0, 126.5, 124.7, 124.1, 118.0, 112.8, 112.4, 110.8, 110.3, 102.5, 102.4, 69.1, 65.4, 62.6, 46.2, 43.4, 42.3, 33.7, 27.6, 27.2, 25.7; MS/ESI-[M-H]⁻ 981.160 Da.

### E. Synthesis of catecholic polymers obtained by oxidative coupling of bis-thiols

By the following approach, examples of polymers were synthesized by coupling thiols to form polydisulfides. They are two classes (homo- and heteropolymers):

### E-1. Synthesis of catecholic homopolymers obtained by oxidative coupling of bis-thiols

1 mmol of the corresponding catechol of formula IIIa, IVa or IVb were weighed into a round bottom flask dissolving it in a minimum necessary amount of ethanol. On the other hand, a solution of molecular iodine was prepared keeping a stoichiometry of 2 to 1 between each thiol and iodine respectively (1 mmol of I₂ for one dithiol, as products IIIa or IVb, 1.5 mmol of I₂ for one trithiol, as the product of formula IVa) in a minimum amount of ethanol. This second solution, which contained molecular iodine (bright orange) was added dropwise under magnetic stirring over the first solution containing the corresponding catechol-thiol (colorless solution) until it had persistently the typical orange color of the molecular iodine. At this point it was estimated that all thiol groups had reacted and there was excess molecular iodine. The appearance of a precipitate of different texture was observed according to the starting catechol. The supernatant liquid was removed, and the precipitate was washed repeatedly with ethanol.

### Example 10: Synthesis of homopolymer 26:

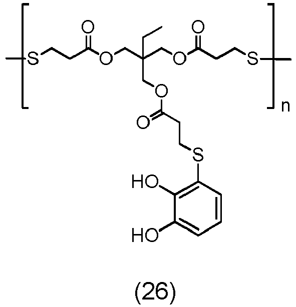

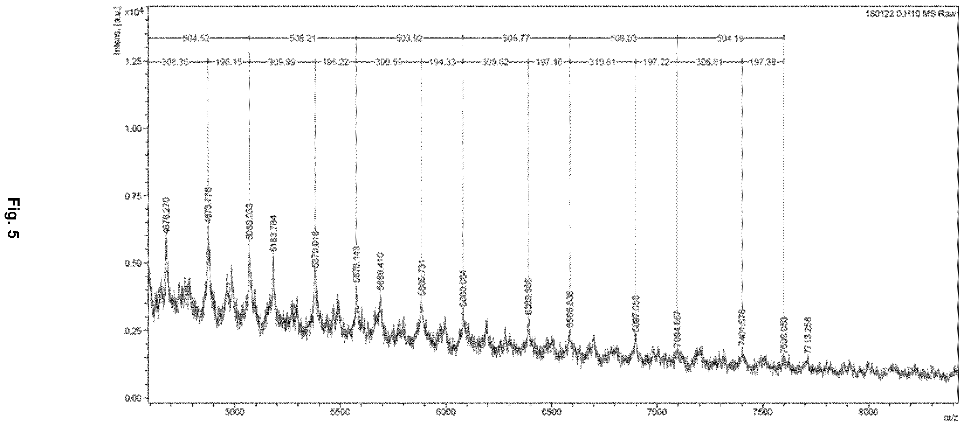

1 mmol of the catechol 20 was weighed into a round bottom flask by dissolving it in a minimum necessary amount of ethanol (approx. 0.5 mL). On the other hand, a solution of molecular iodine was prepared by weighing 253 mg (1 mmol) and dissolving them in 1.5 mL of ethanol. This second solution, which contained molecular iodine (bright orange) was added dropwise under magnetic stirring over the first solution containing the catechol 20 (colorless solution) until it had persistently the typical bright orange color of the molecular iodine. The appearance of a viscous precipitate was observed in parallel. The supernatant liquid was removed, and the precipitate was washed repeatedly with ethanol, obtaining compound 26 in 53% yield. The obtained solid could be re-dissolved in organic solvents such as dichloromethane or chloroform. Characterization of **26**: ¹H-NMR (360 MHz, CDCl₃) δ 6.97 (d, *J*= 7.2 Hz, 1H), 6.92 (d, *J*= 7.2 Hz, 1H), 6.78 (t, *J*= 7.2 Hz, 1H), 4.07 (s, 6H), 2.96 (t, *J*= 6.2 Hz, 2H), 2.90 (m, 4H), 2.76 (t, 4H), 2.55 (t, *J*= 6.1 Hz, 2H), 1.50 (m, 2H), 0.91 (t, *J*= ∼6 Hz, 3H). ¹³C-NMR (400 MHz, CDCl₃) δ 172.14, 171.95, 145.25, 144.72, 127.05, 121.27, 118.23, 117.16, 64.50, 64.30,41.05, 34.26, 34.12, 33.12, 31.56, 23.22, 7.73;FR-IR (ATR) ν(cm⁻¹) 3409, 2969, 2928, 1727, 1602, 1587, 1459, 1352, 1218, 1132, 1056, 1015, 991, 929, 899, 851, 824, 750, 728, 666, 640, 567; MALDI-MS (matrix: dithranol) (Figure 5), m/z (M+Na⁺) 1032, 1537, 2042, 2547, 3051, 3556, 4061, 4565, 5070, 5576, 6080, 6586, corresponding to n=2-13.

### Example 11: Synthesis of homopolymer 33:

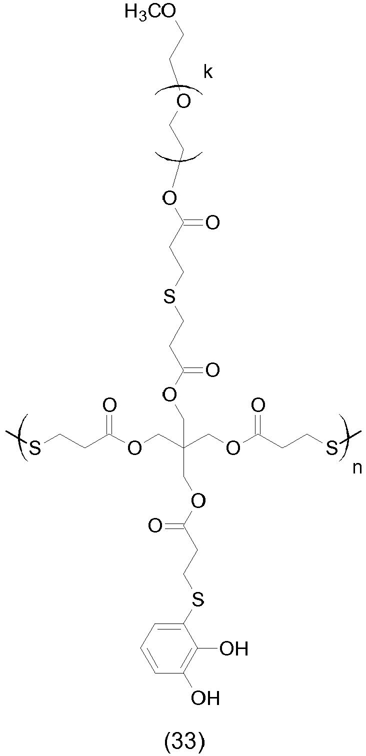

In a 20 mL vial, 0.04 mmol of the catecholic monomer 31 was weighed and dissolved in a minimum necessary amount of methanol (1 mL approx.). On the other hand, a solution of molecular iodine was prepared by weighing 19 mg (0.07 mmol) and dissolving them in 0.2 mL of methanol. This second solution, which contained molecular iodine (bright orange) was added dropwise under magnetic stirring over the first solution containing the catechol derivative until it had persistently the orange color of the molecular iodine. The reaction crude was allowed to stir for one hour. Finally, diethyl ether was added to the final solution until a precipitate appeared. The precipitate was left overnight in the freezer and the next day the precipitate was filtered and repeatedly washed first with cold diethyl ether and then with ethanol, obtaining compound 33 in 45% yield. Characterization of **33**: ¹H-NMR (250 MHz, CDCl₃) δ 7.1-6.6 (broad, 3H), 4.1-4.4 (broad, 10H), 4.05-3.42 (broad, > 150H), 3.38 (s, 3H), 3.26-2.44 (broad, 20H).

### Example 12: Synthesis of homopolymer 28:

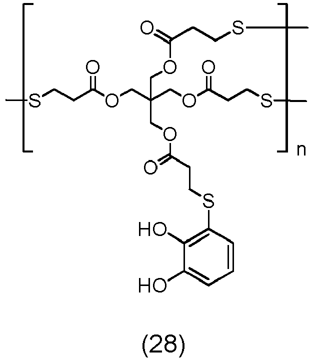

1 mmol of catechol 24 was weighed into a round bottom flask by dissolving it in a minimum necessary amount of ethanol (approx. 0.5 mL). On the other hand, a solution of molecular iodine was prepared by weighing 380 mg (1.5 mmol) and dissolving them in 2 ml of ethanol. This second solution, which contained molecular iodine (bright orange) was added dropwise under magnetic stirring over the first solution containing the catechol 24 (colorless solution) until it had persistently the typical orange color of the molecular iodine. At this point it was estimated that all thiol groups had reacted and there was excess molecular iodine. The appearance of a solid white precipitate was observed. The supernatant liquid was removed and the precipitate was washed repeatedly with ethanol, obtaining product 28 in 18% yield. The obtained white solid was insoluble in usual organic solvents (alcohols, acetone, ethyl acetate, chlorinated solvents, DMF), as corresponding to a fully crosslinked-structure polymer. Characterization of **28**: FR-IR (ATR) ν(cm⁻¹) 3402, 2963, 2927, 1726, 1602, 1587, 1459, 1350, 1227, 1127, 1021, 982, 929, 899, 852, 822, 775, 728, 666, 639, 567.

### E-2. Synthesis of catecholic heteropolymers obtained by oxidative coupling of bis-thiols

1 mmol of the corresponding catechol of formula IIIa, IVa or IVb, and a variable amount of another compound containing two or more thiol groups were weighed into a round bottom flask, which additionally kept the desired stoichiometry with the above compound, dissolving said mixture in a minimum necessary amount of ethanol. On the other hand, a solution of molecular iodine was prepared keeping a stoichiometry of 2 to 1 between each thiol and iodine, respectively, in a minimum amount of ethanol. This second solution, which contained molecular iodine (bright orange color) was added dropwise under vigorous magnetic stirring over the first solution containing the product mixture (colorless solution) until it had persistently the typical orange molecular of the molecular iodine. At this point it was estimated that all thiol groups had reacted and there was excess molecular iodine. The appearance of a precipitate of different texture was observed according to the starting mixture. The liquid supernatant was removed and the precipitate was washed repeatedly with ethanol, leaving a precipitate identified with the corresponding heteropolymer.

### Example 13: Synthesis of heteropolymer 27:

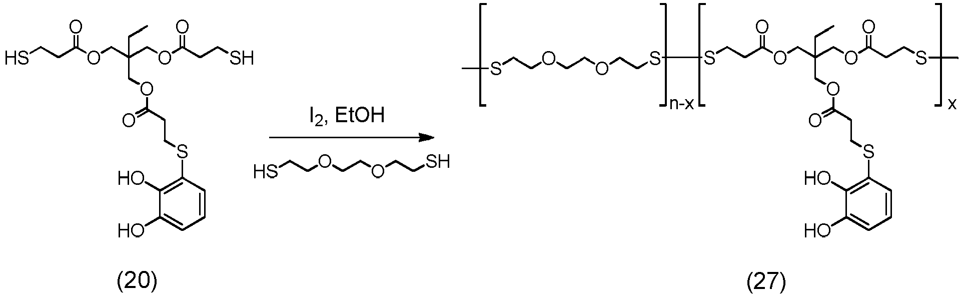

253 mg of catechol 20 (0.5 mmol) and 91 mg of 2,2'-(ethylenedioxy) diethanethiol (0.5 mmol) were weighed into a round bottom flask by dissolving them in a minimum necessary amount of ethanol (0.5 mL approx.). On the other hand, a solution of molecular iodine was prepared by weighing 253 mg (1 mmol) and dissolving them in 1.5 ml of ethanol. This second solution, which contained molecular iodine (bright orange color) was added dropwise under magnetic stirring over the first solution containing the mixture of catechol 20 with 2,2'-(ethylenedioxy) diethanethiol (colorless solution) until it had persistently the typical orange color of the molecular iodine. At this point it was estimated that all thiol groups had reacted and there was excess molecular iodine. The appearance of a viscous precipitate was observed. The liquid supernatant was removed and the precipitate was washed repeatedly with ethanol, and suspended in chlorinated solvents such as dichloromethane or chloroform, identified with polymer 27. Characterization of **27**: ¹H-NMR (360 MHz, CDCl₃) δ 6.97 (1H), 6.91 (1H), 6.77 (1H), 4.06 (6H), 3.76 (4H), 3.66 (4H), 2.98 (2H), 2.90 (4H), 2.88 (4H), 2.76 (4H), 2.57 (2H), 1.49 (2H), 0.89 (3H); ¹³C-NMR (400 MHz, CDCl₃) δ 172.02, 171.90, 145.22, 144.67, 126.99, 121.23, 118.17, 117.12, 70.60, 69.86, 64.43, 64.23, 41.00, 38.56, 34.06, 33.31, 33.06, 31.51, 23.16, 7.70.MALDI-MS (matrix: dithranol) (Figure 5), m/z (M+Na⁺)1031, 1535, 2040, 2546 (n-x=0; x=2-5), 1211, 1716, 2219, 2725 (n-x=1; x=1-5), 1391, 1895, 2401 (n-x=2; x=2-4), 1067, 1571 (n-x=3; x=1-2), 1247 (n-x=4; x=1)

### F. Preparation of ex situ coatings with monopodal catecholic compounds

### Example 14: Coating of a TiO₂ surface with catechols 1, 2, 4 and 5:

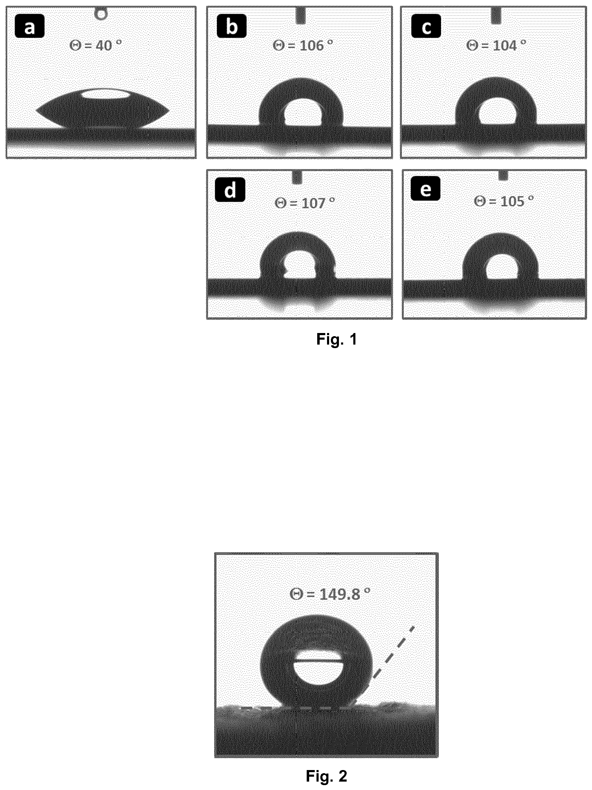

A rectangular surface of approx. 4 cm² coated with TiO₂ was immersed for 3 hours in a 10mM solution of a mono- or bi-substituted catechol in an appropriate solvent (1b or 2b in dichloromethane, 4 or 5 in THF), obtained by the general procedure described in section A. After this time, the substrate was washed thoroughly with MeOH and dried by N₂ flow. To verify the hydrophobicity provided by the catecholic coating to the substrate, contact angle measurements were performed (see Fig. 1).

### Example 15: Coating of magnetite nanoparticles with catechol 6:

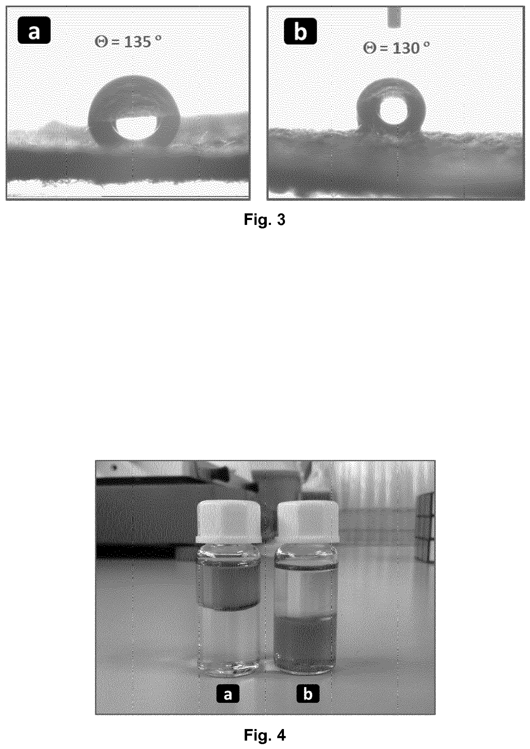

300 µl of a suspension of magnetite nanoparticles in hexane stabilized with oleic acid (approx. 20 mg/mL) was added to a solution containing 15 mg of catechol **6** (approx. 0.017 mmol) in 1 mL dichloromethane, shaking for 15 hours. After this time, the sample was centrifuged, washing once with dichloromethane and 4 times with hexane. In figure 4 two vials are shown with the magnetite nanoparticles stabilized with oleic acid and the nanoparticles after the coating stabilized by the catechol **6.** Figure 4a shows a vial with water (bottom) and a stable suspension of magnetite stabilized with oleic acid in hexane (top). Figure 4b shows a vial with hexane (top) and a stable suspension of magnetite stabilized with catechol 6 in hexane (bottom).

### G. Preparation of compounds obtained by oxidative polymerization of monopodal catecholic compounds in the presence of ammonia and air

Synthesis of the polymers derived from catechols of formula (I) by oxidative polymerization in the presence of ammonia was carried out analogously to that disclosed in EP2589578. For this, 1 mmol of the corresponding catechol of formula (I) was dissolved in 70 mL of isopropanol and 7.55 mL of NH₃ (aq., 25%, 100 mmol) was added. The mixture was stirred for 6 hours by heating to 55°C. After this time, 60 mL of H₂O was added. The remaining solvent was then evaporated, and HCI (conc.) was added dropwise to a pH of 5. At that time the mixture was extracted with an appropriate solvent (3 x 20 mL), dried over anhydrous NaSO₄, and the solvent was removed under reduced pressure. The corresponding polymer was thus obtained.

### Example 16: Preparation of the compound p2.

0.4 grams (1 mmol) of compound 1b (obtained as described in example 1.1) was dissolved in 70 mL of isopropanol and 7.55 mL of NH₃ (aq., 25%, 100 mmol) was added. The mixture was stirred for 6 hours by heating to 55°C. After this time, 60 mL of H₂O was added. The remaining solvent was then evaporated, and HCl (conc.) was added dropwise to pH of 5. At that time the mixture was extracted with hexane (3 x 20 mL), dried over anhydrous NaSO₄, and the hexane was removed under reduced pressure. 350 mg of a very viscous black oil was thus obtained, identified as compound p2.

### Example 17: Preparation of the compound p4.

588 mg (1 mmol) of catechol 4 (obtained by the general procedure described in section A) was dissolved in 70 mL of isopropanol and 7.55 mL of NH₃ (aq., 25%, 100 mmol) was added. The mixture was stirred for 6 hours by heating to 55°C. After this time, 60 mL of H₂O was added. The remaining solvent was then evaporated, and HCl (conc.) was added dropwise to a pH of 5. At that time the mixture was extracted with tetrahydrofuran (THF, 3 x 20 mL), dried over anhydrous NaSO₄, and THF was removed under reduced pressure. 505 mg of a black solid (p4) was thus obtained.

### H. Preparation of ex situ coatings with compounds obtained by oxidative polymerization of monopodal catecholic compounds in the presence of ammonia and air

Once the corresponding polymer was obtained, it was solubilized in an appropriate solvent and said solution was used to carry out the *ex situ* coatings. Macroscopic objects, in particular cotton fabric, glass, or titanium oxide, of about 4 cm² were immersed for times ranging from 2 min to 3 hours in a solution with an appropriate solvent at a concentration of 10 mM of the corresponding catechol derivative, or 1-5% by mass of the polymer formed by reaction with ammonia, as indicated above. After this time, each material was washed thoroughly with MeOH and dried by N₂ flow.

### Example 18: Ex situ coating of a cotton fabric with compound p2

A piece of cotton cloth of about 4 cm² was immersed for 2 min in a 1% by mass solution of **p2** in hexane. After this time, it was washed thoroughly with MeOH and dried by N₂ flow. In order to check the hydrophobicity provided by the polymer, contact angle measurements were made. For this purpose, a drop of water was added to the cotton fabric with and without coating and the contact angle was measured. In uncoated cotton the drop was absorbed instantly, however in the coated fabric the contact angle was 149.8° for p2 (Fig. 2). The material was observed for more than one hour without appreciable absorption by the fabric.

### Example 19: Ex situ coating of a cotton fabric with compound p4

A piece of cotton cloth of about 4 cm² was immersed for 2 min in a 1% by mass solution of **p4** in THF. After this time, it was washed thoroughly with MeOH and dried by N₂ flow. In order to check the hydrophobicity and oleophobicity provided by the polymer, contact angle measurements were performed. For this purpose, a drop of water was first added to the cotton fabric with and without coating and the contact angle was measured (Fig. 3a). In the uncoated cotton the drop was absorbed instantly, however in the coated fabric the contact angle was 135°. The material was observed for more than one hour, without appreciable absorption by the fabric. Secondly, in order to check the oleophobicity of the coated tissue, a drop of tetradecane was added, in this case a contact angle of 130° was observed (Fig. 3.b).

### I. Preparation of ex situ coatings of nanoscopic systems with functional catecholic compounds

### Example 20: Ex situ coating of amorphous SiO₂ nanoparticles with PEGylated compound 31

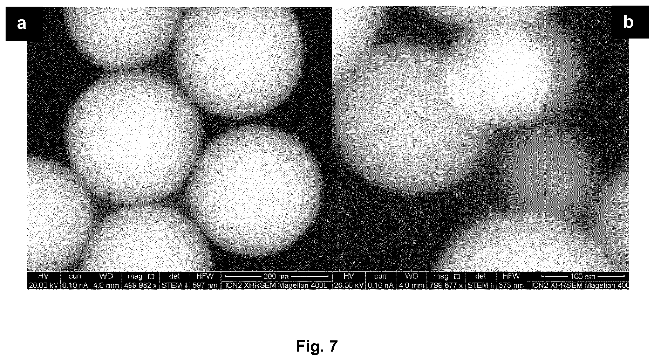

10 mg of silica nanoparticles (Ø = 150-250 nm) were dispersed in 1 mL of anhydrous dichloromethane under an inert atmosphere. To this suspension a solution of 40 mg of the PEGylated catechol compound 31 in 1 mL of anhydrous dichloromethane was added and allowed to stir at 300 rpm overnight at room temperature. After this time, the supernatant was discarded and the nanoparticles were washed three times with dichloromethane. The treated nanoparticles were air-dried, re-suspended in ethanol and observed at STEM, and an ultrafine coating of a few nanometers could be observed by contrast on the surface (Fig. 7).

### Example 21: Ex situ coating of amorphous SiO₂ nanoparticles with fluorescent compound 32

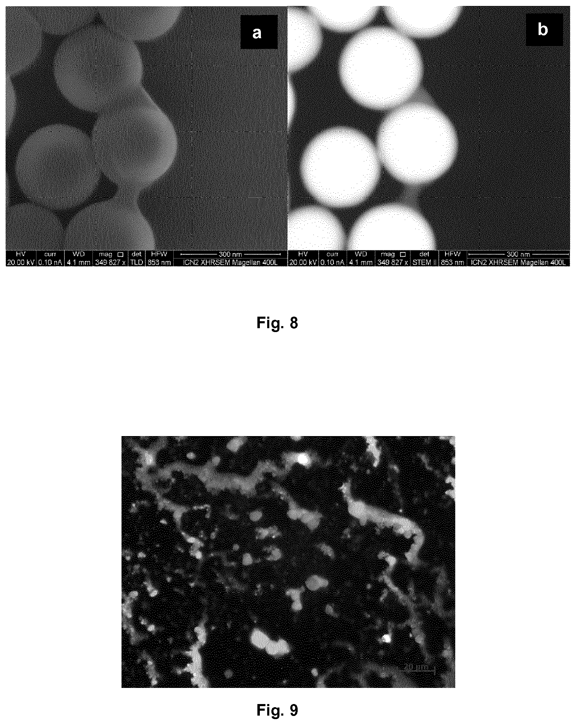

5 mg of silica nanoparticles (Ø = 150-250 nm) were dispersed in 0.5 mL of acetone under an inert atmosphere. To this suspension a solution of 10 mg of the fluorescent catecholic compound 32 in 0.5 mL of acetone was added and left under magnetic stirring at 250 rpm for 16 h at room temperature. After this time, the supernatant was discarded and the nanoparticles were washed three times with HPLC grade acetone. The treated nanoparticles were air-dried, re-suspended in ethanol and observed at STEM, and an ultrafine coating of a few nanometers could be observed by contrast on the surface (Fig. 9). The same coated nanoparticles were observed under fluorescence optical microscopy (Xe lamp, λₑₓ = 450 - 490 nm) observing an intense fluorescent signal (Fig. 10).

### J. Preparation of ex situ coatings of nanoscopic systems with polymers derived from functional catecholic compounds

### Example 22: Ex situ coating of amorphous SiO₂ nanoparticles with PEGylated polymer 33

10 mg of silica nanoparticles (Ø = 150-250 nm) were dispersed in 2 mL of dichloromethane. To this suspension a solution of 40 mg of the PEGylated catecholic polymer 33 in 1 mL of anhydrous dichloromethane was added and left under magnetic stirring at 250 rpm for 16 h at room temperature. After this time, the supernatant was discarded and the nanoparticles were washed three times with dichloromethane. The treated nanoparticles were air-dried, re-suspended in ethanol and observed at STEM, and an ultrafine coating of a few nanometers could be observed by contrast on the surface (Fig. 8).

### K. Adhesion tests with polymers derived from catecholic compounds

### Example 23: Shear bond strength tests (lap shear adhesion test) with the compound 26

Aliquots of 10 mg of compound 26 were dissolved in 1 mL of dichloromethane. In parallel, the substrates in the form of rectangular plates (glass: 75 mm x 25 mm x 1 mm, copper: 100 mm x 25 mm x 1.5 mm) were washed in an ultrasonic bath and, in succession, with mili Q water, ethanol and finally acetone for ten minutes in each solvent. The substrates were dried under a stream of nitrogen. Then 40 µL of the polymer solution were spread over an approximate 25 × 20 mm area of one plate, and a second plate of the same material was overlapped over the first one in the opposite direction, covering the indicated area of adhesion. The joining of the substrates was secured with metal clips for 24 hours. The adhesion strength tests were performed according to ISO 4587 at a separation rate of 0.02 mm/s.

| **Substrate** | **Adhesion/MPa** | **Elongation at breakage/mm** |
|---|---|---|
| Glass/glass | 0.24 | 0.40 |
| Cupper/cupper | 0.25 | 0.16 |

## Claims

1. Catechol-derivative compound of formula (I): 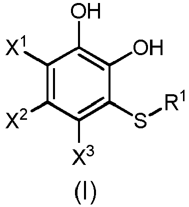 wherein,
**X¹** is selected from the group consisting of hydrogen and a substituent **-SR²**,
**X²** and **X³** are hydrogen atoms,
**R¹** and **R²** are independently selected from the group consisting of:
• a moiety **FUNC,** which is selected from the group consisting of:
∘ an alkyl moiety of formula -CₙH₂ₙ₊₁, wherein n ranges between 12 and 18;
∘ an alkenyl moiety of formula -CₙH₂ₙ₋₁, wherein n ranges between 2 and 6;
∘ an alkynyl moiety of formula -CₙH₂ₙ₋₃, wherein n ranges between 2 and 6;
∘ a polyfluoroalkyl moiety of formula -(CH₂)ₚ₁-CₙF₂ₙ₊₁, wherein p₁ is comprised between 0 and 3, and n is comprised between 5 and 8;
∘ a moiety of formula -(CHRCH₂O)_{q}R', wherein R is a hydrogen atom or a methyl group, and R' is selected from a hydrogen atom, a C₁-C₁₀ alkyl group, terminal C₁-C₁₈ alkenyl, terminal C₁-C₁₈ alkynyl, -COOH, -NH₂, - N₃ or *N*-maleimido, and q is a value comprised between 5 and 300;
∘ a fluorescent tag selected from fluorescent compounds with thiol groups, fluorescein, eosin, rhodamines; boron-dipyrromethene (BODIPY) fluorescent derivatives; fluorescent derivatives of azo structure (diazoarenes), fluorescent derivatives of pyridine-methoxyphenyleneoxazoles (PyMPO), fluorescent derivatives of Lucifer Yellow, fluorescent derivatives of benzoxadiazol (BD), Fluorescent Red, Fluorescent Orange, and other fluorescent labels available under registered trademarks (Atto and Alexa), which can be conjugated through ad hoc reactive groups;
∘ an oligopeptide selected from glutathione and an oligopeptide comprised of 5-25 amino acids, from which from 20% to 100% are arginine; and
• a moiety **-R³-S-Z**, wherein,
∘ S is a sulfur atom,
∘ **R³** is selected from the group consisting of:
▪ an alkandiyl moiety of formula -(C_{n'}H_{2n'})-, wherein n' is comprised between 2 and 18;
▪ a (polyalkylenoxy)alkyl moiety of formula - (CHR"CH₂O)_{q1}(CHR"CH₂)-, wherein **R"** is selected from the group consisting of a hydrogen atom and a methyl moiety, and q₁ is comprised between 2 and 300;
∘ **Z** is selected from the group consisting of:
▪ a hydrogen atom;
▪ a -COCH₃ moiety;
▪ a -CH₂-CH₂-Y-FUNC moiety, wherein **Y** is selected from the group consisting of -O-, -COO-, -CONH-, -(CH₂)ᵣO-, being r a value comprised between 1 and 10; and FUNC is the moiety as previously defined;
▪ a moiety of structure (**J***): 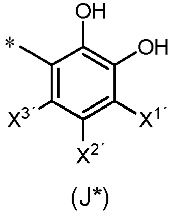 wherein **X^{1'}** is selected from the group consisting of hydrogen, and a substituent **-SR²**; wherein R² is as previously defined; and **X^{2'}** and **X^{3'}** are hydrogen atoms;
• a moiety of the type **(BRANCH*):** 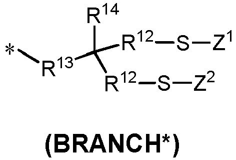 wherein,
∘ **R¹²** is a moiety of formula -(CH₂-O-Q-CH₂-W)-, wherein **Q** is selected from the group consisting of -CO-, and -(CₖH₂ₖO)_{q1}-, wherein k is a value comprised between 2 and 4, and q₁ is a value comprised between 2 and 300; and wherein **W** is selected from the group consisting of - CHR"'-, and -CH(OH)CH₂-, wherein **R'"** is a hydrogen atom or a methyl group.
∘ **R¹³** is a moiety of formula -(W-CH₂-Q-O-CH₂)-, wherein **Q** and **W** are defined in the same way as for R¹²;
∘ **Z¹** and **Z²** are independently selected from the group consisting of:
▪ a hydrogen atom;
▪ a -COCH₃ group;
▪ a moiety of structure (**J***);
▪ a -CH₂-CH₂-Y-FUNC moiety, wherein **Y** is selected from the group consisting of -O-, -COO-, -CONH-, -(CH₂)ᵣO-, being r a value comprised between 1 and 10; and FUNC is the moiety as previously defined;
▪ a moiety of structure **FUNC**, as previously defined.
∘ **R¹⁴** is selected from the group consisting of a hydrogen atom, a methyl moiety, an ethyl moiety and a **-R¹²-S-Z³** moiety, wherein **Z³** is independently selected from the same group as Z¹ and Z².

2. Compound of formula (I), according to claim 1, wherein
**X¹** is a hydrogen atom;
**Z** is a hydrogen atom or an acetyl (-COCH₃) moiety.

3. Compound of formula (I), according to claim 1, wherein
**X¹** is a hydrogen atom;
**R¹** is a **-R³-S-Z** moiety, wherein **Z** is a -CH₂-CH₂-Y-FUNC moiety, wherein Y is selected from the group consisting of -O-, -COO-, -(CH₂)ᵣO-, being r a value comprised between 1 and 10 and FUNC is as defined in claim 1.

4. Compound of formula (I), according to claim 1, wherein,
**X¹** is a hydrogen atom;
**R¹** is a **-R³-S-Z** moiety, wherein **Z** is a moiety of structure (**J***) wherein **X^{1'}** is a hydrogen atom.

5. Compound of formula (I), according to claim 1, wherein,
**X¹** is a hydrogen atom;
**R¹** is a moiety of the type (BRANCH*), as defined in claim 1, wherein,
• **Z¹** and **Z²** are independently selected from the group consisting of:
∘ a hydrogen atom;
∘ a -COCH₃ group;
∘ a moiety of structure (**J***), wherein **X^{1'}** is a hydrogen atom.
∘ a moiety of structure **FUNC**, as defined in claim 1.
• **R¹⁴** is an ethyl moiety.

6. Compound of formula (I), according to claim 5, wherein **Z¹** and **Z²** are independently selected from the group consisting of
• a hydrogen atom;
• a moiety of structure (**J***), wherein **X^{1'}** is a hydrogen atom.

7. Compound of formula (I), according to claim 5, wherein **Z¹** is a moiety of formula - (CH₂CH₂O)_{q}R', wherein R' is a hydrogen atom or a methyl group and q is a value comprised between 5 and 300.

8. Compound of formula (I), according to claim 5, wherein **Z¹** is a fluorescent tag.

9. Compound of formula (I), according to claim 1, wherein
**X¹** is a hydrogen atom;
**R¹** is a moiety of the type (BRANCH*), as defined in claim 1,
wherein,
• **Z¹** and **Z²** are independently selected from the group consisting of:
∘ a hydrogen atom;
∘ a -COCH₃ group;
∘ a moiety of structure (**J***), wherein **X^{1'}** is a hydrogen atom.
∘ a moiety of structure **FUNC,** as defined in claim 1.
• **R¹⁴** is a **-R¹²-S-Z³** moiety, wherein **Z³** is independently selected from the same group as Z¹ and Z².

10. Compound of formula (I), according to claim 9, wherein **Z¹**, **Z²** and **Z³** are independently selected from the group consisting of
• a hydrogen atom;
• a moiety of structure (**J***), wherein **X^{1'}** is a hydrogen atom.

11. Compound of formula (I), according to claim 9, wherein
**Z¹** and **Z²** are hydrogen atoms, and
**Z³** is a moiety of formula -(CH₂CH₂O)_{q}R', wherein R' is a hydrogen atom or a methyl group and q is a value comprised between 5 and 300.

12. Compound of formula (I), according to claim 9, wherein
**Z¹** and **Z²** are hydrogen atoms, and
**Z³** is a fluorescent tag.

13. Polymeric catecholic compound obtained by condensation of at least one monomer of the type (III) or of the type (IV), or a combination thereof; 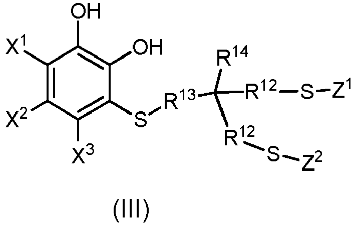 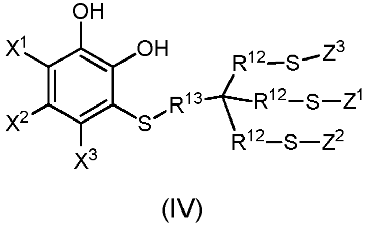 wherein Z¹ and Z² are hydrogen atoms;
X¹, X², X³, R¹², R¹³, R¹⁴ and Z³ are as defined in claim 1;
the polymerization degree is comprised between 2 and 10.000; and the molar fraction of said monomer of the type (III) or of the type (IV) is comprised between 0,01 and 1.

14. Use of a catechol-derivative compound or a polymeric catecholic compound according to any of claims 1-13 for preparing a functional coating.

15. Use of a catecholic compound according to claim 13 as an adhesive substance.

## Patentansprüche

1. Catechinderivatverbindung mit Formel (I): 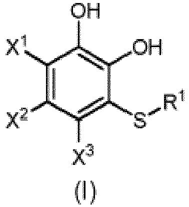 wobei
**X¹** aus der Gruppe, die aus Wasserstoff und einem Substituenten **-SR²** besteht, ausgewählt wird,
**X²** and **X³** Wasserstoffatome sind,
**R¹** und **R²** unabhängig ausgewählt werden aus der Gruppe bestehend aus:
• einem Rest **FUNC**, der ausgewählt wird aus der Gruppe bestehend aus:
∘ Alkylrest der Formel -CₙH₂ₙ₊₁, wobei n 12 bis 18 umfasst;
∘ Alkenylrest der Formel -CₙH₂ₙ₋₁, wobei n 2 bis 6 umfasst;
∘ Alkinylrest der Formel -CₙH₂ₙ₋₃, wobei n 2 bis 6 umfasst;
∘ Polyfluoroalkylrest der Formel -(CH₂)ₚ₁-CₙF₂ₙ₊₁, wobei p₁ 0 bis 3 umfasst, und n 5 bis 8 umfasst;
∘ einem Rest der Formel -(CHRCH₂O)_{q}R', wobei R ein Wasserstoffatom oder eine Methylgruppe ist, und R' ausgewählt wird aus einem Wasserstoffatom, einer C₁-C₁₀ Alkylgruppe, terminalem C₁-C₁₈ Alkenyl, terminalem C₁-C₁₈ Alkinyl, -COOH, -NH₂, -N₃ oder *N-*Maleimido, und q ein Wert ist, der 5 bis 300 umfasst;
∘ Fluoreszenzmarkierung ausgewählt aus Fluoreszenzverbindungen mit Thiolgruppen, Fluorescein, Eosin, Rhodaminen; Bor-Dipyrromethen (BODIPY) Fluoreszenzderivaten; Fluoreszensderivaten mit Azostruktur (Diazoarene), Fluoreszenzderivaten von Pyridin-methoxyphenylenoxazolen (PyMPO), Fluoreszenzderivaten von Lucifer-Gelb, Fluoreszenzderivaten von Benzoxadiazol (BD), Fluoreszenzrot, Fluoreszenzorange, und anderen Fluoreszenzmarkierungen, verfügbar unter eingetragenen Handelsmarken (Atto und Alexa), die durch Ad hoc-reaktive Gruppen konjugiert werden können;
∘ Oligopeptid ausgewählt aus Glutathion und einem Oligopeptid umfassend 5-25 Aminosäuren, von denen von 20 % bis 100 % Arginin sind; und
• einem Rest **-R³-S-Z**, wobei
∘ S ein Schwefelatom ist,
∘ **R³** ausgewählt wird aus der Gruppe bestehend aus:
• Alkandiylrest der Formel -(C_{n'}-H_{2n'})-, wobei n' 2 bis 18 umfasst;
• (Polyalkylenoxy)alkyl-Rest der Formel - (CHR"CH₂O)_{q1}(CHR"CH₂)-, wobei **R"** ausgewählt wird aus der Gruppe bestehend aus einem Wasserstoffatom und einem Methylrest, und q₁ 2 bis 300 umfasst;
∘ **Z** ausgewählt wird aus der Gruppe bestehend aus:
• Wasserstoffatom;
• -COCH₃-Rest;
• -CH₂-CH₂-Y-FUNC-Rest, wobei **Y** ausgewählt wird aus der Gruppe bestehend aus -O-, -COO-, -CONH-, -(CH₂)ᵣO-, wobei r ein Wert ist, der 1 bis 10 umfasst; und FUNC der Rest wie zuvor definiert ist;
• einem Rest der Struktur (**J***): 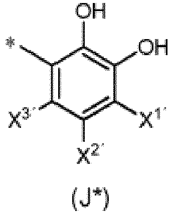 wobei **X^{1'}** ausgewählt wird aus der Gruppe bestehend aus Wasserstoff, und einem Substituenten **-SR²**; wobei R² wie zuvor definiert ist; und **X^{2'}** und **X^{3'}** Wasserstoffatome sind;
• einem Rest des Typs **(BRANCH*)**: 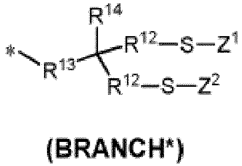 wobei
∘ **R¹²** ein Rest der Formel -(CH₂-O-Q-CH₂-W)- ist, wobei **Q** ausgewählt wird aus der Gruppe bestehend aus -CO-, und - (CₖH₂ₖO)_{q1}-, wobei k ein Wert ist, der 2 bis 4 umfasst, und q₁ ein Wert ist, der 2 bis 300 umfasst; und wobei **W** ausgewählt wird aus der Gruppe bestehend aus -CHR"'-, und -CH(OH)CH₂-, wobei **R'"** Wasserstoffatom oder Methylgruppe ist;
∘ **R¹³** ein Rest der Formel -(W-CH₂-Q-O-CH₂)- ist, wobei **Q** und **W** auf gleiche Weise definiert sind wie für R¹²;
∘ **Z¹** und **Z²** unabhängig ausgewählt werden aus der Gruppe bestehend aus:
• Wasserstoffatom;
• -COCH₃-Gruppe;
• einem Rest der Struktur (**J***);
• -CH₂-CH₂-Y-FUNC-Rest, wobei **Y** ausgewählt wird aus der Gruppe bestehend aus -O-, -COO-, -CONH, -(CH₂)ᵣO-, wobei r ein Wert ist, der 1 bis 10 umfasst; und FUNC der Rest wie zuvor definiert ist;
• einem Rest der Struktur **FUNC,** wie zuvor definiert;
∘ **R¹⁴** ausgewählt wird aus der Gruppe bestehend aus Wasserstoffatom, Methylrest, Ethylrest und **-R¹²-S-Z³**-Rest, wobei **Z³** unabhängig aus der gleichen Gruppe wie Z¹ und Z² ausgewählt wird.

2. Verbindung der Formel (I) nach Anspruch 1, wobei
**X¹** Wasserstoffatom ist;
**Z** Wasserstoffatom oder Acetyl (-COCH₃)-Rest ist.

3. Verbindung der Formel (I) nach Anspruch 1, wobei
**X¹** Wasserstoffatom ist;
**R¹** ein **-R³-S-Z**-Rest ist, wobei **Z** ein -CH₂-CH₂-Y-FUNC-Rest ist, wobei Y ausgewählt wird aus der Gruppe bestehend aus -O-, - COO-, -(CH₂)ᵣO-, wobei r ein Wert ist, der 1 bis 10 umfasst, und FUNC ein Rest wie in Anspruch 1 definiert ist.

4. Verbindung der Formel (I) nach Anspruch 1, wobei
**X¹** Wasserstoffatom ist;
**R¹** ein **-R³-S-Z**-Rest ist, wobei **Z** ein Rest der Struktur (**J***) ist, wobei
**X^{1'}** Wasserstoffatom ist.

5. Verbindung der Formel (I) nach Anspruch 1, wobei
**X¹** Wasserstoffatom ist;
**R¹** ein Rest des Typs (BRANCH*) ist, wie in Anspruch 1 definiert,
wobei
• **Z¹** und **Z²** unabhängig ausgewählt werden aus der Gruppe bestehend aus:
∘ Wasserstoffatom;
∘ -COCH₃-Gruppe;
∘ einem Rest der Struktur (**J***), wobei **X1'** ein Wasserstoffatom ist;
∘ einem Rest der Struktur **FUNC**, wie in Anspruch 1 definiert;
• **R¹⁴** Ethylrest ist.

6. Verbindung der Formel (I) nach Anspruch 5, wobei **Z¹** und **Z²** unabhängig ausgewählt werden aus der Gruppe bestehend aus:
• Wasserstoffatom;
• einem Rest der Struktur (**J***), wobei **X^{1'}** ein Wasserstoffatom ist.

7. Verbindung der Formel (I) nach Anspruch 5, wobei **Z¹** ein Rest der Formel - (CH₂CH₂O)_{q}R' ist, wobei R' Wasserstoffatom oder Methylgruppe ist und q ein Wert ist, der 5 bis 300 umfasst.

8. Verbindung der Formel (I) nach Anspruch 5, wobei **Z¹** eine Fluoreszenzmarkierung ist.

9. Verbindung der Formel (I) nach Anspruch 1, wobei
**X¹** Wasserstoffatom ist;
**R¹** ein Rest des Typs (BRANCH*) ist, wie in Anspruch 1 definiert,
wobei
• **Z¹** und **Z²** unabhängig ausgewählt werden aus der Gruppe bestehend aus:
∘ Wasserstoffatom;
∘ -COCH₃-Gruppe;
∘ einem Rest der Struktur (**J***), wobei **X^{1'}** Wasserstoffatom ist;
∘ einem Rest der Struktur **FUNC**, wie in Anspruch 1 definiert;
• **R¹⁴** ein **-R¹²-S-Z³**-Rest, wobei **Z³** unabhängig aus der gleichen Gruppe wie Z¹ und Z² ausgewählt wird, ist.

10. Verbindung der Formel (I) nach Anspruch 9, wobei **Z¹**, **Z²** und **Z³** unabhängig ausgewählt werden aus der Gruppe bestehend aus:
• Wasserstoffatom;
• einem Rest der Struktur (**J***), wobei **X^{1'}** Wasserstoffatom ist.

11. Verbindung der Formel (I) nach Anspruch 9, wobei
**Z¹** und **Z²** Wasserstoffatome sind, und
**Z³** ein Rest der Formel -(CH₂CH₂O)_{q}R' ist, wobei R' Wasserstoffatom oder Methylgruppe ist und q ein Wert, der 5 bis 300 umfasst, ist.

12. Verbindung der Formel (I) nach Anspruch 9, wobei
**Z¹** und **Z²** Wasserstoffatome sind, und
**Z³** eine Fluoreszenzmarkierung ist.

13. Polymere Catechinverbindung, erhalten durch Kondensation von zumindest einem Monomer des Typs (III) oder des Typs (IV), oder einer Kombination davon; 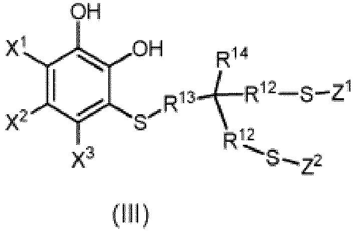 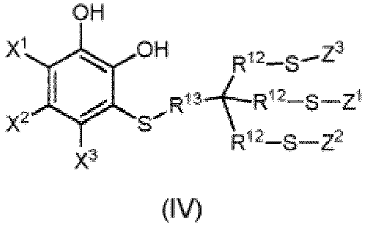 wobei Z¹ und Z² Wasserstoffatome sind;
X¹, X², X³, R¹², R¹³, R¹⁴ und Z³ wie in Anspruch 1 definiert sind;
der Polymerisationsgrad 2 bis 10.000 umfasst; und der Stoffmengenanteil des Monomers des Typs (III) oder des Typs (IV) 0,01 bis 1 umfasst.

14. Verwendung einer Catechinderivatverbindung oder einer polymeren Catechinverbindung nach einem der Ansprüche 1-13 zum Herstellen einer funktionellen Beschichtung.

15. Verwendung einer Catechinverbindung nach Anspruch 13 als Klebmasse.

## Revendications

1. Composé dérivé de catéchol de formule (I) : 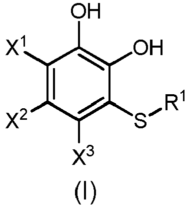 dans laquelle
**X¹** est choisi dans l'ensemble constitué par l'hydrogène et un substituant **-SR²**,
**X²** et **X³** sont des atomes d'hydrogène,
**R¹** et **R²** sont indépendamment choisis dans l'ensemble constitué par :
• un fragment **FUNC**, qui est choisi dans l'ensemble constitué par :
∘ un fragment alkyle de formule -CₙH₂ₙ₊₁, dans laquelle n est situé dans la plage comprise entre 12 et 18 ;
∘ un fragment alcényle de formule -CₙH₂ₙ₋₁, dans laquelle n est situé dans la plage comprise entre 2 et 6 ;
∘ un fragment alcynyle de formule -CₙH₂ₙ₋₃, dans laquelle n est situé dans la plage comprise entre 2 et 6 ;
∘ un fragment polyfluoroalkyle de formule -(CH₂)ₚ₁-CₙF₂ₙ₊₁, dans laquelle p₁ est compris entre 0 et 3, et n est compris entre 5 et 8 ;
∘ un fragment de formule -(CHRCH₂O)_{q}R', dans laquelle R est un atome d'hydrogène ou un groupe méthyle, et R' est choisi parmi un atome d'hydrogène, un groupe alkyle en C₁ à C₁₀, un alcényle en C₁ à C₁₈ terminal, un alcynyle en C₁ à C₁₈ terminal, -COOH, -NH₂, -N₃ ou *N*-maléimido, et q a une valeur comprise entre 5 et 300 ;
∘ une étiquette fluorescente choisie parmi les composés fluorescents avec des groupes thiol, la fluorescéine, l'éosine, les rhodamines ; les dérivés fluorescents de bore-dipyrrométhène (BODIPY) ; les dérivés fluorescents de structure azoïque (diazoarènes), les dérivés fluorescents de pyridine-méthoxyphénylène-oxazoles (PyMPO), les dérivés fluorescents de Jaune Lucifer, les dérivés fluorescents de benzoxadiazole (BD), le rouge fluorescent, l'orange fluorescent, et d'autres marqueurs fluorescents disponibles sous des marques déposées (Atto et Alexa), qui peuvent être conjugués par l'intermédiaire de groupes réactifs ad hoc ;
o un oligopeptide choisi parmi le glutathion et un oligopeptide constitué de 5 à 25 acides aminés, dont 20 % à 100 % sont l'arginine ; et
• un fragment **-R³-S-Z**, dans lequel,
∘ S est un atome de soufre,
∘ **R³** est choisi dans l'ensemble constitué par :
▪ un fragment alcanediyle de formule -(CₙH₂ₙ)-, dans laquelle n'est compris entre 2 et 18 ;
▪ un fragment (polyalkylène-oxy)alkyle de formule -(CHR"CH₂O)_{q1}(CHR"CH₂)-, dans laquelle **R"** est choisi dans l'ensemble constitué par un atome d'hydrogène et un fragment méthyle, et q₁ est compris entre 2 et 300 ;
∘ **Z** est choisi dans l'ensemble constitué par :
▪ un atome d'hydrogène ;
▪ un fragment -COCH₃ ;
▪ un fragment -CH₂-CH₂-Y-FUNC, dans lequel Y est choisi dans l'ensemble constitué par -O-, -COO-, -CONH-, - (CH₂)ᵣO-, r ayant une valeur comprise entre 1 et 10 ; et FUNC est le fragment tel que défini précédemment ;
▪ un fragment de structure (**J***) : 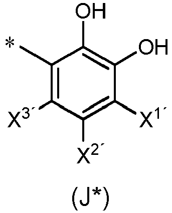 dans laquelle **X^{1'}** est choisi dans l'ensemble constitué par l'hydrogène, et un substituant **-SR²** ; dans lequel R² est tel que défini précédemment ; et
**X^{2'}** et **X^{3'}** sont des atomes d'hydrogène ;
• un fragment du type **(BRANCH*)** : 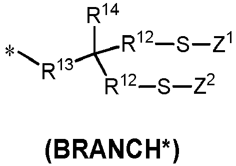 dans lequel,
∘ **R¹²** est un fragment de formule -(CH₂-O-Q-CH₂-W)-, dans laquelle **Q** est choisi dans l'ensemble constitué par -CO-, et -(CₖH₂ₖO)_{q1}-, où k a une valeur comprise entre 2 et 4, et q₁ a une valeur comprise entre 2 et 300 ; et dans laquelle **W** est choisi dans l'ensemble constitué par -CHR"'-, et -CH(OH)CH₂-, où **R'"** est un atome d'hydrogène ou un groupe méthyle ;
∘ **R¹³** est un fragment de formule -(W-CH₂-Q-O-CH₂)-, dans laquelle **Q** et **W** sont définis de la même façon que R¹² ;
∘ **Z¹** et **Z²** sont indépendamment choisis dans l'ensemble constitué par
▪ un atome d'hydrogène ;
▪ un groupe -COCH₃ ;
▪ un fragment de structure (**J***) ;
▪ un fragment -CH₂-CH₂-Y-FUNC, dans lequel Y est choisi dans l'ensemble constitué par -O-, -COO-, -CONH-, - (CH₂)ᵣO-, r ayant une valeur comprise entre 1 et 10 ; et FUNC est le fragment tel que défini précédemment ;
▪ un fragment de structure **FUNC**, tel que défini précédemment ;
∘ **R¹⁴** est choisi dans l'ensemble constitué par un atome d'hydrogène, un fragment méthyle, un fragment éthyle et un fragment **-R¹²-S-Z³**, dans lequel **Z³** est indépendamment choisi dans le même ensemble que Z¹ et Z².

2. Composé de formule (I) selon la revendication 1, dans lequel
**X¹** est un atome d'hydrogène ;
**Z** est un atome d'hydrogène ou un fragment acétyle (-COCH₃).

3. Composé de formule (I) selon la revendication 1, dans lequel
**X¹** est un atome d'hydrogène ;
**R¹** est un fragment **-R³-S-Z**, dans lequel **Z** est un fragment -CH₂-CH₂-Y-FUNC, dans lequel Y est choisi dans l'ensemble constitué par -O-, -COO-, -(CH₂)ᵣO-, r ayant une valeur comprise entre 1 et 10, et FUNC est tel que défini dans la revendication 1.

4. Composé de formule (I) selon la revendication 1, dans lequel
**X¹** est un atome d'hydrogène ;
**R¹** est un fragment **-R³-S-Z**, dans lequel **Z** est un fragment de structure (**J***) dans lequel **X^{1'}** est un atome d'hydrogène.

5. Composé de formule (I) selon la revendication 1, dans lequel
**X¹** est un atome d'hydrogène ;
**R¹** est un fragment du type (BRANCH*), tel que défini dans la revendication 1,
dans lequel
• **Z¹** et **Z²** sont indépendamment choisis dans l'ensemble constitué par :
∘ un atome d'hydrogène ;
∘ un groupe -COCH₃ ;
∘ un fragment de structure (**J***) dans lequel **X^{1'}** est un atome d'hydrogène ;
∘ un fragment de structure **FUNC**, tel que défini dans la revendication 1 ;
• **R¹⁴** est un fragment éthyle.

6. Composé de formule (I) selon la revendication 5, dans lequel **Z¹** et **Z²** sont indépendamment choisis dans l'ensemble constitué par
• un atome d'hydrogène ;
• un fragment de structure (**J***) dans lequel **X^{1'}** est un atome d'hydrogène.

7. Composé de formule (I) selon la revendication 5, dans lequel **Z¹** est un fragment de formule -(CH₂CH₂O)_{q}R', dans laquelle R' est un atome d'hydrogène ou un groupe méthyle et q a une valeur comprise entre 5 et 300.

8. Composé de formule (I) selon la revendication 5, dans lequel **Z¹** est une étiquette fluorescente.

9. Composé de formule (I) selon la revendication 1, dans lequel
**X¹** est un atome d'hydrogène ;
**R¹** est un fragment du type (BRANCH*) tel que défini dans la revendication 1,
dans lequel
• **Z¹** et **Z²** sont indépendamment choisis dans l'ensemble constitué par :
∘ un atome d'hydrogène ;
∘ un groupe -COCH₃ ;
∘ un fragment de structure (**J***) dans lequel **X^{1'}** est un atome d'hydrogène ,
∘ un fragment de structure **FUNC** tel que défini dans la revendication 1 ;
• **R¹⁴** est un fragment **-R¹²-S-Z³**, dans lequel **Z³** est indépendamment choisi dans le même ensemble que Z¹ et Z².

10. Composé de formule (I) selon la revendication 9, dans lequel **Z¹**, **Z²** et **Z³** sont indépendamment choisis dans l'ensemble constitué par
• un atome d'hydrogène ;
• un fragment de structure (**J***) dans lequel **X^{1'}** est un atome d'hydrogène.

11. Composé de formule (I) selon la revendication 9, dans lequel
**Z¹** et **Z²** sont des atomes d'hydrogène, et
**Z³** est un fragment de formule -(CH₂CH₂O)_{q}R', dans laquelle R' est un atome d'hydrogène ou un groupe méthyle et q a une valeur comprise entre 5 et 300.

12. Composé de formule (I) selon la revendication 9, dans lequel
**Z¹** et **Z²** sont des atomes d'hydrogène, et
**Z³** est une étiquette fluorescente.

13. Composé catécholique polymère obtenu par condensation d'au moins un monomère du type (III) ou du type (IV), ou une de leurs combinaisons : 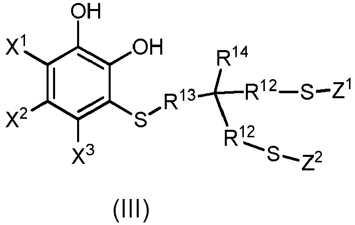 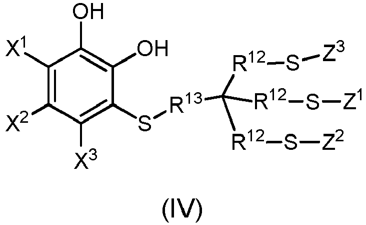 dans lesquelles Z¹ et Z² sont des atomes d'hydrogène ;
X¹, X², X³, R¹², R¹³, R¹⁴ et Z³ sont tels que définis dans la revendication 1 ;
le degré de polymérisation est compris entre 2 et 10 000 ; et la fraction molaire dudit monomère du type (III) ou du type (IV) est comprise entre 0,01 et 1.

14. Utilisation d'un composé dérivé de catéchol ou d'un composé catécholique polymère selon l'une quelconque des revendications 1 à 13 pour la préparation d'un revêtement fonctionnel.

15. Utilisation d'un composé catécholique selon la revendication 13 en tant que substance adhésive.
